# EUROPEAN PATENT APPLICATION

(11) **EP 3 831 941 A1**
(43) Date of publication of application: **09.06.2021**
(21) Application number: 19844229.5
(22) Date of filing: 30.07.2019
(51) Int. Cl.: C12N 15/10, C12Q 1/6844

(54) **METHOD FOR EDITING DNA IN CELL-FREE SYSTEM**

(30) Priority: 30.07.2018 JP 2018142274
(71) Applicant: Oriciro Genomics, Inc., Bunkyo-ku Tokyo 113-8485 (JP)
(72) Inventor: SUETSUGU, Masayuki, Tokyo 171-8501 (JP); TAWARAGI, Ayako, Tokyo 171-8501 (JP); KANOH, Koki, Tokyo 171-8501 (JP)
(74) Representative: Williams, Andrea
(86) International application number: PCT/JP2019/029793
(87) International publication number: WO 2020/027110

(57) **Abstract**

A method for editing DNA in a cell-free system, the method including: (1) a step of introducing a deletion, substitution or addition at a target site of a DNA in a cell-free system, and (2) a step of amplifying, in a cell-free system, the DNA into which a deletion, substitution or addition has been introduced in step (1), wherein the DNA is amplified under temperature conditions that incubate at a temperature within a range from 20°C to 80°C.

## Description

### TECHNICAL FIELD

The present invention relates to a method for editing DNA in a cell-free system.

Priority is claimed on Japanese Patent Application No. 2018-142274, filed July 30, 2018, the content of which is incorporated herein by reference.

### BACKGROUND ART

Genome sequencing is a technique that uses a target sequence-specific nuclease to modify a target gene in an intentional manner. Examples of known nucleases that may be used include endonucleases such as CRISPR-Cas9, ZFN and TALEN. By using genome sequencing techniques, targeted DNA sequences can be edited inside cells in a large variety of species from bacteria to humans (Non-Patent Documents 1 to 3). Target sequence-specific endonucleases are enzymes that cleave the DNA double strand, and therefore in order to obtain a target edited genome, following cleavage of the DNA double strand, an intracellular DNA repair process must be conducted. In those cases where a new DNA sequence is to be inserted into a genome, the repair process functions by homologous recombination. By introducing a DNA fragment containing a homologous sequence at the cleavage site into the cells during the DNA double strand cleavage, a desired DNA sequence can be inserted at the cleavage site by recombination utilizing the homologous sequence. Further, in those cases where a short sequence of several bases is to be deleted, the repair process functions by non-homologous end joining (NHEJ). In these cases, repair of the cleavage site by NHEJ causes deletion or mutation of the bases.

Cleavage of the DNA double strand by a target sequence-specific endonuclease not only functions as a trigger for these intracellular repair processes, but also has the function of counter-selection in which the unedited remaining DNA is selectively inactivated. In other words, the edited DNA can no longer undergo cleavage by the target sequence-specific endonuclease, and is therefore amplified inside the cells by a replication process, whereas the unedited DNA continues to be exposed to double strand cleavage attack, meaning intracellular amplification is suppressed.

In Escherichia coli (E. coli), DNA sequences can also be modified in cells without using a target sequence-specific endonuclease. Known examples of such systems include systems using the RecET recombination enzyme of prophage (non-Patent Document 4) and systems using the Red recombination enzyme of λ phage (Non-Patent Documents 5 and 6). In these systems, an end of the double-stranded DNA fragment introduced into the E. coli is single-stranded, and the recombination reaction is guided by pairing of this portion and a single-stranded portion exposed during DNA replication in the genome. Even when the fragment is not a double-stranded DNA fragment, by introducing a short single-stranded DNA, namely an oligo DNA, into E. coli, that oligo DNA can pair with an exposed single strand, introducing a base substitution in the genome (Non-Patent Document 7).

Although the efficiency of this type of base substitution by oligo DNA in E. coli is low, recently, a method for improving this efficiency by using the CRISPR-Cas9 target sequence cleavage system as a counter-selection has also been reported (Non-Patent Document 8).

On the other hand, a technique that uses a polymerase chain reaction (PCR) is already known as a technique for editing DNA in cell-free systems. For example, by using a DNA primer containing a mutation such as a base substitution, deletion, or addition or the like to amplify the template DNA by the PCR method, and amplifying the ligation product of DNA fragments by the PCR method, a product having a DNA sequence that has been artificially edited can be prepared without using cells.

A target sequence cleavage system using CRISPR-Cas9 can also be used in vitro. For example, the cloning of a 100 kb region of a genome in an E. coli plasmid using CRISPR-Cas9 has been reported (Non-Patent Document 9). In this report, genome cleavage at a target site by CRISPR-Cas9 and an assembly reaction between the cleaved fragment and the plasmid are conducted in a test tube.

Further, known techniques for joining together DNA fragments in cell-free systems include the In-Fusion method (Patent Document 1), the Gibson Assembly method (Patent Documents 2 and 3), and the Recombination Assembly method (Patent Document 4).

Although the PCR method is widely used as a technique for amplifying DNA in a cell-free system, because of the processivity of the enzyme used, there is a limit to the length of DNA that can be amplified. In the PCR method, amplification of long-chain DNA exceeding 50 kb is generally problematic.

One method that has been reported for amplifying that type of long-chain DNA in a cell-free system is an in vitro replication system for amplifying a circular DNA having a replication origin sequence oriC (Patent Documents 5 to 7). This method is called the Replication Cycle Reaction (RCR) method, and is capable of amplifying long-chain DNA of 50 kb or more which are difficult to amplify by the PCR method.

The types of DNA editing methods using cells described above require advanced techniques for the introduction of the enzymes required for cultivation and reaction into the cells, and also require considerable time and effort. Further, in those cases where the DNA sequence produced by editing exhibits cell toxicity, another problem arises in that the target product may be unobtainable. Furthermore, the actual introduction of DNA into cells becomes more difficult as the length of the DNA chain increases.

### PRIOR ART LITERATURE

### Patent Documents

Patent Document 1: U.S. Patent Publication No. 7,575,860
Patent Document 2: U.S. Patent Publication No. 7,776,532
Patent Document 3: U.S. Patent Publication No. 8,968,999
Patent Document 4: International Patent Publication No. 2019/009361
Patent Document 5: International Patent Publication No. 2016/080424
Patent Document 6: International Patent Publication No. 2017/199991
Patent Document 7: International Patent Publication No. 2018/159669

### Non-Patent Documents

Non-Patent Document 1: Carroll, D. (2014) Genome Engineering with Targetable Nucleases. Annual Review of Biochemistry, 83, 409-439.
Non-Patent Document 2: Mougiakos, I., Bosma, E.F., de Vos, W.M., van Kranenburg, R. and van der Oost, J. (2016) Next Generation Prokaryotic Engineering: The CRISPR-Cas Toolkit. Trends in Biotechnology, 34, 575-587.
Non-Patent Document 3: Mougiakos, I., Bosma, E.F., Ganguly, J., van der Oost, J. and van Kranenburg, R. (2018) Hijacking CRISPR-Cas for high-throughput bacterial metabolic engineering: advances and prospects. Current Opinion in Biotechnology, 50, 146-157.
Non-Patent Document 4: Zhang, Y., Buchholz, F., Muyrers, J.P. and Stewart, A.F. (1998) A new logic for DNA engineering using recombination in Escherichia coli. Nature genetics, 20, 123-128.
Non-Patent Document 5: Datsenko, K.A. and Wanner, B.L. (2000) One-step inactivation of chromosomal genes in Escherichia coli K-12 using PCR products. Proceedings of the National Academy of Sciences of the United States of America, 97, 6640-6645.
Non-Patent Document 6: Yu, D., Ellis, H.M., Lee, E.C., Jenkins, N.A., Copeland, N.G. and Court, D.L. (2000) An efficient recombination system for chromosome engineering in Escherichia coli. Proceedings of the National Academy of Sciences of the United States of America, 97, 5978-5983.
Non-Patent Document 7: Ellis, H.M., Yu, D., DiTizio, T. and Court, D.L. (2001) High efficiency mutagenesis, repair, and engineering of chromosomal DNA using single-stranded oligonucleotides. Proceedings of the National Academy of Sciences of the United States of America, 98, 6742-6746.
Non-Patent Document 8: Ronda, C., Pedersen, L.E., Sommer, M.O.A. and Nielsen, A.T. (2016) CRMAGE: CRISPR Optimized MAGE Recombineering. Scientific Reports, 6, 1-11.
Non-Patent Document 9: Wang, H., Li, Z., Jia, R., Yin, J., Li, A., Xia, L., Yin, Y., Muller, R., Fu, J., Stewart, A.F., and Zhang, Y. (2017) ExoCET: exonuclease in vitro assembly combined with RecET recombination for highly efficient direct DNA cloning from complex genomes. Nucleic Acids Research, 10.1093/nar/gkx1296.

### SUMMARY OF INVENTION

### Problems to be Solved by the Invention

The present invention provides a method for editing DNA in a cell-free system that enables the entire process through to the step of selectively amplifying the final DNA edited product to be conducted without using cells.

### Means for Solving the Problems

As a result of intensive research, the inventors of the present invention discovered that by combining a technique for either amplifying DNA at a constant temperature in a cell-free system, or amplifying DNA by repeating incubation at two temperatures not higher than 65°C in a cell-free system, with a DNA editing (modification) technique that can be used in a cell-free system, a DNA edited product could be amplified without using cells. Moreover, the inventors also discovered that by incorporating a step of specifically cleaving the unedited DNA, the yield of the DNA edited product could be improved dramatically. As a result of further research based on these discoveries, the inventors were able to complete the present invention.

In other words, the present invention includes the following aspects.
[1] A method for editing DNA in a cell-free system, the method including the following steps:
   (1) a step of introducing a deletion, substitution or addition at a target site of a DNA in a cell-free system, and
   (2) a step of amplifying, in a cell-free system, the DNA into which a deletion, substitution or addition has been introduced in step (1), wherein the DNA is amplified under temperature conditions that incubate at a temperature within a range from 20°C to 80°C.
[2] The method for editing DNA in a cell-free system according to [1], the method including the following steps:
   (1) a step of introducing a deletion, substitution or addition at a target site of a DNA in a cell-free system, and
   (2) a step of amplifying, in a cell-free system, the DNA into which a deletion, substitution or addition has been introduced in step (1), wherein the DNA is amplified under temperature conditions that either incubate at a constant temperature, or incubate under a repeating temperature cycle in which incubation is conducted at two temperatures of 65°C or lower.
[3] The method for editing DNA in a cell-free system according to [1] or [2], the method including the following steps:
   (1) a step of introducing a deletion, substitution or addition at a target site of a DNA in a cell-free system, and
   (2) a step of amplifying, in a cell-free system, the DNA into which a deletion, substitution or addition has been introduced in step (1), wherein the DNA is amplified under temperature conditions that either incubate at a fixed temperature within a range from 20°C to 80°C, or incubate under a repeating temperature cycle in which incubation is conducted at two temperatures of 65°C or lower.
[4] The method according to any one of [1] to [3], wherein step (2) is conducted in the presence of an artificial DNA cleavage enzyme that specifically cleaves the DNA into which a deletion, substitution or addition has not been introduced.
[5] The method according to [4], wherein the artificial DNA cleavage enzyme is an artificial nuclease or an RNA-guided nuclease.
[6] The method according to [4], wherein the artificial DNA cleavage enzyme is CRISPR-Cas9.
[7] The method according to any one of [1] to [6], wherein the DNA is a circular DNA.
[8] The method according to [7], wherein step (2) includes the following steps:
   (2-1) a step of preparing a reaction mixture of: a reaction solution containing (a) a first enzyme group that catalyzes replication of the circular DNA, (b) a second enzyme group that catalyzes an Okazaki fragment ligation reaction and synthesizes two sister circular DNAs that form a catenane, and (c) a third enzyme group that catalyzes a separation reaction of the two sister circular DNAs; and the circular DNA into which a deletion, substitution or addition has been introduced in step (1); and
   (2-2) a step of incubating the reaction mixture prepared in step (2-1), either at a fixed temperature within a range from 20°C to 80°C, or under a repeating temperature cycle in which incubation is conducted at two temperatures of 65°C or lower.
[9] The method according to [8], wherein the circular DNA contains a replication origin sequence that can bind to an enzyme having DnaA activity.
[10] The method according to [7], wherein in step (2), the circular DNA is amplified by rolling circle amplification.
[11] The method according to any one of [1] to [10], wherein step (1) includes the following steps:
   (1-1) a step of cleaving the DNA at a target site to prepare at least one linear DNA by causing an artificial DNA cleavage enzyme to act upon the DNA;
   (1-2) a step of preparing a reaction solution containing the linear DNA prepared in step (1-1), one or more types of DNA fragment, and a protein having RecA family recombinase activity; and
   (1-3) a step of mutually joining the linear DNA and the one or more types of DNA fragment at regions in which the base sequences are homologous or at regions in which the base sequences are complementary, thereby forming a DNA in which the one or more types of DNA fragment have each been inserted at a target site of the template DNA.
[12] The method according to any one of [1] to [10], wherein step (1) includes the following step:
   a step of conducting a DNA replication reaction in the presence of a single-stranded DNA used for introducing a deletion, substitution or addition, wherein the single-stranded DNA can hybridize with a target site of the DNA under the replication reaction conditions.
[13] The method according to any one of [1] to [12], wherein in step (2), the DNA is amplified under temperature conditions that incubate under a temperature cycle that repeats incubation at 30°C or higher and incubation at 27°C or lower.
[14] The method according to any one of [1] to [13], wherein the size of the DNA into which a deletion, substitution or addition has been introduced is 50 kb or larger.

### Effects of the Invention

By employing the method of the aspects described above, a method and the like for amplifying a DNA edited product, and particularly a long-chain edited DNA product, without using cells can be provided. Further, by using the method of the above aspects, DNA can be amplified under comparatively low temperature conditions, and therefore by combining the method with techniques that have proven difficult to combine with conventional DNA amplification techniques, such as sequence-specific DNA cleavage techniques using artificial DNA cleavage enzymes such as CRISPR-Cas9, DNA edited products can be amplified with good efficiency.

### BRIEF DESCRIPTION OF THE DRAWINGS

FIG. 1 is schematic diagram illustrating linearization of long-chain oriC DNA by sequence-specific cleavage by Cas9, insertion of a linear DNA into the linearized circular DNA by a ligation method (recombination assembly, RA) of the DNA molecules using homologous end sequences, and amplification of the circular DNA following insertion by RCR (Replication Cycle Reaction).
FIG. 2A is a diagram illustrating the structure of pOri8 (a 9.5 kb circular DNA).
FIG. 2B is a diagram illustrating sequence-specific cleavage of pOri8 by CRISPR-Cas9.
FIG. 3A is a diagram illustrating the structure of pMSR227 (a 205 kb circular DNA).
FIG. 3B is a diagram illustrating sequence-specific cleavage of pMSR227 by CRISPR-Cas9.
FIG. 4 is a schematic diagram illustrating that by conducting RCR in the presence of CRISPR-Cas9, amplification of unmodified template DNA is inhibited, and the target ligation product can be specifically amplified.
FIG. 5 is a schematic diagram illustrating that by adding CRISPR-Cas9, which cleaves the template DNA but does not cleave the DNA into which lacZ has been inserted, to a reaction system and conducting RCR, the circular DNA having an inserted lacZ can be amplified with high efficiency.
FIG. 6A is a diagram illustrating the structure of pMSR227 (205 kb) having an inserted lacZ (3.3 kb).
FIG. 6B is a diagram confirming the structure of the lacZ-inserted pMSR227 by restriction enzyme XhoI cleavage.
FIG. 7 is a schematic diagram illustrating a step of conducting a ligation circularization of two long-chain DNA fragments from Example 3 by RA via two adapter fragments.
FIG. 8 is a diagram illustrating the amplification of a long-chain circular DNA by RA-RCR in Example 3.
FIG. 9 is a diagram confirming the size of a plasmid collected from E. coli that has been transformed with the ligation product of 183 kb from Example 3.
FIG. 10 is a diagram confirming the structure of the ligation product of 183 kb obtained in Example 3.
FIG. 11A is a diagram illustrating the structure of a lacZ-modified pPKOZ (pPKOZins and pPKOZmis) used in Example 4.
FIG. 11B is a diagram illustrating the test system of Example 4.
FIG. 12 is a diagram illustrating downstream partial sequences of the start codons of lacZ wt, lacZ ins and lacZ mis (the respective base sequences are shown in SEQ ID NOS: 58 to 60) used in Example 4, and the sequence (SEQ ID NO: 17, SUE1355) of an oligonucleotide (30 mer) used for introducing a base substitution.
FIG. 13A is a diagram illustrating the results of investigating the effects of the length and concentration of the oligonucleotide used for introducing a base substitution on the amplification efficiency of the plasmid having the introduced base substitution (namely, the proportion of blue colonies). The diagram shows the results of electrophoresis.
FIG. 13B is a diagram illustrating the results of investigating the effects of the length and concentration of the oligonucleotide used for introducing a base substitution on the amplification efficiency of the plasmid having the introduced base substitution (namely, the proportion of blue colonies). The diagram shows the results of blue-white determination of the E. coli transformant colonies.
FIG. 14A is a diagram illustrating the results of investigating the effect of the concentration of the oligonucleotide used for introducing a base substitution on the amplification efficiency of the plasmid having the introduced base substitution (namely, the proportion of blue colonies). The diagram shows the results of electrophoresis.
FIG. 14B is a diagram illustrating the results of investigating the effect of the concentration of the oligonucleotide used for introducing a base substitution on the amplification efficiency of the plasmid having the introduced base substitution (namely, the proportion of blue colonies). The diagram shows the results of blue-white determination of the E. coli transformant colonies.
FIG. 15A is a diagram illustrating that by adding CRISPR-Cas9, which specifically cleaves the plasmid prior to the introduction of a base substitution, to the reaction system and conducting RCR, a base substitution introduction ratio close to 100% was able to be achieved. The diagram shows the results of electrophoresis.
FIG. 15B is a diagram illustrating that by adding CRISPR-Cas9, which specifically cleaves the plasmid prior to the introduction of a base substitution, to the reaction system and conducting RCR, a base substitution introduction ratio close to 100% was able to be achieved. The diagram shows the results of blue-white determination of the E. coli transformant colonies.
FIG. 16A is a diagram illustrating the structure of pOri93Zins.
FIG. 16B is a diagram illustrating the results of investigating the effect of the concentration of the oligonucleotide used for introducing a base substitution on the amplification efficiency of the plasmid having the introduced base substitution (namely, the proportion of blue colonies) for a long-chain DNA. The diagram shows the results of electrophoresis.
FIG. 16C is a diagram illustrating the results of investigating the effect of the concentration of the oligonucleotide used for introducing a base substitution on the amplification efficiency of the plasmid having the introduced base substitution (namely, the proportion of blue colonies) for a long-chain DNA. The diagram shows the results of blue-white determination of the E. coli transformant colonies.
FIG. 17A is a diagram illustrating that, for a long-chain DNA, by adding CRISPR-Cas9, which specifically cleaves the plasmid prior to the introduction of a base substitution, to the reaction system and conducting RCR, a base substitution introduction ratio close to 100% was able to be achieved. The diagram shows the results of electrophoresis.
FIG. 17B is a diagram illustrating that, for a long-chain DNA, by adding CRISPR-Cas9, which specifically cleaves the plasmid prior to the introduction of a base substitution, to the reaction system and conducting RCR, a base substitution introduction ratio close to 100% was able to be achieved. The diagram shows the results of blue-white determination of the E. coli transformant colonies.
FIG. 18A is a diagram illustrating a partial sequence (SEQ ID NO: 61) near the start codon of pPKOZins used in Example 9, and the sequence (SEQ ID NO: 56) of the oligonucleotide (60 mer) used for modification (a 3-base substitution).
FIG. 18B is a diagram illustrating a partial sequence (SEQ ID NO: 62) near the start codon of pPKOZ used in Example 9, and the sequence (SEQ ID NO: 57) of the oligonucleotide (60 mer) used for modification (a 4-base insertion).
FIG. 18C is a diagram illustrating the results of investigating the effect of the concentration of the oligonucleotide used for modification (3-base substitution or 4-base insertion) on the amplification efficiency of the plasmid having the 3-base substitution or 4-base insertion (namely, the proportion of the blue colony in the case of the 3-base substitution, and the proportion of the white colony in the case of the 4-base insertion). The diagram shows the results of electrophoresis.
FIG. 18D is a diagram illustrating the results of investigating the effect of the concentration of the oligonucleotide used for modification (3-base substitution) on the amplification efficiency of the plasmid having the 3-base substitution (namely, the proportion of the blue colony). The diagram shows the results of blue-white determination of the E. coli transformant colonies.
FIG. 18E is a diagram illustrating the results of investigating the effect of the concentration of the oligonucleotide used for modification (4-base insertion) on the amplification efficiency of the plasmid having the 4-base insertion (namely, the proportion of the white colony). The diagram shows the results of blue-white determination of the E. coli transformant colonies.
FIG. 18F is a diagram illustrating the EcoRI cleavage site of pPKOZ(wt).
FIG. 18G is a diagram illustrating the EcoRI cleavage site of pPKOZ4ins.
FIG. 18H a diagram illustrating results confirming the plasmid with 4-base insertion by restriction enzyme treatment. The diagram shows the results of electrophoresis.
FIG. 19A illustrates the structure of pK30V_insAC.
FIG. 19B is a diagram illustrating the test system of Example 10.
FIG. 19C is a diagram showing the sequence (SEQ ID NO: 68) of vioA ins of pP30V_insAC used in Example 10, and the sequence (SEQ ID NO: 65, SUE4577) of the oligonucleotide (60 mer) used for base substitution introduction.
FIG. 19D is a diagram showing the sequence (SEQ ID NO: 69) of vioC ins of pP30V_insAC used in Example 10, and the sequence (SEQ ID NO: 66, SUE4381) of the oligonucleotide (60 mer) used for base substitution introduction.
FIG. 19E is a diagram illustrating the results of investigating the effect of the reaction time with the oligonucleotide used for base substitution introduction on the proportion of plasmid having simultaneous base substitution at two locations (namely, the proportion of violet colonies. The diagram shows the results of color determination of the E. coli transformant colonies.

### EMBODIMENTS FOR CARRYING OUT THE INVENTION

### I. Method for Editing DNA in a Cell-Free System

The present invention provides a method for editing DNA in a cell-free system, the method (in this description, sometimes referred to as the method of the present invention) including the following steps:
(1) a step of introducing a deletion, substitution or addition at a target site of a DNA in a cell-free system, and
(2) a step of amplifying, in a cell-free system, the DNA into which a deletion, substitution or addition has been introduced in step (1), wherein the DNA is amplified under temperature conditions that incubate at a temperature within a range from 20°C to 80°C.

In the present invention, the expression "editing DNA in a cell-free system" refers to modifying a target site in the DNA and then amplifying the obtained DNA without using cells. In this description, the DNA prior to modification is sometimes referred to as the template DNA. In conventional systems using cells, it is necessary to either introduce into the cells a DNA that has been modified outside the cells, or introduce into the cells an enzyme or the like required for modifying DNA that exists inside the cells, and culturing and the like of the cells not only requires advanced techniques, but also requires large amounts of time and effort. In the present invention, absolutely no cells are used in the processes for modifying and amplifying the DNA, and therefore the modified DNA can be produced efficiently.

In the present invention, the template DNA may be single-stranded or double-stranded. In those cases where the template DNA is single-stranded, a double strand is formed in step (1) or (2) of the method of the present invention. Accordingly, by using the method of the present invention, a double-stranded DNA in which a target site in the template DNA has been modified can be obtained. Further, the template DNA may be either a circular DNA or a linear DNA.

The size of the DNA having an introduced deletion, substitution or addition can vary depending on the technique used in steps (1) and (2), and for example, may be at least 1 kb (1,000 mer), at least 5 kb, at least 8.8 kb, at least 9.5 kb, at least 10 kb, at least 20 kb, at least 30 kb, at least 40 kb, at least 50 kb, at least 60 kb, at least 70 kb, at least 80 kb, at least 90 kb, at least 100 kb, at least 101 kb, at least 183 kb, at least 200 kb, at least 205 kb, at least 300 kb, at least 500 kb, at least 1,000 kb, or at least 2,000 kb. In step (2), in those cases where the RCR method described below is used, modified DNA of a size that cannot be amplified using conventional PCR methods can be obtained, such as a size of at least 50 kb, at least 60 kb, at least 70 kb, at least 80 kb, at least 90 kb, at least 100 kb, at least 101 kb, at least 183 kb, at least 200 kb, at least 205 kb, at least 300 kb, at least 500 kb, at least 1,000 kb, or at least 2,000 kb. On the other hand, there are no particular limitations on the upper limit for the size of the DNA having an introduced deletion, substitution or addition, and for example, a size of about 10,000 kb may be used.

### 1-1. Step (1)

Step (1) in the method of the present invention is a step of modifying the DNA in a cell-free system. A "cell-free system" is a system that does not directly use cells such as E. coli, but instead uses enzymes or the like that exist within various cells such as E. coli. In the present invention, "modification" of the DNA means substitution of a nucleotide in the DNA chain with a different nucleotide, deletion of one or more nucleotides in the DNA chain, insertion of one or more nucleotides between nucleotides on the DNA chain, or addition of one or more nucleotides to an end of the DNA chain. In this description, insertion and addition of a nucleotide are jointly referred to as "addition". Further, the joining of a DNA fragment to another DNA fragment is also included within the definition of "addition".

The number of deleted, substituted or added nucleotides may be any number of one or greater. Although not a limitation, the number of nucleotides is preferably at least 1 nucleotide, at least 2 nucleotides, at least 3 nucleotides, at least 4 nucleotides, at least 5 nucleotides, at least 8 nucleotides, at least 10 nucleotides, at least 12 nucleotides, at least 15 nucleotides, at least 18 nucleotides, or at least 20 nucleotides. Further, the upper limit for the number of deleted, substituted or added nucleotides can vary depending on the size of the template DNA, and for example, may be not more than 5,000 nucleotides, not more than 4,000 nucleotides, not more than 3,000 nucleotides, not more than 2,000 nucleotides, not more than 1,000 nucleotides, not more than 500 nucleotides, not more than 300 nucleotides, not more than 200 nucleotides, not more than 150 nucleotides, not more than 120 nucleotides, not more than 100 nucleotides, not more than 80 nucleotides, not more than 70 nucleotides, not more than 50 nucleotides, or not more than 30 nucleotides. For example, by using the method of the present invention, one or more complete genes may be deleted, substituted or added in the template DNA. Nucleotides may also be deleted, substituted or added at two or more locations simultaneously within a single DNA.

There are no particular limitations on modifying DNA in a cell-free system, and conventional site-specific mutation introduction techniques from the technical field may be used. Further, techniques for joining a plurality of DNAs such as the RA method (see Patent Document 4) may also be used. In a specific example of the RA method, a reaction solution containing two or more types of DNA fragments and a protein having RecA family recombinase activity is prepared. Subsequently, within the reaction solution, the two or more types of DNA fragments are mutually joined at regions in which the base sequences are homologous or at regions in which the base sequences are complementary, thus obtaining a linear or circular DNA.

Examples of the site-specific mutation introduction techniques include methods in which a DNA replication reaction is conducted in the presence of a single-stranded DNA such as an oligonucleotide containing a desired mutation (for example, a substitution, deletion or addition), thereby producing a double-stranded DNA having the introduced mutation. The single-stranded DNA used in the present invention may be produced, for example, by known methods such as chemical synthesis methods. Further, two or more single-stranded DNAs may be used to introduce mutations at two or more locations in a single DNA simultaneously. In this description, a single-stranded DNA containing a mutation used for introducing that mutation is also referred to as a mutation introduction single-stranded DNA.

For example, in a method in which a DNA replication reaction is conducted in the presence of a mutation introduction single-stranded DNA, the single-stranded DNA designed so as to contain a desired mutation undergoes annealing at a single-stranded region exposed in the replication reaction of the template DNA, and is incorporated within a complementary strand. By conducting a further DNA replication reaction using the thus obtained double-stranded DNA as a template, a double-stranded DNA having the mutation in both strands can be obtained.

The DNA replication reaction may use a technique for amplifying the DNA in a cell-free system, such as that described in step (2). In a preferred embodiment, by conducting the DNA replication reaction in the same manner as the amplification reaction in step (2) described below, the mutation introduction of step (1) and the DNA amplification of step (2) can be combined. By combining the mutation introduction and DNA amplification in a cell-free system, introduction of the mutation into the DNA and amplification can be conducted in a single reaction. A DNA replication reaction in a cell-free system, and particularly, using the RCR method to incorporate a single-stranded DNA containing a mutation into a DNA, enabling the introduction of a site-specific mutation, has not previously been known.

In a conventional DNA amplification reaction using a primer, the primer prescribes the amplification region, and when a mutation introduction single-stranded DNA is added to the reaction system, that can also function as a pseudo-primer. As a result, a DNA strand is also synthesized from the single-stranded DNA containing a mutation, and a nick exists between this DNA strand and the DNA synthetic strand that has progressed from the actual primer to a point prior to the single-stranded DNA containing a mutation, meaning the full-length target product cannot be obtained. In order to join the nick, the 5' end of the single-stranded DNA containing a mutation must be phosphorylated and then acted upon by a ligase, meaning the number so steps increases. Accordingly, when a mutation introduction single-stranded DNA is used in the present invention, the DNA replication reaction preferably employs a DNA amplification technique such as the RCR method that does not use a primer.

There are no particular limitations on the length of the single-stranded DNA used for the site-specific mutation introduction, provided that the single-stranded DNA can hybridize with the target site under the DNA replication reaction conditions. The length may be set appropriately in accordance with the method used for the DNA replication reaction, and for example, may be at least 10 bases, at least 15 bases, at least 20 bases, at least 30 bases, at least 40 bases, at least 50 bases, or at least 60 bases, and may be not more than 5,000 bases, not more than 4,000 bases, not more than 3,000 bases, not more than 2,000 bases, not more than 1,000 bases, not more than 500 bases, not more than 100 bases, not more than 80 bases, not more than 75 bases, not more than 70 bases, or not more than 65 bases. The length of the mutation introduction single-stranded DNA may be, of example, from 40 bases to 75 bases, from 50 bases to 70 bases, or from 55 bases to 65 bases. When the mutation introduction single-stranded DNA hybridizes with the target site, because the sequence of the mutation introduction single-stranded DNA and the sequence of the target site do not match completely, only a partial region of the mutation introduction single-stranded DNA need hybridize with the target site. It is preferable that both ends of the mutation introduction single-stranded DNA anneal to target sites of the template DNA. The length of the annealing to the template DNA may differ depending on the length of the single-stranded DNA, and for example, may be at least 5 bases, at least 10 bases, at least 20 bases, at least 30 bases, at least 40 bases, at least 50 bases, at least 60 bases, or at least 100 bases from the end.

There are no particular limitations on the concentration of the mutation introduction single-stranded DNA in the reaction system, provided that the DNA replication reaction is able to proceed. The concentration may be set appropriately in accordance with the method used for the DNA replication reaction, and for example, the concentration relative to the total volume of the DNA replication reaction solution may be at least 0.1 µM (µmol/L), at least 0.15 µM, at least 0.2 µM, at least 0.25 µM, or at least 0.3 µM, and may be not more than 3 µM, not more than 2.5 µM, not more than 2.0 µM, not more than 1.5 µM, not more than 1.0 µM, or not more than 0.6 µM. When the RCR method is used for the DNA replication reaction, the concentration of the mutation introduction single-stranded DNA in the reaction system, relative to the total volume of the DNA replication reaction solution, may be, for example, from 0.1 µM to 1.5 µM, from 0.15 µM to 1.0 µM, from 0.2 µM to 0.6 µM, or from 0.3 µM to 0.6 µM.

In the technical field, a variety of techniques are already known for joining a plurality of DNAs. In one aspect of the present invention, the Recombination Assembly method (hereafter abbreviated as the RA method, see Patent Document 4) can be used to join a plurality of DNAs.

An embodiment in which a plurality of DNAs are joined using the RA method is described below.

### <RA Method>

The RA method is a method in which DNA fragments having regions in which the base sequences are homologous (hereafter sometimes referred to as simply a "homologous region") or regions in which the base sequences are complementary (hereafter sometimes referred to as simply a "complementary region") are mutually joined at the homologous regions or complementary regions to produce a linear or circular DNA. In the RA method, the ligation reaction is conducted in the presence of a RecA family recombinase protein, and therefore the ligation efficiency is extremely good.

In this description, the expression "the base sequences are homologous" means that "the base sequences are identical" whereas the expression "the base sequences are complementary" means that "the base sequences are mutually complementary".

Specifically, in the RA method, a reaction solution is prepared containing two or more types of DNA fragments and a protein having RecA family recombinase activity (hereafter sometimes referred to as a "RecA family recombinase protein"), and the two or more types of DNA fragments are then mutually joined, within the reaction solution, at the regions in which the base sequences are homologous or complementary. This method yields a linear or circular DNA. In the following description, a linear or circular DNA produced by joining two or more DNA fragments is sometimes referred to as a "ligation product".

In the RA method, the DNA fragments that are joined may be linear double-stranded DNA fragments or single-stranded DNA fragments. In other words, linear double-stranded DNA fragments may be joined together, a linear double-stranded DNA fragment and a single-stranded DNA fragment may be joined, or single-stranded DNA fragments may be joined. One or more types of linear double-stranded DNA fragments and one or more types of single-stranded DNA fragments may also be joined. In those cases where linear double-stranded DNA fragments are joined together, or a linear double-stranded DNA fragment and a single-stranded DNA fragment are joined, the two fragments are mutually joined in the homologous region. In those cases where single-stranded DNA fragments are joined, the two fragments are mutually joined in the complementary region.

In those cases where at least one of the DNA fragments being joined by the RA method is a linear double-stranded DNA fragment, the reaction solution mentioned above also contains an exonuclease.

When linear double-stranded DNA fragments are joined by the RA method, first, a 3' → 5' exonuclease acts upon the first linear double-stranded DNA fragment and the second linear double-stranded DNA fragment having homologous regions, converting the homologous regions to single strands. The RecA family recombinase protein then acts upon the single-stranded homologous regions, causing the complementary homologous regions to join together, thus joining the first linear double-stranded DNA fragment and the second linear double-stranded DNA fragment. The trimming of the DNA strand by the 3' → 5' exonuclease may be conducted on only one of the first linear double-stranded DNA fragment and the second linear double-stranded DNA fragment. For example, the homologous region of the first linear double-stranded DNA fragment that has been converted to a single-stranded state may act upon the homologous region of the second linear double-stranded DNA fragment in a double-stranded state, thereby joining the two together.

In the RA method, when linear double-stranded DNA fragments are joined together or a linear double-stranded DNA fragment and a single-stranded DNA fragment are joined, because the double-stranded DNA fragment is first trimmed by an exonuclease to convert the homologous region to a single-stranded form, and the ligation reaction is then conducted in the presence of the RecA family recombinase protein, the ligation efficiency is extremely good. As a result, by using the RA method, the joining of multiple linear double-stranded DNA fragments, which has conventionally been problematic, can be achieved in a single reaction.

In the RA method, in the case where single-stranded DNA fragments are joined together, the RecA family recombinase protein rapidly forms filaments on each of the single-stranded DNA fragments, thus suppressing any digestion by the exonuclease. Subsequently, the action of the RecA family recombinase protein causes binding of the complementary homologous regions, thereby joining the single-stranded DNA fragments.

The number of DNA fragments joined by the RA method may be two (two fragments) or more, and for example, may be at least 4 (4 fragments), at least 5 (5 fragments), at least 7 (7 fragments), at least 10 (10 fragments), or at least 20 (20 fragments). There are no particular limitations on the upper limit for the number of DNA fragments that can be joined by the RA method, but typically, a number of not more than 100 (100 fragments) are joined. In the RA method, by optimizing the reaction conditions and the like, for example, about 50 linear double-stranded DNA fragments can be joined together. The DNA fragments joined in the RA method may be all different DNA fragments, or may include two or more fragments of the same DNA fragment.

The two or more types of DNA fragments joined in the RA method each includes a homologous region or complementary region for joining to at least one other type of DNA fragment. In the RA method, when linear double-stranded DNA fragments are joined together, or a linear double-stranded DNA fragment and a single-stranded DNA fragment are joined, first, one strand of a linear double-stranded DNA fragment is trimmed by an exonuclease to convert the homologous region to a single-stranded state. Accordingly, the homologous region preferably exists at the end of the linear double-stranded DNA fragment, but may also exist near the end. For example, the base at the end portion of the homologous region on the side of the end of the linear double-stranded DNA fragment is preferably within 300 bases of that end, more preferably within 100 bases of the end, even more preferably within 30 bases of the end, and still more preferably within 10 bases of the end. On the other hand, in those cases where single-stranded DNA fragments are joined together, because digestion by the exonuclease is inhibited by filaments of the RecA family recombinase protein, the complementary regions may exist anywhere on the single-stranded DNA fragments.

The base sequences of the homologous regions or complementary regions may be the same base sequences on all of the DNA fragments being joined, but in order to achieve joining in the desired order, the base sequences preferably differ for each type of DNA fragment being joined. For example, in order to join a double-stranded DNA fragment A, a double-stranded DNA fragment B and a double-stranded DNA fragment C in that order, a homologous region a is provided at the downstream end of the double-stranded DNA fragment A and at the upstream end of the double-stranded DNA fragment B, and a homologous region b is provided at the downstream end of the double-stranded DNA fragment B and at the upstream end of the double-stranded DNA fragment C. As a result, the double-stranded DNA fragment A and the double-stranded DNA fragment B are joined at the homologous region a, and the double-stranded DNA fragment B and the double-stranded DNA fragment C are joined at the homologous region b, meaning a linear DNA in which the double-stranded DNA fragment A, the double-stranded DNA fragment B and the double-stranded DNA fragment C are joined in that order can be obtained. In this case, by also providing a homologous region c at the downstream end of the double-stranded DNA fragment C and at the upstream end of the double-stranded DNA fragment A, the double-stranded DNA fragment A and the double-stranded DNA fragment B are joined at the homologous region a, the double-stranded DNA fragment B and the double-stranded DNA fragment C are joined at the homologous region b, and the double-stranded DNA fragment C and the double-stranded DNA fragment A are joined at the homologous region c, meaning a circular DNA in which the double-stranded DNA fragment A, the double-stranded DNA fragment B and the double-stranded DNA fragment C are joined in that order can be obtained.

The homologous regions and complementary regions may have any base sequences that are capable of undergoing specific hybridization of the single strands in the ligation reaction solution, and the base pair length and GC ratio and the like may be determined appropriately with reference to typical probe and primer design methods. Generally, the base pair length of the homologous region must have at least a certain length in order to inhibit non-specific hybridization and enable precise joining of the target linear double-stranded DNA fragments, but if the base pair length of the homologous region is too long, then there is possibility that the ligation efficiency may deteriorate. In the RA method, the base pair length of the homologous region or complementary region is preferably at least 10 base pairs (bp), more preferably at least 15 bp, even more preferably at least 20 bp, and particularly preferably 60 bp or greater. Further, the base pair length of the homologous region or complementary region is preferably not more than 500 bp, more preferably not more than 300 bp, and even more preferably 200 bp or fewer.

In the RA method, there are no particular limitations on the length of the DNA fragments that are joined, and for example, in the case of a linear double-stranded DNA fragment, the length is preferably at least 50 bp, more preferably at least 100 bp, and even more preferably 200 bp or greater. In the case of a single-stranded DNA fragment, the length is preferably at least 50 b, more preferably at least 100 b, and even more preferably 200 b or greater. In the RA method, even double-stranded DNA fragments of 325 kbp can be joined. Furthermore, the lengths of the joined DNA fragments may differ for each fragment.

In the RA method, the linear double-stranded DNA fragments that are joined together may be any double-stranded structure in which either the entire region or a partial region of the homologous region is composed of two hybridized single-stranded DNAs. In other words, the linear double-stranded DNA fragments may be complete linear double-stranded DNA fragments having no gaps or nicks, or may be linear DNA fragments that include a single-stranded structure at one or a plurality of locations. For example, the linear double-stranded DNA fragments that are joined may have blunt ends or sticky ends. By using the RA method, a blunt-ended linear double-stranded DNA fragment and a sticky-ended linear double-stranded DNA fragment can be joined.

The molar ratio between each of the DNA fragments contained in the reaction solution is preferably aligned with the ratio of the number of molecules of each DNA fragment that constitute the target ligation product. By providing the appropriate number of molecules of each DNA fragment in the reaction system at the start of the ligation reaction, the ligation reaction can be conducted more efficiently. For example, in the case where DNA fragments that are all different are joined together, the molar concentrations of the various DNA fragments included in the reaction solution are preferably equal.

There are no particular limitations on the total amount of DNA fragments included in the reaction solution. In order to ensure that a sufficient amount of the ligation product can be readily obtained, the total concentration of DNA fragments included in the reaction solution at the start point of the ligation reaction, relative to the total volume of the reaction solution, is preferably at least 0.01 nM (nmol/L), more preferably at least 0.1 nM, even more preferably at east 0.3 nM, particularly preferably at least 5.09 nM, and most preferably 6.7 nM or greater. In order to achieve higher ligation efficiency more suitable to the joining of multiple fragments, the total concentration of DNA fragments included in the reaction solution at the start point of the ligation reaction is preferably not more than 100 nM, more preferably not more than 96.027 nM, even more preferably not more than 50 nM, particularly preferably not more than 25 nM, and most preferably 20 nM or less.

In the RA method, there are no particular limitations on the size of the ligation product obtained in the ligation reaction. The size of the obtained ligation product is, for example, preferably at least 1 kb (1,000 base length), more preferably at least 5 kb, even more preferably at least 10 kb, particularly preferably at least 13 kb, and most preferably 20 kb or greater. By using the RA method, ligation products with a length of at least 183 kb, preferably at least 208 kb, more preferably at least 300 kb, even more preferably at least 500 kb, and particularly preferably at least 2,000 kb, can also be obtained. On the other hand, although there are no particular limitations on the upper limit for the size of the ligation product obtained in the ligation reaction, the upper limit may, for example, be about 10,000 kb.

The exonuclease used in the RA method is an enzyme that sequentially hydrolyzes from the 3' end or the 5' end of a linear DNA. Provided that the exonuclease used in the RA method has enzymatic activity capable of sequentially hydrolyzing from the 3' end or the 5' end of a linear DNA, there are no particular limitations on the type or biological origin of the exonuclease. For example, examples of enzymes that sequentially hydrolyze from the 3' end (3' → 5' exonucleases) include linear double-stranded DNA-specific 3' → 5' exonucleases such as exonuclease III family AP (apurinic/apyrimidinic) endonucleases, and single-stranded DNA-specific 3' → 5' exonucleases such as DnaQ superfamily proteins. Examples of the exonuclease III family AP endonucleases include exonuclease III (derived from E. coli), ExoA (a bacillus subtilis homolog of exonuclease III), Mth212 (an archaea homolog of exonuclease III), and AP endonuclease I (a human homolog of exonuclease III). Examples of the DnaQ superfamily proteins include exonuclease I (derived from E. coli), exonuclease T (RNase T), exonuclease X, DNA polymerase III epsilon subunit, DNA polymerase I, DNA polymerase II, T7 DNA polymerase, T4 DNA polymerase, Klenow DNA polymerase 5, Phi29 DNA polymerase, ribonuclease III (RNase D), and oligoribonuclease (ORN). Examples of enzymes that sequentially hydrolyze from the 5' end (5' → 3' exonucleases) include λ exonuclease, exonuclease VIII, T5 exonuclease, T7 exonuclease, and RecJ exonuclease.

In terms of achieving a good balance between the processivity of the trimming of linear double-stranded DNA fragments and the ligation efficiency in the presence of the RecA family recombinase protein, the exonuclease used in the RA method is preferably a 3' → 5' exonuclease. Among such exonucleases, linear double-stranded DNA-specific 3' → 5' exonucleases are more preferred, exonuclease III family AP endonucleases are even more preferred, and exonuclease III is particularly desirable.

The exonuclease included in the reaction solution in the RA method preferably includes both a linear double-stranded DNA-specific 3' → 5' exonuclease and a single-stranded DNA-specific 3' → 5' exonuclease. By combining a single-stranded DNA-specific 3' → 5' exonuclease with a linear double-stranded DNA-specific 3' → 5' exonuclease, the ligation efficiency can be further improved compare with the case where a linear double-stranded DNA-specific 3' → 5' exonuclease is used alone. Although the reason for this improvement in the ligation efficiency as a result of combing both types of 3' → 5' exonuclease is not entirely clear, it is surmised that linear double-stranded DNA-specific 3' → 5' exonucleases often have difficulty targeting the 3' protruding end, but this 3' protruding end is digested by the single-stranded DNA-specific 3' → 5' exonuclease, resulting in an acceleration of the ligation reaction of the linear double-stranded DNA-specific 3' → 5' exonuclease and RecA. Further, even in those cases where a linear DNA fragment that is to be joined has a blunt end or a 5' protruding end, using a single-stranded DNA-specific 3' → 5' exonuclease in combination with the double-stranded DNA-specific 3' → 5' exonuclease improves the ligation efficiency, but it is surmised that the 3' protruding end formed secondarily within the ligation product formed by the linear double-stranded DNA-specific 3' → 5' exonuclease and RecA is digested by the single-stranded DNA-specific 3' → 5' exonuclease, resulting in a further improvement in the ligation efficiency.

The concentration of the exonuclease in the ligation reaction solution in the RA method at the start point of the ligation reaction, for example, relative to the total volume of the reaction solution, is preferably from 1 to 1000 mU/µL, more preferably from 5 to 1000 mU/µL, even more preferably from 5 to 500 mU/µL, particularly preferably from 10 to 150 mU/µL, and most preferably from 80 to 150 mU/µL. In particular, in those cases where the exonuclease is a linear double-stranded DNA-specific 3' → 5' exonuclease, the concentration of the linear double-stranded DNA-specific 3' → 5' exonuclease in the reaction solution at the start point of the ligation reaction is, for example, relative to the total volume of the reaction solution, preferably from 5 to 500 mU/µL, more preferably from 5 to 250 mU/µL, even more preferably from 5 to 150 mU/µL, particularly preferably from 10 to 150 mU/µL, and most preferably from 80 to 150 mU/µL. Further, when the exonuclease is a single-stranded DNA-specific 3' → 5' exonuclease, the concentration of the single-stranded DNA-specific 3' → 5' exonuclease, relative to the total volume of the reaction solution, is preferably from 1 to 1,000 mU/µL, more preferably from 100 to 1,000 mU/µL, and even more preferably from 200 to 1,000 mU/µL. When a linear double-stranded DNA-specific 3' → 5' exonuclease and a single-stranded DNA-specific 3' → 5' exonuclease are used in combination, the concentration of each exonuclease in the reaction solution at the start point of the ligation reaction may be set to the preferred concentration of each exonuclease described above.

In this description, the RecA family recombinase protein means a protein which polymerizes on single-stranded or double-stranded DNA to form a filament, has hydrolysis activity relative to nucleoside triphosphates such as ATP (adenosine triphosphate), and has a function (RecA family recombinase activity) of searching for a homologous region and performing homologous recombination. Examples of the RecA family recombinase protein include prokaryotic RecA homologs, bacteriophage RecA homologs, archaeal RecA homologs, and eukaryotic RecA homologs. Examples of the prokaryotic RecA homologs include E. coli RecA; RecA derived from highly thermophilic bacteria including Thermus bacteria such as Thermus thermophiles and Thermus aquaticus, Thermococcus bacteria, Pyrococcus bacteria and Thermotoga bacteria; and RecA derived from radiation-resistant bacteria such as Deinococcus radiodurans. Examples of the bacteriophage RecA homologs include T4 phage UvsX, examples of the archaeal RecA homologs include RadA, and examples of the eukaryotic RecA homologs include Rad51 and paralogs thereof, and Dcm1. The amino acid sequences of these RecA homologs can be obtained from databases such as NCBI (http://www.ncbi.nlm.nih.gov/).

The RecA family recombinase protein used in the RA method may be a wild-type protein, or may be a variant which retains the RecA family recombinase activity in which one or more mutations have been introduced which delete, add or substitute 1 to 30 amino acids in the wild-type protein. Examples of the variants include variants having amino acid substitution mutations that enhance the function of searching for homologous regions in wild-type proteins, variants having various types of tags added to the N-terminus or C-terminus of wild-type proteins, and variants with improved heat resistance (International Patent Publication No. 2016/013592). Examples of tags that may be used include the types of tags widely used in the expression or purification of recombinant proteins such as a His tag, HA (hemagglutinin) tag, Myc tag, and Flag tag. The expression "wild-type RecA family recombinase protein" means a protein having the same amino acid sequence as that of a RecA family recombinase protein held in an organism isolated from the natural world.

The RecA family recombinase protein used in the RA method is preferably a variant that retains the RecA family recombinase activity. Examples of such variants include a F203W mutant in which the phenylalanine of the 203rd amino acid residue of E. coli RecA has been substituted with tryptophan, and mutants among the various RecA homologs in which the phenylalanine corresponding with the 203rd phenylalanine of E. coli RecA has been substituted with tryptophan.

There are no particular limitations on the amount of the RecA family recombinase protein in the ligation reaction solution in the RA method. The concentration of the RecA family recombinase protein in the reaction solution at the start point of the ligation reaction by the RA method, for example, relative to the total volume of the reaction solution, is preferably from 0.01 µM to 100 µM (µmol/L), more preferably from 0.1 µM to 100 µM, even more preferably from 0.1 µM to 50 µM, still more preferably from 0.5 µM to 10 µM, and particularly preferably from 1.0 µM to 5.0 µM.

In order for the RecA family recombinase protein to exhibit RecA family recombinase activity, a nucleoside triphosphate or a deoxynucleotide triphosphate is required. Accordingly, the reaction solution for conducting a ligation reaction in the present invention contains at least one of a nucleoside triphosphate or deoxynucleotide triphosphate. The nucleoside triphosphate included in the ligation reaction solution in the RA method is preferably one or more nucleoside triphosphates selected from the group consisting of ATP, GTP (guanosine triphosphate), CTP (cytidine triphosphate), UTP (uridine triphosphate) and m5UTP (5-methyluridine triphosphate), and the use of ATP is particularly desirable. The deoxynucleotide triphosphate included in the ligation reaction solution in the RA method is preferably one or more deoxynucleotide triphosphates selected from the group consisting of dATP (deoxyadenosine triphosphate), dGTP (deoxyguanosine triphosphate), dCTP (deoxycytidine triphosphate) and dTTP (deoxythymidine triphosphate), and the use of dATP is particularly desirable. There are no particular limitations on the total amount of nucleoside triphosphate and deoxynucleotide triphosphate included in the reaction solution, provided the amount is sufficient to ensure that the RecA family recombinase protein exhibits RecA family recombinase activity. The nucleoside triphosphate concentration or deoxynucleotide triphosphate concentration in the reaction solution in the RA method at the start point of the ligation reaction, for example, relative to the total volume of the reaction solution, is preferably at least 1 µM (µmol/L), more preferably at least 10 µM, even more preferably at least 30 µM, and particularly preferably 100 µM or greater. On the other hand, if the nucleoside triphosphate concentration of the reaction solution is too high, then there is a possibility that the ligation efficiency of multiple fragments may actually deteriorate. Accordingly, the nucleoside triphosphate concentration or deoxynucleotide triphosphate concentration of the reaction solution at the start point of the ligation reaction, relative to the total volume of the reaction solution, is preferably not more than 1,000 µM, more preferably not more than 500 µM, and even more preferably 300 µM or less.

In order for the RecA family recombinase protein to exhibit RecA family recombinase activity and the exonuclease to exhibit exonuclease activity, magnesium ions (Mg²⁺) are required. Accordingly, the reaction solution for conducting the ligation reaction in the RA method must contain a magnesium ion source. The magnesium ion source is a material that provides magnesium ions in the reaction solution. Examples of the magnesium ion source include magnesium salts such as magnesium acetate [Mg(OAc)₂], magnesium chloride [MgCl₂] and magnesium sulfate [MgSO₄]. A preferred magnesium ion source is magnesium acetate.

There are no particular limitations on the magnesium ion source concentration in the ligation reaction solution in the RA method, provided the concentration is sufficient for the RecA family recombinase protein to exhibit RecA family recombinase activity, and for the exonuclease to exhibit exonuclease activity. The magnesium ion source concentration of the reaction solution at the start point of the ligation reaction, for example, relative to the total volume of the reaction solution, is preferably at least 0.5 mM (mmol/L), and is more preferably 1 mM or greater. On the other hand, if the magnesium ion source concentration of the reaction solution is too high, then there is a possibility that the exonuclease activity may become too strong, resulting in a deterioration in the ligation efficiency for multiple fragments. Accordingly, the magnesium ion source concentration of the reaction solution at the start point of the ligation reaction, for example, relative to the toral volume of the reaction solution, is preferably not more than 20 mM, more preferably not more than 15 mM, even more preferably not more than 12 mM, and still more preferably 10 mM or less.

The reaction solution for conducting the ligation reaction in the RA method is prepared, for example, by adding the DNA fragments, the RecA family recombinase protein, the exonuclease, at least one of a nucleoside triphosphate and a deoxynucleotide triphosphate and the magnesium ion source to a buffer solution. There are no particular limitations on the buffer solution, provided it is suitable for use within a range from pH 7 to 9, and preferably at pH 8. Examples of the buffer include Tris-HCl, Tris-acetate (Tris-OAc), Hepes-KOH, phosphate buffer, MOPS-NaOH, and Tricine-HCl. Preferred buffers include Tris-HCl and Tris-OAc. There are no particular limitations on the concentration of the buffer solution, and the concentration may be selected appropriately by a person skilled in the art. In the case of Tris-HCl or Tris-OAc, for example, the concentration of the buffer solution, relative to the total volume of the reaction solution, may be from 10 mM (mmol/L) to 100 mM, preferably from 10 mM to 50 mM, and more preferably 20 mM.

In addition to the DNA fragments, the RecA family recombinant enzyme protein, the exonuclease, at least one of a nucleoside triphosphate and a deoxynucleotide triphosphate, and the magnesium ion source, the reaction solution for conducting the ligation reaction in the RA method preferably also contains a regenerating enzyme for the nucleoside triphosphate or deoxynucleotide triphosphate, and a substrate for that enzyme. By regenerating the nucleoside triphosphate or deoxynucleotide triphosphate in the reaction solution, multiple DNA fragments can be joined more efficiently. Examples of combinations of a regenerating enzyme and a substrate therefor used for regenerating the nucleoside triphosphate or deoxynucleotide triphosphate include a combination of creatine kinase and creatine phosphate, a combination of pyruvate kinase and phosphoenolpyruvate, a combination of acetate kinase and acetyl phosphate, a combination of polyphosphate kinase and polyphosphate, and a combination of nucleoside diphosphate kinase and nucleoside triphosphate. The nucleoside triphosphate used as the substrate (phosphate supply source) for nucleoside diphosphate kinase may be any one of ATP, GTP, CTP and UTP. An additional example of the regenerating enzyme is myokinase.

There are no particular limitations on the concentration of the nucleoside triphosphate regenerating enzyme or the concentration of the substrate for that enzyme in the reaction solution for conducting the ligation reaction in the RA method, provided the concentrations are sufficient to enable regeneration of the nucleoside triphosphate in the reaction solution during the ligation reaction. For example, in the case where creatine kinase and creatine phosphate are used, the concentration of the creatine kinase in the ligation reaction solution in the present invention, relative to the total volume of the reaction solution, is preferably from 1 ng/µL to 1,000 ng/µL, more preferably from 5 ng/µL to 1,000 ng/µL, even more preferably from 5 ng/µL to 500 ng/µL, particularly preferably from 5 ng/µL to 250 ng/µL, and most preferably from 20 ng/µL to 250 ng/µL, and the concentration of the creatine phosphate, relative to the total volume of the reaction solution, is preferably from 0.4 mM to 20 mM (mmol/L), more preferably from 0.4 mM to 10 mM, even more preferably from 1 mM to 7 mM, and particularly preferably from 4 mM to 7 mM.

In the case where multiple fragments are joined in a desired order, the base sequence of the homologous region or complementary region is preferably different for each combination of DNA fragments to be joined. However, under the same temperature conditions, a homologous region having a high content of G (guanine base) and C (cytosine base) tends to more readily form a secondary structure in a single strand, whereas a homologous region having a high content of A (adenine base) and T (thymine base) tends to have low hybridization efficiency, meaning there is a possibility of a reduction in the ligation efficiency. By suppressing secondary structure formation of single-stranded DNA and promoting specific hybridization, the joining of DNA fragments can be promoted.

Accordingly, a substance that suppresses secondary structure formation of single-stranded DNA and promotes specific hybridization is preferably added to the reaction solution for conducting the ligation reaction in the RA method. Examples of this substance include dimethyl sulfoxide (DMSO) and tetramethylammonium chloride (TMAC). DMSO suppresses secondary structure formation of base pairs rich in GC. TMAC promotes specific hybridization. In the RA method, in those cases where this substance that suppresses secondary structure formation of single-stranded DNA and promotes specific hybridization is included in the reaction solution for conducting the ligation reaction, there are no particular limitations on the concentration of the substance, provided the effect of the substance in promoting DNA fragment joining is realized. For example, when DMSO is used as the substance, the concentration of DMSO in the reaction solution for conducting the ligation reaction in the RA method, relative to the total volume of the reaction solution, is preferably from 5% by volume (v/v) to 30% by volume (v/v), more preferably from 8% by volume to 25% by volume, and even more preferably from 8% by volume to 20% by volume. When TMAC is used as the substance, the concentration of TMAC in the reaction solution for conducting the ligation reaction in the RA method is preferably from 60 mM to 300 mM, more preferably from 100 mM to 250 mM, and even more preferably from 100 mM to 200 mM, and a concentration of 150 mM is particularly desirable.

Moreover, a substance having a macromolecular crowding effect is preferably also added to the reaction solution for conducting the ligation reaction in the RA method. The macromolecular crowding effect can enhance the interaction between DNA molecules and promote the joining of DNA fragments. Examples of such substances include polyethylene glycol (PEG) 200 to 20,000, polyvinyl alcohol (PVA) 200 to 20,000, dextran 40 to 70, Ficoll 70, and bovine serum albumin (BSA). In the RA method, in those cases where this substance having a macromolecular crowding effect is included in the ligation reaction solution, there are no particular limitations on the concentration of the substance, provided a DNA fragment ligation promotion effect can be achieved. For example, when PEG 8,000 is used as the substance, the concentration of PEG 8,000 included in the reaction solution for conducting the ligation reaction in the RA method, relative to the total mass of the reaction solution, is preferably from 2% by mass (w/w) to 20% by mass (w/w), more preferably from 2% by mass to 10% by mass, and even more preferably from 4% by mass to 6% by mass.

An alkali metal ion source may also be included in the reaction solution for conducting the ligation reaction in the RA method. The alkali metal ion source is a substance that provides alkali metal ions in the reaction solution. The alkali metal ions introduced into the reaction solution for conducting the ligation reaction in the RA method are preferably sodium ions (Na⁺) or potassium ions (K⁺). Examples of the alkali metal ion source include potassium glutamate [KGlu], potassium aspartate, potassium chloride, potassium acetate [KOAc], sodium glutamate, sodium aspartate, sodium chloride, and sodium acetate. The alkali metal ion source introduced into the reaction solution for conducting the ligation reaction in the RA method is preferably potassium glutamate or potassium acetate, and potassium glutamate is particularly preferred in terms of improving the ligation efficiency for multiple fragments. There are no particular limitations on the concentration of the alkali metal ion source in the reaction solution at the start point of the ligation reaction, and for example, the concentration may be adjusted so that the concentration of alkali metal ions in the reaction solution, relative to the total volume of the reaction solution, is preferably at least 10 mM (mmol/L), and is more preferably within a range from 30 mM to 300 mM, and even more preferably within a range from 50 mM to 150 mM.

A reducing agent may also be added to the reaction solution for conducting the ligation reaction in the RA method. Examples of the reducing agent include dithiothreitol (DTT), β-mercaptoethanol (2-mercaptoethanol), tris (2-carboxyethyl) phosphine (TCEP), and glutathione. A preferred reducing agent is DTT. The reducing agent may be included in the reaction solution at a concentration, relative to the total volume of the reaction solution, within a range from 1.0 mM (mmol/L) to 15.0 mM (mmol/L), preferably from 2.0 mM to 10.0 mM, and more preferably from 4.0 mM to 10.0 mM.

In the RA method, the ligation reaction is performed by incubating the reaction solution, which is prepared by adding, to a buffer solution, two or more types of DNA fragments, a RecA family recombinant enzyme protein, a nucleoside triphosphate and a magnesium ion source, and if necessary, also adding one or more substances selected from the group consisting of an exonuclease, a set of a nucleoside triphosphate regenerating enzyme and a substrate therefor, a substance that suppresses secondary structure formation of single-stranded DNA and promotes specific hybridization, a substance having a macromolecular crowding effect, an alkali metal ion source and a reducing agent, with the incubation performed under isothermal conditions at a temperature at which the RecA family recombinant enzyme protein and the exonuclease in the reaction solution can exhibit their respective enzyme activities. The reaction temperature for the ligation reaction is preferably within a temperature range from 25°C to 48°C, and more preferably within a temperature range from 27°C to 45°C. In particular, in those cases where the length of the homologous region or complementary region is at least 50 bases, the reaction temperature of the ligation reaction is preferably within a temperature range from 30°C to 45°C, more preferably within the temperature range from 37°C to 45°C, even more preferably within a temperature range from 40°C to 43°C, and a temperature of 42°C is particularly desirable. On the other hand, in those cases where the length of the homologous region or complementary region is 50 bases or less, the reaction temperature of the ligation reaction is preferably within a temperature range from 27°C to 43°C, more preferably within a temperature range from 27°C to 37°C, and even more preferably within a temperature range from 27°C to 33°C. In relation to the RA method, the term "isothermal conditions" means that the temperature is maintained within a range of ±3°C or ±1 °C from the set temperature during the reaction. There are no particular limitations on the reaction time of the ligation reaction, and for example, the time is typically from 15 minutes to 6 hours, preferably from 15 minutes to 3 hours, and more preferably from 1 hour to 3 hours.

Gaps and nicks exist in the ligation product (linear or circular DNA) obtained in the ligation reaction. A gap is a state in which one or a plurality of consecutive nucleotides are missing in a double-stranded DNA. A nick is a state in which a phosphodiester linkage between adjacent nucleotides in a double-stranded DNA is cleaved. Accordingly, in the RA method, gaps and nicks in the obtained ligation product are preferably repaired using gap repair enzymes and dNTP following completion of the ligation reaction. By repairing these gaps and nicks, the ligation product can be converted to a complete double-stranded DNA.

Specifically, gaps and nicks in the ligation product can be repaired by adding gap repair enzymes and dNTP to the reaction solution following the ligation reaction, and then incubating the resulting mixture for a predetermined time under isothermal conditions at a temperature at which the gap repair enzymes can exert enzyme activity. There are no particular limitations on the type or biological origin of the enzymes that constitute the gap repair enzymes, provided the enzymes are capable of repairing gaps and nicks in double-stranded DNA. For example, a combination of an enzyme having DNA polymerase activity and an enzyme having DNA ligase activity can be used as the gap repair enzymes. When using a DNA ligase derived from E. coli as the DNA ligase, the cofactor NAD (nicotinamide adenine dinucleotide) is also included in the reaction solution at a concentration level, relative to the total volume of the reaction solution, that is typically from 0.01 mM to 1.0 mM (mmol/L), and preferably about 0.25 mM. The treatment with the gap repair enzymes may be conducted, for example, at 25°C to 40°C, and preferably at 30°C, for a period of 5 minutes to 120 minutes, preferably 10 minutes to 60 minutes, and more preferably 30 minutes.

The term dNTP is a generic term that includes dATP, dGTP, dCTP and dTTP. The concentration of dNTP in the reaction solution at the start point of the repair reaction, for example, relative to the total volume of the reaction solution, may be within a range from 0.01 mM (mmol/L) to 1 mM (mmol/L), preferably within a range from 0.05 mM to 1 mM, and more preferably within a range from 0.25 mM to 1 mM.

In the preparation of the DNA fragments for the RA method, sequence-specific DNA cleavage enzymes such as restriction enzymes and artificial DNA cleavage enzymes may be used. Even in cases where the template DNA is long, and selection of an appropriate restriction enzyme is difficult, if an artificial DNA cleavage enzyme is used, then the DNA can be specifically cleaved at the desired target site.

In this description, the term "artificial DNA cleavage enzyme" describes an enzyme produced artificially that can specifically recognize a desired sequence and cleave the DNA, and includes artificial nucleases and RNA-guided nucleases. Artificial nucleases are artificial enzymes obtained by joining a domain that binds specifically to DNA and a domain that cleaves the DNA, and known examples include zinc finger nucleases (ZFN) and TALE nucleases (TALEN). Further, RNA-guided nucleases are complexes of a short RNA known as a guide RNA (gRNA) that recognizes a target sequence and an enzyme that cleaves the DNA, and known examples include CRISPR-Cas9 and the like. In the present invention, either type of artificial DNA cleavage enzyme may be used, but the use of an artificial nuclease or RNA-guided nuclease is preferred, and the use of CRISPR-Cas9 is more preferred.

In one embodiment that uses an artificial DNA cleavage enzyme in the RA method, step (1) may, for example, include the following steps:
(1-1) a step of cleaving a DNA at a target site to prepare at least one linear DNA by causing the artificial DNA cleavage enzyme to act upon the DNA;
(1-2) a step of preparing a reaction solution containing the linear DNA prepared in step (1-1), one or more types of DNA fragment, and a protein having RecA family recombinase activity; and
(1-3) a step of mutually joining the linear DNA and the one or more types of DNA fragment at regions in which the base sequences are homologous or at regions in which the base sequences are complementary, thereby forming a DNA in which the one or more types of DNA fragment have each been inserted at a target site of the template DNA.

The DNA treated by the artificial DNA cleavage enzyme in step (1-1) (namely, the template DNA) may be linear or circular. By using an artificial DNA cleavage enzyme, the template DNA can be cleaved at the desired target site. The one or more types of DNA fragment in step (1-2) contain a homologous region or complementary region for joining to the cleaved site produced in step (1-1) by the linear DNA and the artificial DNA cleavage enzyme. By mutually joining the homologous regions or complementary regions, the DNA obtained following the joining in step (1-3) has a configuration in which another DNA sequence has been inserted at the cleavage site of the template DNA. Step (1-2) and step (1-3) may be conducted in the same manner as the RA method described above.

In another embodiment, step (1) of the present invention may be a step in which a plurality of DNAs are joined by the In-Fusion method.

The In-Fusion method is a method of conducting a ligation reaction using an In-Fusion enzyme that has a function of recognizing and fusing homologous sequences of the end 15 bases of each double-stranded DNA fragment. Specifically, first, PCR is used to add a homologous region composed of the same base sequence to the ends of the target double-stranded DNA fragments that are to be joined. The two double-stranded DNA fragments with the added homologous region of 15 bases at both ends are mixed with an In-Fusion enzyme and incubated, thereby joining the DNA fragments. The principles of the In-Fusion method are described, for example, in Nucleic Acids Research, 2007, Vol. 35, No. 1, pp. 143 to 151. The In-Fusion method may also be conducted using commercially available reagents such as those manufactured by Takara Bio Inc.

Further, in another embodiment, step (1) of the present invention may be a step in which a plurality of DNAs are joined by the Gibson Assembly method.

The Gibson Assembly method is a method in which the distal region of a first DNA molecule and the proximal region of a second DNA molecule are digested by an enzyme having exonuclease activity, the respective homologous regions (regions having sequence identity of sufficient length to ensure mutual specific hybridization) are converted to single-stranded states, the homologous regions are then specifically annealed and joined, and gaps and nicks are then repaired, thus obtaining a complete double-stranded DNA ligation product. In other words, the Gibson Assembly method comprises the formation of single-stranded 3' overhangs and annealing between the fragments using an exonuclease, the repair of gaps between the annealed fragments using a DNA polymerase, and the joining of nicks using a DNA ligase. The Gibson Assembly method may also be conducted using commercially available reagents such as those manufactured by New England Biolabs Ltd.

### 1-2. Step (2)

Step (2) in the method of the present invention is a step of amplifying the DNA that has been modified in step (1) in a cell-free system, wherein the DNA is amplified under temperature conditions that incubate at a temperature within a range from 20°C to 80°C. Among the various options, the DNA is preferably amplified under temperature conditions that either incubate at a constant temperature, or incubate under a repeating temperature cycle in which incubation is conducted at two temperatures of 65°C or lower, and is more preferably amplified under temperature conditions that either incubate at a fixed temperature within a range from 20°C to 80°C, or incubate under a repeating temperature cycle in which incubation is conducted at two temperatures of 65°C or lower.

Conventional techniques known in the technical field may be used to amplify the DNA in a cell-free system. In the technical field, various techniques are known for amplifying DNA at a constant temperature (for example, see J. Li and J. Macdonald, Biosensors and Bioelectronics, Vol. 64, pp. 196 to 211 (2015)). In one aspect, the DNA may be amplified using the Replication Cycle Reaction method (hereafter referred to as the RCR method, see Patent Documents 5 to 7). In those cases where step (2) is conducted by the RCR method, the template DNA is a circular DNA.

Examples of techniques other than RCR that may be used in the present invention include Helicase-dependent amplification (HAD) (Vincent, M., Xu, Y., Kong, H., 2004. EMBO Rep. 5 (8), 795 to 800), Recombinase polymerase amplification (RPA) (Piepenburg, O., Williams, C.H., Stemple, D.L., Armes, N.A., 2006. PLoS. Biol. 4 (7), e204), Rolling circle amplification (RCA) (Fire, A., Xu, S.Q., 1995. Proc. Natl. Acad. Sci. 92 (10), 4641 to 4645), Ramification amplification (RAM) (Zhang, D.Y., Brandwein, M., Hsuih, T., Li, H.B., 2001. Mol. Diagn. 6 (2), 141 to 150), Multiple displacement amplification (MDA) (Dean, F.B., Nelson, J.R., Giesler, T.L., Lasken, R.S., 2001. Genome Res. 11 (6), 1095 to 1099, and Spits, C., Le Caignec, C., De Rycke, M., Van Haute, L., Van Steirteghem, A., Liebaers, 1., Sermon, K., 2006. Nat. Protoc. 1 (4), 1965 to 1970), and Loop-mediated isothermal amplification (LAMP) (Notomi, T., Okayama, H., Masubuchi, H., Yonekawa, T., Watanabe, K., Amino, N., Hase, T., 2000. Nucleic Acids Res. 28 (12), E63). Any of these methods may be used in the usual manner. The method used may be selected appropriately in accordance with factors such as the form (linear or circular) of the DNA being amplified.

In step (2), in those cases where the DNA having an introduced deletion, substitution or addition is amplified under temperature conditions that involve incubation at a constant temperature, there are no particular limitations on the isothermal conditions, provided the DNA amplification reaction or DNA replication reaction proceeds satisfactorily, but the constant temperature is, for example, a fixed temperature within the optimal temperature range for the DNA polymerase. Examples of the isothermal conditions include a fixed temperature that is at least 20°C, at least 25°C, or at least 30°C, and not more than 80°C, not more than 65°C, not more than 60°C, not more than 50°C, not more than 45°C, not more than 40°C, not more than 35°C, or 33°C or lower. Further, the isothermal conditions may be a fixed temperature included within a range from 20°C to 80°C, a fixed temperature included within a range from 20°C to 65°C, a fixed temperature included within a range from 25°C to 50°C, a fixed temperature included within a range from 25°C to 40°C, a fixed temperature included within a range from 30°C to 33°C, or a temperature of about 30°C. In this description, expressions such as "incubate at a constant temperature", "hold the temperature under isothermal conditions", and "react at a constant temperature" mean that the temperature is held within a temperature range of ±7°C, ±5°C, ±3°C or ±1°C relative to the temperature that has been set for the reaction. The time for which the temperature is held may be set appropriately in accordance with the desired amount of the amplified product of the target circular DNA, but is typically within a range from 1 hour to 24 hours, and preferably from 18 hours to 21 hours.

In step (2), in those cases where the DNA having an introduced deletion, substitution or addition is amplified under temperature conditions that incubate the DNA under a repeating temperature cycle in which incubation is conducted at two temperatures of 65°C or lower, the first temperature is a temperature at which replication initiation of the circular DNA is possible, and the second temperature is a temperature at which replication initiation is inhibited and DNA elongation reactions can proceed. The first temperature may be at least 30°C, and for example, may be within a range from 30°C to 80°C, from 30°C to 50°C, from 30°C to 40°C, or may be 37°C. There are no particular limitations on the incubation conducted at the first temperature, but the time per one cycle may be within a range from 10 seconds to 10 minutes, and is preferably one minute. The second temperature may be not higher than 27°C, and for example, may be within a range from 10°C to 27°C, from 16°C to 25°C, or may be 24°C. There are no particular limitations on the incubation conducted at the second temperature, but the time is preferably set in accordance with the length of the circular DNA being amplified, and for example, the time per one cycle may be within a range from 1 seconds to 10 seconds per 1,000 bases. The number of cycles of the temperature cycle is not particularly limited, and may be from 10 cycles to 50 cycles, from 20 cycles to 45 cycles, from 25 cycles to 45 cycles, or may be 40 cycles.

In the method of the present invention, following step (1), a step may be included for specifically cleaving the DNA into which a deletion, substitution or addition has not been introduced with an artificial DNA cleavage enzyme. By cleaving the DNA into which a deletion, substitution or addition has not been introduced, that DNA is not amplified by the DNA amplification techniques described above, and therefore the yield of the DNA into which a deletion, substitution or addition has been introduced can be improved dramatically. In one embodiment, two or more artificial DNA cleavage enzymes which cleave different sequences may be used. For example, in those cases where two or more template DNAs are used in step (1), two or more artificial DNA cleavage enzymes may be used to specifically cleave each of the template DNAs. Alternatively, in those cases where a single template DNA is used in step (1), but mutations are introduced at two or more locations, two or more artificial DNA cleavage enzymes may be used to specifically cleave sequences in which the mutation has not been introduced at each of the mutation introduction sites.

Here, the expression "specifically cleaving the DNA into which a deletion, substitution or addition has not been introduced" means that the DNA into which a deletion, substitution or addition has not been introduced is cleaved, but the DNA into which a deletion, substitution or addition has been introduced is not cleaved. An artificial DNA cleavage enzyme that performs that type of function can be prepared using normal methods. For example, when CRISPR-Cas9 is used as the artificial DNA cleavage enzyme, by designing a guide RNA that can bind to a region containing the sequence prior to deletion, substitution or addition, the DNA into which a deletion, substitution or addition has not been introduced can be specifically cleaved.

This type of specific cleavage by an artificial DNA cleavage enzyme of the DNA into which a deletion, substitution or addition has not been introduced may be performed after step (1) but before step (2).

These artificial DNA cleavage enzymes are generally readily inactivated by heat treatment, and can sometimes lose functionality in DNA amplification reaction systems that require incubation at a high temperature of 94°C or higher such as that required in the PCR reaction. Step (2) in the method of the present invention does not include incubation at a high temperature of 94°C or higher, and therefore treatment with an artificial DNA cleavage enzyme may be conducted simultaneously with the DNA amplification reaction. Accordingly, in a preferred embodiment, step (2) may be conducted in the presence of an artificial DNA cleavage enzyme that specifically cleaves the DNA into which a deletion, substitution or addition has not been introduced. In such a case, the yield of the DNA into which a deletion, substitution or addition has been improved can be increased dramatically without increasing the number of reaction steps.

An embodiment for amplifying DNA by the RCR method is described below as one aspect of the present invention.

### <RCR Method>

In one embodiment, step (2) of the method of the present invention may be performed using the RCR method. The RCR method is a circular DNA amplification method that includes the following steps:
(2-1) a step of preparing a reaction mixture of: a reaction solution containing (a) a first enzyme group that catalyzes replication of the circular DNA, (b) a second enzyme group that catalyzes an Okazaki fragment ligation reaction and synthesizes two sister circular DNAs that form a catenane, and (c) a third enzyme group that catalyzes a separation reaction of the two sister circular DNAs; and the circular DNA into which a deletion, substitution or addition has been introduced in step (1); and
(2-2) a step of incubating the reaction mixture prepared in step (2-1), either isothermally, namely at a fixed temperature within a range from 20°C to 80°C, or under a repeating temperature cycle in which incubation is conducted at two temperatures of 65°C or lower.

### 1. Circular DNA

The circular DNA used as the template in the RCR method is preferably double-stranded. Examples of the circular DNA used as the template include natural circular DNA such as circular chromosomes of microorganisms, circular DNA formed by joining a separate DNA fragment to a DNA produced by cleaving a natural circular DNA by enzyme treatment or the like, and then circularizing the resulting product, circular DNA produced by subjecting a DNA that exists in a linear state in nature to a circularization treatment, and circular DNA synthesized entirely artificially. The circular DNA may or may not contain a replication origin sequence to which a replication initiation protein can bind, but preferably does contain a replication origin sequence to which a replication initiation protein can bind, and more preferably contains a replication origin sequence (oriC) that can bind to an enzyme having DnaA activity.

Combinations of replication origin sequences and replication initiation proteins that can bind to those sequences are well-known in the technical field (for example, see Cell, Volume 54, Issue 7, Pages 915 to 918 (1988), and any of those combinations may be used in the present invention. Examples of those types of combinations include oriC and an enzyme having DnaA activity (for example, a replication origin sequence that exists in a bacteria such as E. coli or Bacillus subtilis, and DnaA protein), the replication origin sequence of pSC101 and pSC101 repA, the replication origin sequence of PI and PI repA, the replication origin sequence of F and an E protein, the replication origin sequence of R1 and R1 repA, R6K ori γ and π protein, the replication origin sequence of λ and the λ0 protein, the replication origin sequence of ϕ82 and the ϕ820 protein, the replication origin sequence of ϕ80 and the ϕ800 protein, the replication origin sequence of RK2 and the RK2 trfA protein, and the replication origin sequence of P4 and the α protein.

The replication initiation protein and replication origin sequence may be obtained based on the sequence information registered in public databases such as NCBI (http://www.ncbi.nlm.nih.gov/). Further, the replication origin sequence may also be obtained by cloning a DNA fragment capable of binding with the replication initiation protein, and then analyzing the base sequence of that fragment.

The circular DNA used as the template in the present invention may be a circular DNA that already contains a replication origin sequence, or may be a DNA obtained by introducing a replication origin sequence into a circular DNA that does not originally contain a replication origin sequence.

In those cases where the circular DNA used as the template does not contain a replication origin sequence to which a replication initiation protein can bind, replication of the DNA is initiated via formation of a DNA recombination intermediate (D-loop) or formation of a transcriptive intermediate (R-loop).

The circular DNA used as the template in the present invention may also contain, depending on the intended purpose, a drug-resistant marker gene sequence that is resistant to kanamycin, ampicillin or tetracycline or the like.

The circular DNA used as the template in the present invention may be a purified DNA, but may also be in the form of a suspension such as a bacterial cell extract containing the circular DNA. Further, a single type of circular DNA may be used as the template, but a mixture of a plurality of types of circular DNA such as a DNA library may be used in a single test tube as the template.

There are no particular limitations on the amount of template DNA used per one reaction, and a single molecule of the circular DNA may be sued as the template per one reaction.

There are no particular limitations on the length of the circular DNA used as the template in the present invention, and for example, the length may be at least 1 kb (1,000 base length), at least 5 kb (5,000 base length), at least 8 kb (8,000 base length), at least 8.8 kb (8,800 base length), at least 9.5 kb (9,500 base length), at least 10 kb (10,000 base length), at least 50 kb (50,000 base length), at least 100 kb (100,000 base length), at least 101 kb (101,000 base length), at least 183 kb (183,000 base length), at least 200 kb (200,000 base length), at least 205 kb (205,000 base length), at least 500 kb (500,000 base length), at least 1,000 kb (1,000,000 base length), or at least 2,000 kb (2,000,000 base length). On the other hand, although there are no particular limitations on the upper limit for circular DNA, the upper limit may, for example, be set to about 10,000 kb.

In the present invention, using the circular DNA described above as a template, that DNA can be amplified at least 10-fold, 50-fold, 100-fold, 200-fold, 500-fold, 1,000-fold, 2,000-fold, 3,000-fold, 4,000-fold, 5,000-fold or 10,000-fold.

### 2. First, Second and Third Enzyme Groups

### 2-1. First Enzyme Group

In this description, the "first enzyme group" means an enzyme group that catalyzes the replication of the circular DNA.

### 2-1-1. Cases where the Circular DNA Contains a Replication Origin Sequence

An enzyme group disclosed, for example, in Kaguni J M & Kornberg A. Cell. 1984, 38:183-90, may be used as the first enzyme group that catalyzes the replication of the circular DNA. Specifically, examples of the first enzyme group include one or more enzymes or enzyme groups selected from the group consisting of replication initiation proteins (such as enzymes having DnaA activity), one or more types of nucleoid proteins, enzymes or enzyme groups having DNA gyrase activity, single-stranded DNA binding proteins (SSB), enzymes having DNA helicase activity (such as enzymes having DnaB helicase activity), enzymes having DNA helicase loader activity, enzymes having DNA primase activity, enzymes having DNA clamp activity, and enzymes or enzyme groups having DNA polymerase III* activity (but excluding enzymes in which a clamp has been removed from a DNA polymerase III holoenzyme), all of which are described below, or a combination of all of the aforementioned enzymes or enzyme groups.

There are no particular limitations on the biological origins of the replication initiation proteins, provided the protein has initiator activity relative to the replication origin sequence that exists in the circular DNA. Further, there are no particular limitations on the biological origins of the enzymes having DnaA activity, provided the enzyme has initiator activity that is similar to that of the E. coli initiator protein DnaA, and for example, DnaA derived from E. coli can be used favorably. The replication initiation protein may be included as a monomer in the reaction solution at a concentration within a range from 1 nM (nmol/L) to 10 µM (µmol/L) relative to the total volume of the reaction solution, and is preferably included at a concentration within a range from 1 nM to 5 µM, 1 nM to 3 µM, 1 nM to 1.5 µM, 1 nM to 1.0 µM, 1 nM to 500 nM, 50 nM to 200 nM, or 50 nM to 150 nM, and more preferably included at a concentration of 100 nM, although the invention is not limited to this concentration.

The term "nucleoid proteins" refers to proteins contained in a nucleoid. There are no particular limitations on the biological origins of the one or more types of nucleoid proteins used in the present invention, provided the enzyme has a similar activity to an E. coli nucleoid protein, and for example, E. coli-derived IHF, namely a complex (heterodimer or homodimer) of one or more proteins selected from the group consisting of IhfA and IhfB, or E. coli-derived HU, namely a complex of hupA and hupB, can be used favorably. The E. coli-derived IHF hetero or homodimer may be included in the reaction solution at a concentration within a range from 5 nM (nmol/L) to 400 nM relative to the total volume of the reaction solution, and is preferably included at a concentration within a range from 5 nM to 200 nM, 5 nM to 100 nM, 5 nM to 50 nM, 10 nM to 50 nM, 10 nM to 40 nM or 10 nM to 30 nM, and more preferably included at a concentration of 20 nM, although the invention is not limited to this concentration. The E. coli-derived HU may be included in the reaction solution at a concentration within a range from 1 nM to 50 nM, and is preferably included at a concentration within a range from 5 nM to 50 nM or 5 nM to 25 nM, although the invention is not limited to these ranges.

There are no particular limitations on the biological origins of the enzymes or enzyme groups having DNA gyrase activity, provided the enzyme has activity that is similar to that of the DNA gyrase of E. coli. For example, a complex of E. coli-derived GyrA and GyrB can be used favorably. The complex of E. coli-derived GyrA and GyrB may be included as a heterotetramer in the reaction solution in a concentration, relative to the total volume of the reaction solution, within a range from 20 nM (nmol/L) to 500 nM, and preferably within a range from 20 nM to 400 nM, 20 nM to 300 nM, 20 nM to 200 nM, 50 nM to 200 nM, or 50 nM to 100 nM, and is more preferably included at a concentration of 50 nM, although the invention is not limited to this concentration.

There are no particular limitations on the biological origins of the single-stranded binding proteins (SSB), provided the enzyme has activity that is similar to that of the single-stranded binding protein of E. coli, but for example, E. coli-derived SSB can be used favorably. The E. coli-derived SSB may be included as a homotetramer in the reaction solution in a concentration, relative to the total volume of the reaction solution, within a range from 20 nM (nmol/L) to 1,000 nM, and preferably within a range from 20 nM to 500 nM, 20 nM to 300 nM, 20 nM to 200 nM, 50 nM to 500 nM, 50 nM to 400 nM, 50 nM to 300 nM, 50 nM to 200 nM, 50 nM to 150 nM, 100 nM to 500 nM or 100 nM to 400 nM, and is more preferably included at a concentration of 400 nM, although the invention is not limited to this concentration.

There are no particular limitations on the biological origins of the enzymes having DnaB helicase activity, provided the enzyme has activity that is similar to that of the DnaB of E. coli, but for example, E. coli-derived DnaB can be used favorably. The E. coli-derived DnaB may be included as a homohexamer in the reaction solution in a concentration, relative to the total volume of the reaction solution, within a range from 5 nM (nmol/L) to 200 nM, and preferably within a range from 5 nM to 100 nM, 5 nM to 50 nM, or 5 nM to 30 nM, and is more preferably included at a concentration of 20 nM, although the invention is not limited to this concentration.

There are no particular limitations on the biological origins of the enzymes having DNA helicase loader activity, provided the enzyme has activity that is similar to that of the DnaC of E. coli, but for example, E. coli-derived DnaB can be used favorably. The E. coli-derived DnaC may be included as a homohexamer in the reaction solution in a concentration, relative to the total volume of the reaction solution, within a range from 5 nM (nmol/L) to 200 nM, and preferably within a range from 5 nM to 100 nM, 5 nM to 50 nM, or 5 nM to 30 nM, and is more preferably included at a concentration of 20 nM, although the invention is not limited to this concentration.

There are no particular limitations on the biological origins of the enzymes having DNA primase activity, provided the enzyme has activity that is similar to that of the DnaG of E. coli, but for example, E. coli-derived DnaG can be used favorably. The E. coli-derived DnaG may be included as a monomer in the reaction solution in a concentration, relative to the total volume of the reaction solution, within a range from 20 nM (nmol/L) to 1,000 nM, and preferably within a range from 20 nM to 800 nM, 50 nM to 800 nM, 100 nM to 800 nM, 200 nM to 800 nM, 250 nM to 800 nM, 250 nM to 500 nM, or 300 nM to 500 nM, and is more preferably included at a concentration of 400 nM, although the invention is not limited to this concentration.

There are no particular limitations on the biological origins of the enzymes having DNA clamp activity, provided the enzyme has activity that is similar to that of the DnaN of E. coli, but for example, E. coli-derived DnaN can be used favorably. The E. coli-derived DnaG may be included as a homodimer in the reaction solution in a concentration, relative to the total volume of the reaction solution, within a range from 10 nM (nmol/L) to 1,000 nM, and preferably within a range from 10 nM to 800 nM, 10 nM to 500 nM, 20 nM to 500 nM, 20 nM to 200 nM, 30 nM to 200 nM, or 30 nM to 100 nM, although the invention is not limited to this concentration range.

There are no particular limitations on the biological origins of the enzymes or enzyme groups having DNA polymerase III* activity, provided the enzyme or enzyme group has activity that is similar to that of a DNA polymerase III* complex of E. coli, and for example, enzyme groups containing any of E. coli-derived DnaX, HolA, HolB, HolC, HolD, DnaE, DnaQ and HolE, preferably enzyme groups containing a complex of E. coli-derived DnaX, HolA, HolB and DnaE, and more preferably enzyme groups containing a complex of E. coli-derived DnaX, HolA, HolB, HolC, HolD, DnaE, DnaQ and HolE, can be used favorably. The E. coli-derived DNA polymerase III* complex may be included as a heteromultimer in the reaction solution in a concentration, relative to the total volume of the reaction solution, within a range from 2 nM (nmol/L) to 50 nM, and preferably within a range from 2 nM to 40 nM, 2 nM to 30 nM, 2 nM to 20 nM, 5 nM to 40 nM, 5 nM to 30 nM, or 5 nM to 20 nM, and is more preferably included at a concentration of 5 nM, although the invention is not limited to this concentration.

### 2-1-2. Cases where the Circular DNA does not Contain a Replication Origin Sequence

In those cases where the circular DNA does not contain a replication origin sequence and a D-loop is used for replication initiation, examples of enzymes that may be used for the first enzyme group include recombination enzymes (such as RecA or homologs thereof (such as UvsX or T4 phage)), enzymes having a function of introducing a recombination enzyme into DNA (such as RecO, RecR and homologs thereof (such as UvsY to UvsX)), and primosome protein groups having the function of helicase introduction into D-loop (such as PriA, PriB, PricC and DnaT).

In those cases where the circular DNA does not contain a replication origin sequence and an R-loop is used for replication initiation, examples of the enzymes that may be used for the first enzyme group include RNA polymerases, and primosome protein groups having the function of helicase introduction into R-loop (such as PriA, PriB, PricC and DnaT).

### 2-2. Second Enzyme Group

In this description, the "second enzyme group" means an enzyme group that catalyzes an Okazaki fragment ligation reaction and synthesizes two sister circular DNAs that form a catenane.

In the present invention, the two sister circular DNAs that form a catenane describe a state in which two circular DNAs synthesized by the DNA replication reaction are linked.

Examples of the second enzyme group that catalyzes an Okazaki fragment ligation reaction and synthesizes two sister circular DNAs that form a catenane include one or more enzymes selected from the group consisting of enzymes having DNA polymerase I activity, enzymes having DNA ligase activity, and enzymes having RNaseH activity, or a combination of these enzymes.

There are no particular limitations on the biological origins of the enzymes having DNA polymerase I activity, provided the enzyme has activity that is similar to that of the DNA polymerase I of E. coli, but for example, E. coli-derived DNA polymerase I can be used favorably. The E. coli-derived DNA polymerase I may be included as a monomer in the reaction solution in a concentration, relative to the total volume of the reaction solution, within a range from 10 nM (nmol/L) to 200 nM (nmol/L), and preferably within a range from 20 nM to 200 nM, 20 nM to 150 nM, 20 nM to 100 nM, 40 nM to 150 nM, 40 nM to 100 nM, or 40 nM to 80 nM, and is more preferably included at a concentration of 50 nM, although the invention is not limited to this concentration.

There are no particular limitations on the biological origins of the enzymes having DNA ligase activity, provided the enzyme has activity that is similar to that of the DNA ligase of E. coli, but for example, E. coli-derived DNA ligase or the DNA ligase of T4 phage can be used favorably. The E. coli-derived DNA ligase may be included as a monomer in the reaction solution in a concentration, relative to the total volume of the reaction solution, within a range from 10 nM (nmol/L) to 200 nM (nmol/L), and preferably within a range from 15 nM to 200 nM, 20 nM to 200 nM, 20 nM to 150 nM, 20 nM to 100 nM, or 20 nM to 80 nM, and is more preferably included at a concentration of 50 nM, although the invention is not limited to this concentration.

There are no particular limitations on the biological origins of the enzymes having RNaseH activity, provided the enzyme has activity that decomposes the RNA chain of an RNA:DNA hybrid, but for example, E. coli-derived RNaseH can be used favorably. The E. coli-derived RNaseH may be included as a monomer in the reaction solution in a concentration, relative to the total volume of the reaction solution, within a range from 0.2 nM (nmol/L) to 200 nM (nmol/L), and preferably within a range from 0.2 nM to 200 nM, 0.2 nM to 100 nM, 0.2 nM to 50 nM, 1 nM to 200 nM, 1 nM to 100 nM, 1 nM to 50 nM, or 10 nM to 50 nM, and is more preferably included at a concentration of 10 nM, although the invention is not limited to this concentration.

### 2-3. Third Enzyme Group

In this description, the "third enzyme group" means an enzyme group that catalyzes a separation reaction of the two sister circular DNAs.

Examples of the third enzyme group that catalyzes the separation reaction of the two sister circular DNAs include those enzyme groups disclosed, for example, in Peng H & Marians K J. PNAS. 1993, 90: 8571 to 8575. Specific examples of the third enzyme group include one or more enzymes selected from the group consisting of enzymes having topoisomerase IV activity, enzymes having topoisomerase III activity, and enzymes having RecQ helicase activity, all of which are described below; or a combination of these enzymes.

There are no particular limitations on the biological origins of the enzymes having topoisomerase III activity, provided the enzyme has activity similar to that of the topoisomerase III of E coli, but for example, E. coli-derived topoisomerase III can be used favorably. The E. coli-derived topoisomerase III may be included as a monomer in the reaction solution in a concentration, relative to the total volume of the reaction solution, within a range from 20 nM (nmol/L) to 500 nM (nmol/L), and preferably within a range from 20 nM to 400 nM, 20 nM to 300 nM, 20 nM to 200 nM, 20 nM to 100 nM, or 30 nM to 80 nM, and is more preferably included at a concentration of 50 nM, although the invention is not limited to this concentration.

There are no particular limitations on the biological origins of the enzymes having RecQ helicase activity, provided the enzyme has activity similar to that of the RecQ of E coli, but for example, E. coli-derived RecQ can be used favorably. The E. coli-derived RecQ may be included as a monomer in the reaction solution in a concentration, relative to the total volume of the reaction solution, within a range from 20 nM (nmol/L) to 500 nM (nmol/L), and preferably within a range from 20 nM to 400 nM, 20 nM to 300 nM, 20 nM to 200 nM, 20 nM to 100 nM, or 30 nM to 80 nM, and is more preferably included at a concentration of 50 nM, although the invention is not limited to this concentration.

There are no particular limitations on the biological origins of the enzymes having topoisomerase IV activity, provided the enzyme has activity similar to that of the topoisomerase IV of E coli, but for example, E. coli-derived topoisomerase IV, which is a complex of ParC and ParE, can be used favorably. The E. coli-derived topoisomerase IV may be included as a tetramer in the reaction solution in a concentration, relative to the total volume of the reaction solution, within a range from 0.1 nM (nmol/L) to 50 nM (nmol/L), and preferably within a range from 0.1 nM to 40 nM, 0.1 nM to 30 nM, 0.1 nM to 20 nM, 1 nM to 40 nM, 1 nM to 30 nM, 1 nM to 20 nM, 1 nM to 10 nM, or 1 nM to 5 nM, and is more preferably included at a concentration of 5 nM, although the invention is not limited to this concentration.

The first, second and third enzyme groups described above may use commercially available products, or may be extracted from microorganisms or the like and then purified as required. Extraction of enzymes from microorganisms and subsequent purification may be conducted appropriately using techniques widely usable by those skilled in the art.

When enzymes other than the E. coli-derived enzymes described above are used as the first, second and third enzyme groups, these other enzymes may be used within a concentration range that yields an enzyme activity unit equivalent to that obtained for the concentration range specified above for the E. coli-derived enzymes.

The reaction solution containing a cell-free protein expression system for the above enzymes may simply be mixed with the circular DNA that represents the template, thus forming a reaction mixed liquid for replication or amplification of the circular DNA. The cell-free protein expression system may be a cell-free translation system in which a total RNA, mRNA or in vitro transcription product or the like containing an RNA composed of a sequence complementary to the base sequence of a gene that encodes each of the above enzymes functions as the template RNA, or may be a cell-free transcription-translation system in which a gene that encodes each enzyme or an expression vector containing a gene that encodes each enzyme functions as the template DNA.

Embodiments for amplifying circular DNA using the RCR method are described below in RCR methods I to V. In these RCR methods I to V, the terms "replication origin sequence that can bind to an enzyme having DnaA activity " and "oriC" may be read to mean "replication origin sequence", and the term "enzyme having DnaA activity" may be read to mean "replication initiation protein that can bind to a replication origin sequence".

### <RCR Method I>

In one embodiment, the RCR method in the present invention includes a step of forming a reaction mixture of a reaction solution containing the aforementioned first through third enzyme groups, and a circular DNA that represents the template (see Patent Document 5).

There are no particular limitations on the composition of the reaction solution containing the first through third enzyme groups, provided the DNA replication reaction is able to proceed, and for example, a solution prepared by adding, to a buffer solution such as a Tris-HCl buffer solution, a solution prepared by adding the first through third enzyme group to a solution containing rNTP, dNTP, a magnesium ion source, and an ATP source and the like, may be used. Further, the reaction solution may also contain additional components such as components that inhibit the production of secondary products. Specific examples of the reaction solution are exemplified in the examples described below.

The RCR method 1 also include a step of holding the temperature of the above reaction mixture under isothermal conditions. There are no particular limitations on the isothermal conditions, provided the DNA replication reaction can proceed, and for example, the temperature may be set to a constant temperature within the range from 20 to 80°C that represents the optimal temperature for DNA polymerase, may be set to a constant temperature within a range from 25°C to 50°C, or may be set to about 30°C to 33°C. The time for which the temperature is held may be set appropriately in accordance with the amount of the amplified product of the circular DNA that represents the target, and for example, may be set to a time within a range from 1 hour to 24 hours, or a time of 16 hours to 21 hours.

Depending on the intended purpose, the RCR method I may also include a step of purifying the amplified product of the target circular DNA after the above step of holding the temperature of the reaction mixture under isothermal conditions. Purification of the circular DNA may be conducted appropriately using methods typically usable by those skilled in the art.

The circular DNA that has been amplified using the RCR method I may be used for subsequent purposes such as transformation either in the form of the reaction mixture obtained following reaction, or in an appropriately purified form.

### <RCR Method II>

In one embodiment, the RCR method in the present invention includes the following step:
(II-1) a step of forming a reaction mixture of a circular DNA that represents the template, and a reaction solution containing:
a first enzyme group that catalyzes replication of the circular DNA,
a second enzyme group that catalyzes an Okazaki fragment ligation reaction and synthesizes two sister circular DNAs that form a catenane,
a third enzyme group that catalyzes a separation reaction of the two sister circular DNAs,
a buffer solution,
ATP,
GTP, CTP and UTP,
dNTP,
a magnesium ion source, and
an alkali metal ion source, wherein the circular DNA contains a replication origin sequence (origin of chromosome (oriC)) that can bind to an enzyme having DnaA activity (see Patent Document 6).

In this embodiment, the method of the present invention may also include, prior to the step (II-1) described above, a step of preincubating the reaction solution. In other words, the method of the present invention may include the following steps:
(II-1-1) a step of preincubating a reaction solution containing:
   a first enzyme group that catalyzes replication of the circular DNA,
   a second enzyme group that catalyzes an Okazaki fragment ligation reaction and synthesizes two sister circular DNAs that form a catenane,
   a third enzyme group that catalyzes a separation reaction of the two sister circular DNAs,
   a buffer solution,
   ATP,
   GTP, CTP and UTP,
   dNTP,
   a magnesium ion source, and
   an alkali metal ion source; and
(II-1-2) a step of forming a reaction mixture of the above reaction solution and a circular DNA that represents the template, wherein the circular DNA contains a replication origin sequence (origin of chromosome (oriC)) that can bind to an enzyme having DnaA activity.

The preincubation may be conducted by holding the temperature, for example, at a temperature within a range from 0°C to 40°C, 10°C to 40°C, 15°C to 37°C or 16°C to 30°C, for a period within a range from 5 minutes to 60 minutes, 5 minutes to 45 minutes, 5 minutes to 30 minutes, 15 minutes to 60 minutes, 15 minutes to 45 minutes, or 15 minutes to 30 minutes. Provided the preincubation is conducted with the temperature of the reaction solution maintained within the above temperature range, the temperature may vary slightly during the preincubation.

Although not constrained by theory, in the RCR method II, the replication cycle is usually repeated, thereby amplifying the circular DNA exponentially. In the method of the present invention, the circular DNA mentioned above is used as a template, and that DNA can be amplified at least 10-fold, 50-fold, 100-fold, 200-fold, 500 fold, 1,000-fold, 2,000-fold, 3,000-fold, 4,000-fold, 5,000-fold, or 10,000-fold.

The circular DNA that is mixed with the reaction solution is as described above in the section entitled "1. Circular DNA". There are no particular limitations on the amount of the template DNA used per reaction, and for example, the template DNA may be added to the reaction solution at a concentration, relative to the total volume of the reaction solution at the start of the reaction, of not more than 10 ng/µL, not more than 5 ng/µL, not more than 1 ng/µL, not more than 0.8 ng/µL, not more than 0.5 ng/µL, or 0.3 ng/µL or less. On the other hand, the lower limit for the amount of the template DNA used per reaction is not particularly limited, and for example, may be 7.5 fg/µL, 0.67 pg/µL, 1 pg/µL, 10 pg/µL. 14 pg/µL, 50 pg/µL, or 75 pg/µL. Moreover, at the start of the reaction, a single molecule of the circular DNA per reaction may be introduced as the template and then used in the amplification.

There are no particular limitations on the buffer solution included in the reaction solution, provided the buffer solution is suitable for used within a pH range of 7 to 9, and preferably pH 8. Examples of the buffer solution include Tris-HCl, Tris-OAc, Hepes-KOH, phosphate buffer solution, MOPS-NaOH, and Tricine-HCL. Preferred buffer solutions include Tris-HCl and Tris-OAc. There are no particular limitations on the concentration of the buffer solution, which may be selected appropriately by a person skilled in the art, but in the case of Tris-HCl or Tris-OAc, the concentration of the buffer solution, for example, relative to the total volume of the reaction solution, may be selected within a range from 10 mM (mmol/L) to 100 mM (mmol/L) or from 10 mM to 50 mM, or may be 20 mM.

ATP means adenosine triphosphate. The concentration of ATP included in the reaction solution at the start of the reaction, for example, relative to the total volume of the reaction solution, may be within a range from 0.1 mM (mmol/L) to 3 mM (mmol/L), is preferably within a range from 0.1 mM to 2 mM, from 0.1 mM to 1.5 mM, or from 0.5 mM to 1.5 mM, and is more preferably 1 mM.

GTP, CTP and UTP mean guanosine triphosphate, cytidine triphosphate and uridine triphosphate respectively. The concentration of each of GTP, CTP and UTP included in the reaction solution at the start of the reaction, for example, relative to the total volume of the reaction solution, may be within a range from 0.1 mM (mmol/L) to 3.0 mM (mmol/L), and is preferably within a range from 0.5 mM to 3.0 mM, or from 0.5 mM to 2.0 mM.

Moreover, dNTP is a generic term for deoxyadenosine triphosphate (dATP), deoxyguanosine triphosphate (dGTP), deoxycytidine triphosphate (dCTP) and deoxythymidine triphosphate (dTTP). The concentration of dNTP included in the reaction solution at the start of the reaction, for example, relative to the total volume of the reaction solution, may be within a range from 0.01 mM (mmol/L) to 1 mM (mmol/L), is preferably within a range from 0.05 mM to 1 mM, or from 0.1 mM to 1 mM, and is more preferably within a range from 0.25 mM to 1 mM.

The magnesium ion source is a substance that provides magnesium ions (Mg²⁺) in the reaction solution. Examples of the magnesium ion source include Mg(OAc)₂, MgCl₂ and MgSO₄. Mg(OAc)₂ is a preferred magnesium ion source. The concentration of the magnesium ion source included in the reaction solution at the start of the reaction, relative to the total volume of the reaction solution, may be a concentration which provides, for example, from 5 mM (mmol/L) to 50 mM (mmol/L) of magnesium ions, and preferably 1 mM of magnesium ions, in the reaction solution.

The alkali metal ion source is a substance that provides alkali metal ions in the reaction solution. Examples of the alkali metal ions include sodium ions (Na⁺) and potassium ions (K⁺). Examples of the alkali metal ion source include potassium glutamate, potassium aspartate, potassium chloride, potassium acetate, sodium glutamate, sodium aspartate, sodium chloride, and sodium acetate. Preferred alkali metal ion sources include potassium glutamate and potassium acetate. The concentration of the alkali metal ion source included in the reaction solution at the start of the reaction, relative to the total volume of the reaction solution, may be a concentration which provides at least 100 mM (mmol/L), preferably from 100 mM to 300 mM, and more preferably 50 mM, of alkali metal ions in the reaction solution, although the invention is not limited to this concentration. In combination with an earlier application, 150 mM may be subtracted from the above concentration of the alkali metal ion source.

The reaction solution used in the RCR II method may also contain a protein non-specific adsorption inhibitor or a nucleic acid non-specific adsorption inhibitor. It is preferable that the reaction solution contains both a protein non-specific adsorption inhibitor and a nucleic acid non-specific adsorption inhibitor. By including one or more non-specific adsorption inhibitors selected from the group consisting of protein non-specific adsorption inhibitors and nucleic acid non-specific adsorption inhibitors in the reaction solution, the reaction efficiency improves. It is thought that the one or more non-specific adsorption inhibitors selected from the group consisting of protein non-specific adsorption inhibitors and nucleic acid non-specific adsorption inhibitors inhibit at least one type of non-specific adsorption selected from the group consisting of adsorption between proteins and adsorption between a protein and a circular DNA, and also inhibit adhesion of proteins and the circular DNA to the surfaces of the reaction vessel, thereby improving the reaction efficiency.

Protein non-specific adsorption inhibitors are proteins that are unrelated to the amplification reaction in the method of the present invention. Examples of these types of proteins include bovine serum albumin (BSA), lysozyme, gelatin, heparin and casein. The protein non-specific adsorption inhibitor may be included in the reaction solution in a concentration, relative to the total volume of the reaction solution, that is within a range from 0.02 mg/mL to 2.0 mg/mL, preferably within a range from 0.1 mg/mL to 2.0 mg/mL, 0.2 mg/mL to 2.0 mg/mL, or 0.5 mg/mL to 2.0 mg/mL, and more preferably at a concentration of 0.5 mg/mL, although the invention is not limited to this concentration.

Nucleic acid non-specific adsorption inhibitors are nucleic acid molecules or nucleic acid-related molecules that are unrelated to the amplification reaction in the method of the present invention. Examples of these types of nucleic acid molecules or nucleic acid-related molecules include tRNA (transfer RNA), rRNA (ribosomal RNA), mRNA (messenger RNA), glycogen, heparin, oligo DNA, poly(I-C) polyinosine-polycytidine, poly(dI-dC) (polydeoxyinosine-polydeoxycytidine), poly(A) (polyadenine), and poly(dA) (polydeoxyadenine). The nucleic acid non-specific adsorption inhibitor may be included in the reaction solution in a concentration, relative to the total volume of the reaction solution, that is within a range from 1 ng/µL to 500 ng/µL, preferably within a range from 10 ng/µL to 500 ng/µL, 10 ng/µL to 200 ng/µL, or 10 ng/µL to 100 ng/µL, and more preferably at a concentration of 50 ng/µL, although the invention is not limited to this concentration. In combination with an earlier application, in those cases where tRNA is selected as the nucleic acid non-specific adsorption inhibitor, 50 ng/µL may be subtracted from the above concentration of tRNA.

The reaction solution used in the method of the present invention may also contain a DNA stabilizing factor. It is thought that by including a DNA stabilizing factor in the reaction solution, cleavage of the DNA is suppressed, enabling the template DNA and the amplified product to be protected. Addition of a DNA stabilizing factor leads to an improvement in the yield of the target product. Particularly in those cases where the template DNA is a long-chain circular DNA, the template DNA and the amplified product are more prone to decomposition, and therefore addition of a DNA stabilizing factor is beneficial. The DNA stabilizing factor is not particularly limited, and for example, may be selected from the group consisting of glucose, sucrose, dimethyl sulfoxide (DMSO), bovine serum albumin (BSA), glycol ether diamine tetraacetate (EGTA), disodium bathocuproine disulfonate (BDA), penicillamine, Tiron (1,2-dihydroxybenzene-3,5-sulfonate), diethylenetriamine pentaacetate (DTPA), ethylenediamine tetraacetate (EDTA), Dps protein (derived from E. coli), and metallothionein protein (derived from humans). Among these, DTPA, Tiron, BDA, Dps protein and BSA have an action that enhances the efficiency of the circular DNA amplification reaction, and are therefore particularly preferred. DTPA and Tiron may be included in the reaction solution in a concentration, relative to the total volume of the reaction solution, within a range from 0.01 mM (mmol/mL) to **0.3** mM (mmol/mL), preferably within a range from 0.05 mM to 0.25 mM, and more preferably at a concentration of 0.25 mM, although the invention is not limited to this concentration. BDA may be included in the reaction solution in a concentration, relative to the total volume of the reaction solution, within a range from **0.01 mM (mmol/mL) to 0.5 mM (mmol/mL),** and preferably within a range from 0.05 mM to 0.3 mM, although the invention is not limited to this concentration range. The Dps protein may be included in the reaction solution in a concentration, relative to the total volume of the reaction solution, within a range from 0.3 µM (µmol/mL) to 3.0 mM (µmol/mL), and preferably within a range from 0.3 µM to 1.5 µM, although the invention is not limited to this concentration range. BSA may be included in the reaction solution in a concentration, relative to the total volume of the reaction solution, within a range from 0.02 mg/mL to 2.0 mg/mL, preferably within a range from 0.1 mg/mL to 2.0 mg/mL, from 0.2 mg/mL to 2.0 mg/mL, or from 0.5 mg/mL to 2.0 mg/mL, and is more preferably included at a concentration of 0.5 mg/mL, although the invention is not limited to this concentration.

The reaction solution used in the RCR II method may also contain a linear DNA-specific exonuclease or a RecG helicase. The reaction solution preferably also contains both a linear DNA-specific exonuclease and a RecG helicase. By including one or more substances selected from the group consisting of linear DNA-specific exonucleases and RecG helicases in the reaction solution, the amount of linear DNA produced by double strand cleavage or the like in the amplification reaction can be reduced, resulting in an effect of improving the yield of the target supercoiled product.

The reaction solution used in the RCR II method may also contain a RecG helicase or a single-stranded DNA-specific exonuclease. The reaction solution preferably also contains both a RecG helicase and a single-stranded DNA-specific exonuclease. By including one or more substances selected from the group consisting of RecG helicases and single-stranded DNA-specific exonucleases in the reaction solution, the amount of low-molecular weight secondary amplification products produced in the amplification reaction can be reduced, resulting in an effect of improving the yield of the target supercoiled product.

The reaction solution used in the RCR II method may also contain a linear DNA-specific exonuclease or a single-stranded DNA-specific exonuclease. The reaction solution preferably also contains both a linear DNA-specific exonuclease and a single-stranded DNA-specific exonuclease. By including one or more substances selected from the group consisting of linear DNA-specific exonucleases and single-stranded DNA-specific exonucleases in the reaction solution, the amount of linear DNA produced by double strand cleavage or the like in the amplification reaction can be reduced, resulting in an effect of improving the yield of the target supercoiled product.

Linear DNA-specific exonucleases are enzymes that sequentially hydrolyze the 5' end or the 3' end of a linear DNA. Provided that the linear DNA-specific exonuclease has activity capable of sequentially hydrolyzing from the 5' end or the 3' end of a linear DNA, there are no particular limitations on the type or biological origin of the exonuclease. For example, RecBCD, λ exonuclease, exonuclease III, exonuclease VIII, T5 exonuclease, T7 exonuclease, and Plasmid-SafeTM ATP-Dependent DNase (epicentre) and the like may be used. A preferred linear DNA-specific exonuclease is RecBCD. The linear DNA exonuclease may be included in the reaction solution in a concentration, relative to the total volume of the reaction solution, within a range from 0.001 U/µL to 1.0 U/µL, preferably within a range from 0.005 U/µL to 1.0 U/µL, from 0.01 U/µL to 1.0 U/µL, from 0.05 U/µL to 1.0 U/µL, from 0.08 U/µL to 1.0 U/µL, or from 0.1 U/µL to 1.0 U/µL, although the invention is not limited to this concentration range. The enzyme activity unit (U) of the linear DNA exonuclease is a unit indicating enzymatic activity, wherein the amount of enzyme required to make 1 nmol of deoxyribonucleotide of the linear DNA soluble in acid in a reaction at 37°C for 30 minutes is deemed to be 1 U.

The RecG helicase is an enzyme that is thought to be a helicase that resolves the problem of secondary DNA structures generated as a result of collision between replication forks during termination of the elongation reaction. There are no particular limitations on the biological origin of the RecG helicase, provided the enzyme has activity that is similar to that of the E. coli-derived RecG, and for example, the E. coli-derived RecG can be used favorably. The E. coli-derived RecG may be included as a monomer in the reaction solution at a concentration within a range from 100 nM (nmol/L) to 800 nM (nmol/L) relative to the total volume of the reaction solution, and is preferably included at a concentration within a range from 100 nM to 500 nM, from 100 nM to 400 nM, or from 100 nM to 300 nM, and more preferably included at a concentration of 60 nM, although the invention is not limited to this concentration. The RecG helicase may be used within a concentration range that yields an enzyme activity unit equivalent to that obtained for the concentration range specified above for the E. coli-derived RecG.

A single-stranded DNA-specific exonuclease is an enzyme that sequentially hydrolyzes the nucleotide at the 5' end or the 3' end of a single-stranded DNA. Provided that the single-stranded DNA-specific exonuclease has activity capable of sequentially hydrolyzing the nucleotide at the 5' end or the 3' end of a single-stranded DNA, there are no particular limitations on the type or biological origin of the exonuclease. For example, exonuclease I (exo I), RecJ, and exonuclease T and the like may be used. A preferred single-stranded DNA-specific exonuclease is exo 1. The single-stranded DNA-specific exonuclease may be included in the reaction solution in a concentration, relative to the total volume of the reaction solution, within a range from 0.1 U/µL to 1.0 U/µL, preferably within a range from 0.15 U/µL to 1.0 U/µL, from 0.2 U/µL to 1.0 U/µL, or from 0.2 U/µL to 0.5 U/µL, although the invention is not limited to this concentration range. The enzyme activity unit (U) of exo I is a unit indicating enzymatic activity, wherein the amount of enzyme required to make 10 nmol of deoxyribonucleotide of the single-stranded DNA soluble in acid in a reaction at 37°C for 30 minutes is deemed to be 1 U. Furthermore, the enzyme activity unit (U) of RecJ is a unit indicating enzymatic activity, wherein the amount of enzyme required to make 0.05 nmol of deoxyribonucleotide of the single-stranded DNA soluble in acid in a reaction at 37°C for 30 minutes is deemed to be 1 U.

The reaction solution used in the RCR method II may also contain an ammonium salt. Examples of the ammonium salt include ammonium sulfate, ammonium chloride and ammonium acetate. Particularly preferred ammonium salts include ammonium sulfate and ammonium acetate. The ammonium salt may be included in the reaction solution in a concentration, relative to the total volume of the reaction solution, within a range from 0.1 mM (mmol/L) to 100 mM (mmol/L), preferably within a range from 0.1 mM to 50 mM, from 1 mM to 50 mM, or from 1 mM to 20 mM, and more preferably a concentration of 4 mM, although the invention is not limited to this concentration.

In those cases where E. coli-derived DNA ligase is used as an enzyme having DNA ligase activity in the second enzyme group, the cofactor NAD (nicotinamide adenine dinucleotide) is also included in the reaction solution. NAD may be included in the reaction solution in a concentration, relative to the total volume of the reaction solution, within a range from 0.01 mM (mmol/L) to 1.0 mM (mmol/L), preferably within a range from 0.1 mM to 1.0 mM, or from 0.1 mM to 0.5 mM, and more preferably a concentration of 0.25 mM, although the invention is not limited to this concentration.

The reaction solution used in the RCR method II may also include a reducing agent. Examples of preferred reducing agents include DTT, β-mercaptoethanol (2-mercaptoethanol), tris (2-carboxyethyl) phosphine (TCEP), and glutathione. A preferred reducing agent is DTT. The reducing agent may be included in the reaction solution in a concentration, relative to the total volume of the reaction solution, within a range from 1.0 mM (mmol/L) to 15.0 mM (mmol/L), and preferably from 2.0 mM to 10.0 mM, or from 4.0 mM to 8.0 mM.

The reaction solution used in the RCR method II may also include an enzyme and substrate for regenerating ATP. Examples of combinations of an ATP-regenerating enzyme and substrate include a combination of creatine kinase and creatine phosphate, and a combination of pyruvate kinase and phosphoenolpyruvate. An example of the ATP-regenerating enzyme is myokinase. A preferred combination of the ATP-regenerating enzyme and substrate is a combination of creatine kinase and creatine phosphate.

The first, second and third enzyme groups included in the reaction solution are as described above in the section entitled "2. First, Second and Third Enzyme Groups".

In one aspect, the first enzyme group used in the RCR method II may include a combination of an enzyme having DnaA activity, one or more types of nucleoid proteins, an enzyme or enzyme group having DNA gyrase activity, a single-stranded DNA binding protein (SSB), an enzyme having DnaB helicase activity, an enzyme having DNA helicase loader activity, an enzyme having DNA primase activity, an enzyme having DNA clamp activity, and an enzyme or enzyme group having DNA polymerase III* activity. The one or more types of nucleoid proteins may be IHF or HU, the enzyme or enzyme group having DNA gyrase activity may be a complex composed of GyrA and GyrB, the enzyme having DnaB helicase activity may be DnaB helicase, the enzyme having DNA helicase loader activity maybe DnaC helicase loader, the enzyme having DNA primase activity may be DnaG primase, enzyme having DNA clamp activity may be DnaN clamp, and the enzyme or enzyme group having DNA polymerase III* activity may be an enzyme or enzyme group containing any of DnaX, HolA, HolB, HolC, HolD, DnaE, DnaQ and HolE.

In another aspect, the second enzyme group used in the RCR method II may include a combination of an enzyme having DNA polymerase I activity and an enzyme having DNA ligase activity. Alternatively, the second enzyme group may include a combination of an enzyme having DNA polymerase I activity, an enzyme having DNA ligase activity, and an enzyme having RNaseH activity.

In another aspect, the third enzyme group used in the RCR method II may include one or more enzymes selected from the group consisting of enzymes having topoisomerase III activity and enzymes having topoisomerase IV activity. Alternatively, the third enzyme group may include a combination of an enzyme having topoisomerase III activity and an enzyme having RecQ helicase activity. Alternatively, the third enzyme group may include a combination of an enzyme having topoisomerase III activity, an enzyme having RecQ helicase activity, and an enzyme having topoisomerase IV activity.

In one aspect, the step (II-2) may be conducted in water-in-oil emulsion. The water-in-oil emulsion may be prepared by adding a mineral oil and a surfactant to the reaction mixture formed in the step (II-1) and mixing the resulting mixture. The types and amounts of the mineral oil and the surfactant may be selected appropriately by a person skilled in the art.

The RCR method II may also include, after step (II-2), a step of diluting the solution at least 5-fold with a reaction solution that does not contain the first through third enzyme groups, and then holding the resulting solution at a specified temperature. Dilution of the enzyme groups suppresses any new replication initiation, but enables partly completed replication elongation, catenane formation and separation reactions to continue to proceed under the effects of the residual enzymes. Further, by-products produced due to the inclusion of nicks or the like during the reaction can also be repaired in this step under the effects of residual ligase and the like. Accordingly, it can be expected that transition from amplification intermediates or by-products to the final product can be specifically promoted, enabling an improvement in the yield of the circular DNA having the target supercoiled structure.

The RCR method II may also include, after step (II-2), a step of conducting a treatment with one or more types of exonuclease selected from the group consisting of linear DNA-specific exonucleases and single-stranded DNA-specific exonucleases. By conducting a treatment with one or more types of exonuclease selected from the group consisting of linear DNA-specific exonucleases and single-stranded DNA-specific exonucleases, linear DNA that represents a by-product produced during the amplification reaction can be decomposed and removed. The types and amounts used of the one or more types of exonuclease selected from the group consisting of linear DNA-specific exonucleases and single-stranded DNA-specific exonucleases may be the same as described above. The treatment with one or more types of exonuclease selected from the group consisting of linear DNA-specific exonucleases and single-stranded DNA-specific exonucleases may be conducted, for example, at 25°C to 40°C for 30 minutes to 3 hours.

The RCR method II may also include, after step (II-2), a step of conducting a treatment with gap repair enzymes. Gap repair enzymes are an enzyme group that repair gaps, which describes a state in which one or a plurality of consecutive nucleotides are missing in a double-stranded DNA, or nicks, which describes a state in which a phosphodiester linkage between adjacent nucleotides in a double-stranded DNA has been cleaved, thus enabling conversion to a complete double-stranded supercoiled DNA. By conducting a treatment with gap repair enzymes, DNA containing gaps or nicks that have been produced as by-products during the amplification reaction can be repaired, producing an effect that improves the yield of the target supercoiled product.

There are no particular limitations on the type or biological origin of the gap repair enzymes, provided the enzymes are capable of repairing gaps and nicks in double-stranded DNA. For example, a combination of enzymes or an enzyme group having exonuclease III, DNA polymerase I, DNA ligase and DNA gyrase activity may be used. The enzyme having exonuclease III activity may be used in a concentration of 5 mU/µL to 100 mU/µL, but the invention is not limited to this concentration. The enzyme activity unit (U) of the exonuclease III is a unit indicating enzymatic activity, wherein the amount of enzyme required to make 1 nmol of deoxyribonucleotide of a double-stranded DNA soluble in acid in a reaction at 37°C for 30 minutes is deemed to be 1 U. The enzymes or enzyme groups having DNA polymerase I, DNA ligase and DNA gyrase activity may each be used in the concentration specified above in relation to the aforementioned first or second enzyme groups, although the invention is not limited to these values. The treatment using gap repair enzymes may be conducted, for example, at 25°C to 40°C for 5 minutes to 120 minutes, and preferably for 10 minutes to 60 minutes.

Depending on the purpose, the RCR method II may also include, after step (II-2), a step of purifying the amplified product of the circular DNA. Purification of the circular DNA may be conducted as appropriate using techniques widely usable by those skilled in the art.

The circular DNA that has been amplified using the RCR method II may be used for subsequent purposes such as transformation either in the form of the reaction mixture obtained following reaction, or in an appropriately purified form.

### <RCR Method III>

In one embodiment, the method of the present invention includes the following steps:
(III-1) a step of forming a reaction mixture of a circular DNA that represents the template, and a reaction solution containing:
   a first enzyme group that catalyzes replication of the circular DNA,
   a second enzyme group that catalyzes an Okazaki fragment ligation reaction and synthesizes two sister circular DNAs that form a catenane, and
   a third enzyme group that catalyzes a separation reaction of the two sister circular DNAs; and
(III-2) a step of reacting the reaction mixture formed in step (III-1), wherein
   the circular DNA contains a replication origin sequence (origin of chromosome (oriC)) that can bind to an enzyme having DnaA activity, and also contains at least one type of sequence selected from the group consisting of a pair of ter sequences that are each inserted facing outward relative to oriC, and a base sequence that is recognized by XerCD, and
   in those cases where the circular DNA has ter sequences, the reaction solution of step (III-1) also contains a protein having activity that binds to the ter sequences and inhibits replication, whereas in those cases where the circular DNA has a base sequence that is recognized by XerCD, the reaction solution of step (III-1) also contains an XerCD protein (see Patent Document 7).

The RCR method III replicates or amplifies the circular DNA. In this description, replication of the circular DNA means the production of the molecules that are the same as the circular DNA of the template. Replication of the circular DNA can be confirmed by an increase in the amount of the circular DNA in the reaction mixture following reaction relative to the amount of the circular DNA of the template at the start of the reaction. It is preferable that replication of the circular DNA describes a process in which the amount of the circular DNA in the reaction mixture relative to the amount of the circular DNA at the start of the reaction is increased at least 2-fold, 3-fold, 5-fold, 7-fold, or 9-fold. Amplification of the circular DNA means that replication of the circular DNA proceeds, so that the amount of the circular DNA in the reaction mixture increases exponentially relative to the amount of the circular DNA of the template at the start of the reaction. Accordingly, amplification of the circular DNA is one aspect of replication of the circular DNA. In this description, amplification of the circular DNA describes a process in which the amount of the circular DNA in the reaction mixture relative to the amount of the circular DNA that functions as the template at the start of the reaction is increased at least 10-fold, 50-fold, 100-fold, 200-fold, 500-fold, 1,000-fold, 2,000-fold, 3,000-fold, 4,000-fold, 5,000-fold or 10,000-fold.

The circular DNA that is mixed with the reaction solution is as described above in the section entitled "1. Circular DNA". There are no particular limitations on the amount of the template DNA used per reaction, and for example, the template DNA may be added to the reaction solution at a concentration, relative to the total volume of the reaction solution at the start of the reaction, of not more than 10 ng/µL, not more than 5 ng/µL, not more than 1 ng/µL, not more than 0.8 ng/µL, not more than 0.5 ng/µL, not more than 0.1 ng/µL, not more than 75 pg/µL, not more than 50 pg/µL, not more than 14 pg/µL, not more than 5 pg/µL, not more than 1 pg/µL, not more than 0.67 pg/µL, not more than 0.5 pg/µL, not more than 50 fg/µL, not more than 7.5 fg/µL, not more than 5 fg/µL, or 0.5 fg/µL or less. Moreover, at the start of the reaction, a single molecule of the circular DNA per reaction may be introduced as the template and then used in the replication or amplification.

The circular DNA that functions as the template used in the RCR method III contains at least one sequence selected from the group consisting of a pair of ter sequences that are each inserted facing outward relative to oriC, and base sequences that are recognized by XerCD. In those cases where the circular DNA has ter sequences, the reaction solution of step (III-1) also contains a protein having activity that binds to the ter sequences and inhibits replication, whereas in those cases where the circular DNA has a base sequence that is recognized by XerCD, the reaction solution of step (III-1) also contains an XerCD protein.

The one or more proteins selected from the group consisting of proteins having activity that binds to the ter sequences and inhibits replication and XerCD proteins may use commercially available products, or may be extracted from microorganisms or the like and then purified as required. Extraction of enzymes from microorganisms and subsequent purification may be conducted appropriately using techniques widely usable by those skilled in the art.

A combination of ter sequences on the DNA and a protein having activity that binds to the ter sequences and inhibits replication is a mechanism for terminating replication. This mechanism was discovered in a plurality of types of bacteria, and for example, in E. coli is known as the Tus-ter system (Hiasa, H., and Marians, K. J., J. Biol. Chem., 1994, 269: 26959 to 26968; Neylon, C., et al., Microbiol. Mol. Biol. Rev., September 2005, pp. 501 to 526) and in Bacillus bacteria is known as the RTP-ter system (Vivian, et al., J. Mol. Biol., 2007, 370: 481 to 491). In the RCR method III, by utilizing this mechanism, generation of DNA multimers as by-products can be suppressed. There are no particular limitations on the biological origin of the combination of the ter sequences on the DNA and the protein having activity that binds to the ter sequences and inhibits replication.

In one preferred embodiment, the RCR method III uses a combination of ter sequences and a Tus protein. The ter sequences used in combination with the Tus protein may be a sequence containing 5'-GN[A/G][T/A]GTTGTAAC[T/G]A-3' (SEQ ID NO: 23), or more preferably 5'-G[T/G]A[T/A]GTTGTAAC[T/G]A-3' (SEQ ID NO: 24), 5'-GTATGTTGTAACTA-3' (SEQ ID NO: 25), 5'-AGTATGTTGTAACTAAAG-3' (SEQ ID NO: 26), 5'-GGATGTTGTAACTA-3' (SEQ ID NO: 27), 5'-GTATGTTGTAACGA-3' (SEQ ID NO: 28), 5'-GGATGTTGTAACTA-3' (SEQ ID NO: 29), 5'-GGAAGTTGTAACGA-3' (SEQ ID NO: 30), or 5'-GTAAGTTGTAACGA-3' (SEQ ID NO: 31). Although there are no particular limitations on the origin of the Tus protein, an E. coli-derived Tus protein is preferred. The Tus protein may be included in the reaction solution in a concentration, relative to the total volume of the reaction solution, within a range from 1 nM (nmol/L) to 200 nM (nmol/L), and preferably within a range from 2 nM to 200 nM, from 2 nM to 100 nM, from 5 nM to 200 nM, from 5 nM to 100 nM, from 10 nM to 100 nM, from 20 nM to 100 nM, or from 20 nM to 80 nM, although the invention is not limited to these ranges.

In another preferred embodiment, the RCR method III uses a combination of ter sequences and an RTP protein. The ter sequences used in combination with the RTP protein are sequences having a length of 23 to 30 bases containing 5'-AC[T/A][A/G]ANNNNN[C/T]NATGTACNAAAT-3' (SEQ ID NO: 32), or preferably containing 5'-ACTAATT[A/G]A[A/T]C[T/C]ATGTACTAAAT-3' (SEQ ID NO: 33), 5'-ACTAATT[A/G]A[A/T]C[T/C]ATGTACTAAATTTTCA-3' (SEQ ID NO: 34), 5'-GAACTAATTAAACTATGTACTAAATTTTCA-3' (SEQ ID NO: 35), or 5'-ATACTAATTGATCCATGTACTAAATTTTCA-3' (SEQ ID NO: 36). In those cases where a sequence having a length of 23 to 30 bases containing any of SEQ ID NOS: 10 to 12 is selected as the ter sequence, the sequence may have sequence identity with SEQ ID NO: 13 or 14 of at least 70%, at least 80%, at least 90%, or at least 95%. Although there are no particular limitations on the origin of the RTP protein, RTP proteins derived from Bacillus bacteria are preferred, and an RTP protein derived from Bacillus subtilis is more preferred. The Tus protein may be included in the reaction solution in a concentration, relative to the total volume of the reaction solution, within a range from 1 nM (nmol/L) to 200 nM (nmol/L), and preferably within a range from 2 nM to 200 nM, 2 nM to 100 nM, 5 nM to 200 nM, 5 nM to 100 nM, 10 nM to 100 nM, 20 nM to 100 nM, or 20 nM to 80 nM, although the invention is not limited to these ranges.

In relation to the ter sequences, the expression "inserted facing outward relative to oriC" means that the ter sequences are inserted in a direction such that the action of the combination with the protein having activity that binds to the ter sequences and inhibits replication permits replication in the direction facing outside oriC, but does not permit and terminates replication in the direction facing toward oriC. Accordingly, the expression "a pair of ter sequences that are each inserted facing outward relative to oriC" used in relation to the ter sequences means that a sequence containing any of the sequences shown in SEQ ID NOS: 1 to 14 is inserted as one ter sequence on the 5' side of oriC, and a sequence containing a complementary sequence to any of the sequences shown in SEQ ID NOS: 1 to 14 is inserted as the other ter sequence on the 3' side of oriC.

The ter sequences may exist in any positions, provided each of the pair of ter sequences is inserted facing outward relative to oriC. For example, the pair of ter sequences may exist in regions far removed from oriC, or may exist in a region close to or adjacent to both sides of oriC. In those cases where the pair of ter sequences exists in a region close to or adjacent to both sides of oriC, the oriC and the pair of ter sequences can be prepared as a functional cassette, which offers the advantages that introduction of oriC and the pair of ter sequences into the DNA is simpler, and the cost of preparing the circular DNA used as a template can be reduced.

A combination of a sequence that is recognized by XerCD on the DNA and a XerCD protein functions in a mechanism for separating a DNA multimer (Ip, S. C. Y., et al., EMBO J., 2003, 22: 6399 to 6407). The XerCD protein is a complex of XerC and XerD. Known sequences recognized by the XerCD protein include a dif sequence, a cer sequence, and a psi sequence (Colloms, et al., EMBO J., 1996, 15(5): 1172 to 1181; Arciszewska, L. K., et al., J. Mol. Biol., 2000, 299: 391 to 403). In the method of the present embodiment, by utilizing this mechanism, the production of DNA multimers as by-products can be suppressed. There are no particular limitations on the biological origin of the combination of the sequence that is recognized by XerCD on the DNA and the XerCD protein. Further, promotion factors for XerCD are known, and for example, the function in dif is promoted by an FtsK protein (Ip, S. C. Y., et al., EMBO J., 2003, 22: 6399 to 6407). In one aspect, a FtsK protein may be included in the reaction solution in the RCR method III.

The sequence that is recognized by XerCD may be a sequence containing 5'-GGTGCG[C/T][A/G][T/C]AANNNNNNTTATG[T/G]TAAA[T/C]-3' (SEQ ID NO: 37), 5'-GGTGCG[C/T]A[T/C]AANNNNNNTTATG[T/G]TAAAT-3' (SEQ ID NO: 38), 5'-GGTGCGC[A/G][T/C]AANNNNNNTTATGTTAAA[T/C]-3' (SEQ ID NO: 39), 5'-GGTGCG[C/T][A/G]CAANNNNNNTTATG[T/G]TAAA[T/C]-3' (SEQ ID NO: 40), 5'-GGTGCGCATAANNNNNNTTATGTTAAAT-3' (SEQ ID NO: 41), 5'-GGTGCGTACAANNNNNNTTATGGTAAAT-3' (SEQ ID NO: 42), 5'-GGTGCGCGCAANNNNNNTTATGTTAAAC-3' (SEQ ID NO: 43), 5'-GGTGCGCATAATGTATATTATGTTAAAT-3' (SEQ ID NO: 44/dif sequence), 5'-GGTGCGTACAAGGGATGTTATGGTAAAT-3' (SEQ ID NO: 45/cer sequence), or 5'-GGTGCGCGCAAGATCCATTATGTTAAAC-3' (SEQ ID NO: 46/psi sequence), or a complementary sequence to any of these sequences. The portion from bases 1 to 11 in SEQ ID NOS: 15 to 24 represent an XerC binding site, and the portion from bases 18 to 28 in SEQ ID NOS: 15 to 24 represents an XerD binding site. Because the portion from bases 12 to 17 in SEQ ID NOS: 15 to 21 (the 6-base portion indicated by NNNNNN) is not a binding region for XerC or XerD, there are no particular limitations on the sequence of that portion. The sequence of the 12th to 17th bases in SEQ ID NOS: 15 to 21 (the 6-base portion indicated by NNNNNN) may have sequence identity with the sequence of the 12th to 17th bases in SEQ ID NOS: 22 to 24 of at least 70%, at least 80%, at least 90%, or at least 95%.

The XerCD protein is preferably an XerCD protein derived from E. coli. The XerCD protein may be include in the reaction solution in a concentration, relative to the total volume of the reaction solution, within a range from 1 nM (nmol/L) to 200 nM (nmol/L), and preferably within a range from 5 nM to 200 nM, 5 nM to 150 nM, 10 nM to 200 nM, 10 nM to 150 nM, 20 nM to 200 nM, 20 nM to 150 nM, or 20 nM to 100 nM, although the invention is not limited to these ranges.

The sequence that is recognized by XerCD may exist at any position on the circular DNA. For example, the sequence that is recognized by XerCD may exist in a region far removed from oriC, or may exist in a region close to or adjacent to oriC. In those cases where the sequence that is recognized by XerCD exists in a region close to or adjacent to oriC, oriC and the sequence that is recognized by XerCD can be prepared as a functional cassette, which offers the advantages that introduction of oriC and the sequence that is recognized by XerCD into the DNA is simpler, and the cost of preparing the circular DNA used as a template can be reduced.

In this description, the identity (%) between two base sequences can be determined by visual inspection and mathematical calculation. Further, the identity (%) can also be determined using computer programs. Examples of such sequence comparison computer programs include the BLASTN program, which can be used from the website of the United States National Library of Medicine (http://www.ncbi.nlm.nih.gov/blast/bl2seq/bls.html) (Altschul et al. (1990) J. Mol. Biol. 215: 403 to 10) (version 2.2.7), or the WU-BLAST2.0 algorithm. Setting of the standard default parameters in WU-BLAST2.0 may be conducted using the parameters disclosed at the following internet site (http://blast.wustl.edu).

The first, second and third enzyme groups included in the reaction solution are as described above in the section entitled "2. First, Second and Third Enzyme Groups".

In one aspect, the first enzyme group used in the RCR method III may include a combination of an enzyme having DnaA activity, one or more types of nucleoid proteins, an enzyme or enzyme group having DNA gyrase activity, a single-stranded DNA binding protein (SSB), an enzyme having DnaB helicase activity, an enzyme having DNA helicase loader activity, an enzyme having DNA primase activity, an enzyme having DNA clamp activity, and an enzyme or enzyme group having DNA polymerase III* activity. The one or more types of nucleoid proteins may be IHF or HU, the enzyme or enzyme group having DNA gyrase activity may be a complex composed of GyrA and GyrB, the enzyme having DnaB helicase activity may be DnaB helicase, the enzyme having DNA helicase loader activity maybe DnaC helicase loader, the enzyme having DNA primase activity may be DnaG primase, enzyme having DNA clamp activity may be DnaN clamp, and the enzyme or enzyme group having DNA polymerase III* activity may be an enzyme or enzyme group containing any of DnaX, HolA, HolB, HolC, HolD, DnaE, DnaQ and HolE.

In another aspect, the second enzyme group used in the RCR method III may include a combination of an enzyme having DNA polymerase I activity and an enzyme having DNA ligase activity. Alternatively, the second enzyme group may include a combination of an enzyme having DNA polymerase I activity, an enzyme having DNA ligase activity, and an enzyme having RNaseH activity.

In another aspect, the third enzyme group used in the RCR method III may include one or more enzymes selected from the group consisting of enzymes having topoisomerase III activity and enzymes having topoisomerase IV activity. Alternatively, the third enzyme group may include a combination of an enzyme having topoisomerase III activity and an enzyme having RecQ helicase activity. Alternatively, the third enzyme group may include a combination of an enzyme having topoisomerase III activity, an enzyme having RecQ helicase activity, and an enzyme having topoisomerase IV activity.

The reaction solution may also contain a buffer solution, ATP, GTP, CTP, UTP, dNTP, a magnesium ion source, and an alkali metal ion source.

There are no particular limitations on the buffer solution included in the reaction solution, provided it is suitable for use within a range from pH 7 to 9, and preferably at pH 8. Examples of the buffer include Tris-HCl, Hepes-KOH, phosphate buffer, MOPS-NaOH, and Tricine-HCl. A preferred buffer is Tris-HCl. There are no particular limitations on the concentration of the buffer solution, and the concentration may be selected appropriately by a person skilled in the art. In the case of Tris-HCl, for example, the concentration of the buffer solution, relative to the total volume of the reaction solution, may be within a range from 10 mM (mmol/L) to 100 mM or from 10 mM to 50 mM, or a concentration of 20 mM may be selected.

ATP means adenosine triphosphate. The concentration of ATP contained in the reaction solution at the start of the reaction, for example, relative to the total volume of the reaction solution, may be within a range from 0.1 mM (mmol/L) to 3 mM (mmol/L), is preferably within a range from 0.1 mM to 2 mM, from 0.1 mM to 1.5 mM, or from 0.5 mM to 1.5 mM, and is more preferably 1 mM.

GTP, CTP and UTP mean guanosine triphosphate, cytidine triphosphate and uridine triphosphate respectively. The concentration of each of GTP, CTP and UTP included in the reaction solution at the start of the reaction, for example, relative to the total volume of the reaction solution, may be within a range from 0.1 mM (mmol/L) to 3.0 mM (mmol/L), and is preferably within a range from 0.5 mM to 3.0 mM, or from 0.5 mM to 2.0 mM.

Moreover, dNTP is a generic term for deoxyadenosine triphosphate (dATP), deoxyguanosine triphosphate (dGTP), deoxycytidine triphosphate (dCTP) and deoxythymidine triphosphate (dTTP). The concentration of dNTP included in the reaction solution at the start of the reaction, for example, relative to the total volume of the reaction solution, may be set within a range from 0.01 mM (mmol/L) to 1 mM (mmol/L), is preferably within a range from 0.05 mM to 1 mM, or from 0.1 mM to 1 mM, and is more preferably within a range from 0.25 mM to 1 mM.

The magnesium ion source is a substance that provides magnesium ions (Mg²⁺) in the reaction solution. Examples of the magnesium ion source include Mg(OAc)₂, MgCl₂ and MgSO₄. Mg(OAc)₂ is a preferred magnesium ion source. The concentration of the magnesium ion source included in the reaction solution at the start of the reaction, relative to the total volume of the reaction solution, may be a concentration which provides, for example, from 5 mM (mmol/L) to 50 mM (mmol/L) of magnesium ions, and preferably 1 mM of magnesium ions, in the reaction solution.

The alkali metal ion source is a substance that provides alkali metal ions in the reaction solution. Examples of the alkali metal ions include sodium ions (Na⁺) and potassium ions (K⁺). Examples of the alkali metal ion source include potassium glutamate, potassium aspartate, potassium chloride, potassium acetate, sodium glutamate, sodium aspartate, sodium chloride, and sodium acetate. Preferred alkali metal ion sources include potassium glutamate and potassium acetate. The concentration of the alkali metal ion source included in the reaction solution at the start of the reaction, relative to the total volume of the reaction solution, may be a concentration which provides, for example, at least 100 mM (mmol/L), preferably from 100 mM to 300 mM, and more preferably 50 mM, of alkali metal ions in the reaction solution, although the invention is not limited to this concentration. In combination with an earlier application, 150 mM may be subtracted from the above concentration of the alkali metal ion source.

The reaction solution may also contain a protein non-specific adsorption inhibitor or a nucleic acid non-specific adsorption inhibitor. It is preferable that the reaction solution contains both a protein non-specific adsorption inhibitor and a nucleic acid non-specific adsorption inhibitor. Including one or more non-specific adsorption inhibitors selected from the group consisting of protein non-specific adsorption inhibitors and nucleic acid non-specific adsorption inhibitors in the reaction solution enables inhibition of at least one type of non-specific adsorption selected from the group consisting of adsorption between proteins and adsorption between a protein and a circular DNA, and can also inhibit adhesion of proteins and the circular DNA to the surfaces of the reaction vessel, meaning an improvement in the reaction efficiency can be expected.

Protein non-specific adsorption inhibitors are proteins that are unrelated to the replication or amplification reaction in the method of the present embodiment. Examples of these types of proteins include bovine serum albumin (BSA), lysozyme, gelatin, heparin and casein. The protein non-specific adsorption inhibitor may be included in the reaction solution in a concentration, relative to the total volume of the reaction solution, that is within a range from 0.02 mg/mL to 2.0 mg/mL, preferably within a range from 0.1 mg/mL to 2.0 mg/mL, 0.2 mg/mL to 2.0 mg/mL, or 0.5 mg/mL to 2.0 mg/mL, and more preferably at a concentration of 0.5 mg/mL, although the invention is not limited to this concentration.

Nucleic acid non-specific adsorption inhibitors are nucleic acid molecules or nucleic acid-related molecules that are unrelated to the replication or amplification reaction in the RCR method III. Examples of these types of nucleic acid molecules or nucleic acid-related molecules include tRNA (transfer RNA), rRNA (ribosomal RNA), mRNA (messenger RNA), glycogen, heparin, oligo DNA, poly(I-C) polyinosine-polycytidine, poly(dI-dC), (polydeoxyinosine-polydeoxycytidine), poly(A) (polyadenine), and poly(dA) (polydeoxyadenine).

The nucleic acid non-specific adsorption inhibitor may be included in the reaction solution in a concentration, relative to the total volume of the reaction solution, that is within a range from 1 ng/µL to 500 ng/µL, preferably within a range from 10 ng/µL to 500 ng/µL, 10 ng/µL to 200 ng/µL, or 10 ng/µL to 100 ng/µL, and more preferably at a concentration of 50 ng/µL, although the invention is not limited to this concentration. In combination with an earlier application, in those cases where tRNA is selected as the nucleic acid non-specific adsorption inhibitor, 50 ng/µL may be subtracted from the above concentration of tRNA.

The reaction solution may also contain a linear DNA-specific exonuclease or a RecG helicase. The reaction solution preferably also contains both a linear DNA-specific exonuclease and a RecG helicase. By including one or more substances selected from the group consisting of linear DNA-specific exonucleases and RecG helicases in the reaction solution, the amount of linear DNA produced by double strand cleavage or the like in the replication or amplification reaction can be reduced, resulting in an effect of improving the yield of the target supercoiled product.

Linear DNA-specific exonucleases are enzymes that sequentially hydrolyze from the 5' end or the 3' end of a linear DNA. Provided that the linear DNA-specific exonuclease has activity capable of sequentially hydrolyzing from the 5' end or the 3' end of a linear DNA, there are no particular limitations on the type or biological origin of the exonuclease. For example, RecBCD, λ exonuclease, exonuclease III, exonuclease VIII, T5 exonuclease, T7 exonuclease, and Plasmid-SafeTM ATP-Dependent DNase (epicentre) and the like may be used. A preferred linear DNA-specific exonuclease is RecBCD. The linear DNA-specific exonuclease may be included in the reaction solution in a concentration, relative to the total volume of the reaction solution, within a range from 0.01 U/µL to 1.0 U/µL, and preferably within a range from 0.08 U/µL to 1.0 U/µL, or from 0.1 U/µL to 1.0 U/µL, although the invention is not limited to this concentration range. The enzyme activity unit (U) of the linear DNA exonuclease is a unit indicating enzymatic activity, wherein the amount of enzyme required to make 1 nmol of deoxyribonucleotide of the linear DNA soluble in acid in a reaction at 37°C for 30 minutes is deemed to be 1 U.

The RecG helicase is an enzyme that is thought to be a helicase that resolves the problem of secondary DNA structures generated as a result of collision between replication forks during termination of the elongation reaction. There are no particular limitations on the biological origin of the RecG helicase, provided the enzyme has activity that is similar to that of the E. coli-derived RecG, and for example, the E. coli-derived RecG can be used favorably. The E. coli-derived RecG may be included as a monomer in the reaction solution at a concentration within a range from 100 nM (nmol/L) to 800 nM (nmol/L) relative to the total volume of the reaction solution, and is preferably included at a concentration within a range from 100 nM to 500 nM, from 100 nM to 400 nM, or from 100 nM to 300 nM, and more preferably included at a concentration of 60 nM, although the invention is not limited to this concentration. The RecG helicase may be used within a concentration range that yields an enzyme activity unit equivalent to that obtained for the concentration range specified above for the E. coli-derived RecG.

The reaction solution may also contain an ammonium salt. Examples of the ammonium salt include ammonium sulfate, ammonium chloride and ammonium acetate. A particularly preferred ammonium salt is ammonium sulfate. The ammonium salt may be included in the reaction solution in a concentration, relative to the total volume of the reaction solution, within a range from 0.1 mM (mmol/L) to 100 mM (mmol/L), preferably within a range from 0.1 mM to 50 mM, from 1 mM to 50 mM, or from 1 mM to 20 mM, and more preferably a concentration of 4 mM, although the invention is not limited to this concentration.

In those cases where E. coli-derived DNA ligase is used as an enzyme having DNA ligase activity in the second enzyme group, the cofactor NAD (nicotinamide adenine dinucleotide) is also included in the reaction solution. NAD may be included in the reaction solution in a concentration, relative to the total volume of the reaction solution, within a range from 0.01 mM (mmol/L) to 1.0 mM, preferably within a range from 0.1 mM to 1.0 mM, or from 0.1 mM to 0.5 mM, and more preferably a concentration of 0.25 mM, although the invention is not limited to this concentration.

The reaction solution used in the RCR method III may also include a reducing agent. Examples of preferred reducing agents include DTT, β-mercaptoethanol, and glutathione. A preferred reducing agent is DTT.

The reaction solution used in the RCR method III may also include an enzyme and substrate for regenerating ATP. Examples of combinations of an ATP-regenerating enzyme and substrate include a combination of creatine kinase and creatine phosphate, and a combination of pyruvate kinase and phosphoenolpyruvate. An example of the ATP-regenerating enzyme is myokinase. A preferred combination of the ATP-regenerating enzyme and substrate is a combination of creatine kinase and creatine phosphate.

The aforementioned step (III-2) is a step of reacting the reaction mixture formed in step (III-1). Step (III-2) may be, for example, a step of reacting the reaction mixture within a range from 15°C to 80°C, from 15°C to 50°C, or from 15°C to 40°C. Step (III-2) is preferably a step of holding the temperature under isothermal conditions. There are no particular limitations on the isothermal conditions, provided the DNA replication reaction can proceed, and for example, the temperature may be set to a constant temperature within the range from 20 to 80°C that represents the optimal temperature for DNA polymerase, may be set to a constant temperature within a range from 25°C to 50°C, may be set to a constant temperature within a range from 25°C to 40°C, or may be set to about 30°C to 33°C. In this description, expressions such as "hold the temperature under isothermal conditions" and "react at a constant temperature" mean that the temperature is held within the above temperature range during the reaction. The time for which the temperature is held may be set appropriately in accordance with the amount of the replicated product or amplified product of the circular DNA that represents the target, and for example, may be set to a time within a range from 1 to 24 hours, or from 16 to 21 hours.

Alternatively, a step in which the reaction mixture formed in step (III-1) is incubated under a temperature cycle that repeats incubation at 30°C or higher and incubation at 27°C or lower may be included as the above step (III-2). There are no particular limitations on the incubation at 30°C or higher, provided the temperature enables initiation of the replication of the circular DNA containing oriC, and for example, the temperature may be within a range from 30°C to 80°C, from 30°C to 50°C, from 30° to 40°C, or may be 37°C. There are no particular limitations on the length of the incubation at 30°C or higher, and the time per one cycle may be from 10 seconds to 10 minutes, or may be 1 minute. There are no particular limitations on the incubation at 27°C or lower, provided the temperature suppresses replication initiation but allows elongation reactions of the DNA to proceed, and for example, the temperature may be within a range from 10°C to 27°C, from 16°C to 25°C, or may be 24°C. There are no particular limitations on the incubation conducted at 27°C or lower, but the time is preferably set in accordance with the length of the circular DNA being amplified, and for example, the time per one cycle may be within a range from 1 seconds to 10 seconds per 1,000 bases. The number of cycles of the temperature cycle is not particularly limited, and may be from 10 cycles to 50 cycles, from 20 cycles to 45 cycles, from 25 cycles to 45 cycles, or may be 40 cycles.

Depending on the purpose, the RCR method III may also include, after the above step of holding the temperature of the reaction mixture under isothermal conditions, a step of purifying the replicated product or amplified product of the circular DNA. Purification of the circular DNA may be conducted as appropriate using techniques widely usable by those skilled in the art.

The circular DNA that has been replicated or amplified using the RCR method III may be used for subsequent purposes such as transformation either in the form of the reaction mixture obtained following reaction, or in an appropriately purified form.

### <RCR Method IV>

It is known that, in a similar manner to the combination of XerCD and dif, use of a combination of Cre and the recognition sequence loxP can guide the separation of DNA multimers (Ip, S. C. Y., et al., EMBO J., 2003, 22: 6399 to 6407). The inventors of the present invention discovered that even when using a combination of a DNA multimer separation enzyme and its recognition sequence instead of the combination of XerCD and dif in the RCR method III, production of DNA multimers as by-products could be suppressed.

In one embodiment, the present invention includes the following steps:
(IV-1) a step of forming a reaction mixture of a circular DNA that represents the template, and a reaction solution containing:
   a first enzyme group that catalyzes replication of the circular DNA,
   a second enzyme group that catalyzes an Okazaki fragment ligation reaction and synthesizes two sister circular DNAs that form a catenane, and
   a third enzyme group that catalyzes a separation reaction of the two sister circular DNAs; and
(IV-2) a step of reacting the reaction mixture formed in step (IV-1), wherein
   the circular DNA contains a replication origin sequence (origin of chromosome (oriC)) that can bind to an enzyme having DnaA activity, and also contains at least one type of sequence selected from the group consisting of a pair of ter sequences that are each inserted facing outward relative to oriC, and a base sequence that is recognized by a DNA multimer separation enzyme, and
   in those cases where the circular DNA has ter sequences, the reaction solution of step (IV-1) also contains a protein having activity that binds to the ter sequences and inhibits replication, whereas in those cases where the circular DNA has a base sequence that is recognized by a DNA multimer separation enzyme, the reaction solution of step (IV-1) also contains a DNA multimer separation enzyme (see Patent Document 7).

In other words, the RCR method IV is a method in which "XerCD" and "base sequence that is recognized by XerCD" from RCR method III are expanded to "DNA multimer separation enzyme" and "base sequence that is recognized by DNA multimer separation enzyme" respectively. Accordingly, the descriptions disclosed above in the section entitled <RCR Method III> for the various configurations of the RCR method III may also be applied to the RCR method IV.

The DNA multimer separation enzyme is an enzyme which, by causing genetic recombination, can guide separation of DNA multimers. A site-specific recombination enzyme, which can recognize a specific base sequence and cause genetic recombination at the site of that base sequence, can be used as the DNA multimer separation enzyme. The specific base sequence recognized by the DNA multimer separation enzyme is referred to as "the base sequence that is recognized by the DNA multimer separation enzyme". By conducting genetic recombination using a combination of a DNA multimer separation enzyme and a base sequence that is recognized by the DNA multimer separation enzyme, DNA multimers can be separated. In the RCR method IV, by utilizing this mechanism, the production of DNA multimers as by-products can be suppressed. The DNA multimer separation enzyme may use commercially available products, or may be extracted from microorganisms or the like and then purified as required. Extraction of the enzyme from a microorganism and subsequent purification may be conducted appropriately using techniques widely usable by those skilled in the art.

Examples of the combination of the DNA multimer separation enzyme and the base sequence that is recognized by the DNA multimer separation enzyme include XerCD and a dif sequence; Cre and a loxP sequence (Siegel, R. W., et al.., FEBS Lett., 2001, 499(1-2): 147 to 153; Araki, K., et al., Nucleic Acids Res.: 1997, 25(4): 868 to 872); budding yeast (Saccharomyces verevisiae)-derived recombinant enzyme FLP and an FRT sequence (Broach, J. R., et al., Cell, 1982, 29(1): 227 to 234); bacteriophage D6-derived recombinant enzyme DreO and a rox sequence (Anastassiadis, K., et al., Dis. Model. Meeh., 2009, 2: 508 to 515); Zygosacchromyces rouxii-derived recombinant enzyme R and an RS sequence (Araki, H., et al., J. Mol. Biol., 1985, 182(2): 191 to 203); and a serine recombinant enzyme family (for example, Gin, γδ, Tn3, and Hin) and the recognition sequences thereof (Smith, M. C., et al., Mol. Microbiol., 2002, 44: 299), although the invention is not limited to these combinations.

The XerCD and the dif sequence are as described above in the section entitled <RCR Method III>.

There are no particular limitations on the biological origin of the combination of Cre and a loxP sequence. The Cre is preferably a bacteriophage P1-derived Cre protein. The Cre may be included in the reaction solution in a concentration, relative to the total volume of the reaction solution, within a range from 0.01 mU/µL to 200 mU/µL, and preferably within a range from 0.1 mU/µL to 150 mU/µL, from 0.1 mU/µL to 100 mU/µL, from 0.5 mU/µL to 100 mU/µL, from 0.5 mU/µL to 80 mU/µL, from 0.1 mU/µL to 50 mU/µL, from 1 mU/µL to 50 mU/µL, or from 1 mU/µL to 30 mU/µL, although the invention is not limited to these ranges.

The loxP sequence that is recognized by Cre may be a sequence containing a loxP consensus represented by 5'-ATAACTTCGTATAGCATACATTATACGAAGTTAT-3' (SEQ ID NO: 47), a mutant loxP sequence represented by 5'-ATAACTTCGTATAGtATACATTATACGAAGTTAT-3' (SEQ ID NO: 48/lox511), 5'-ATAACTTCGTATAGgATACtTTATACGAAGTTAT-3' (SEQ ID NO: 49/lox2272), 5'-ATAACTTCGTATAtacctttcTATACGAAGTTAT-3' (SEQ ID NO: 50/loxFAS), 5'-ATAACTTCGTATAGCATACATTATACGAAcggta-3' (SEQ ID NO: 51/lox RE), or 5'-taccgTTCGTATAGCATACATTATACGAAGTTAT-3' (SEQ ID NO: 52/lox LE) (wherein each lowercase letter indicates a mutant base relative to the consensus), or a complementary sequence to any of these sequences.

The budding yeast (Saccharomyces verevisiae)-derived recombinant enzyme FLP may be included in the reaction solution in a concentration, relative to the total volume of the reaction solution, within a range from 1 nM (nmol/L) to 200 nM (nmol/L), and preferably within a range from 5 nM to 200 nM, from 5 nM to 150 nM, from 10 nM to 200 nM, from 10 nM to 150 nM, from 20 nM to 200 nM, from 20 nM to 150 nM, or from 20 nM to 100 nM, although the invention is not limited to these ranges. The FRT sequence that is recognized by FLP may be a sequence containing 5'-GAAGTTCCTATTCTCTAGAAAGTATAGGAACTTC-3' (SEQ ID NO: 53) or a complementary sequence thereto.

The bacteriophage D6-derived recombinant enzyme DreO may be included in the reaction solution in a concentration, relative to the total volume of the reaction solution, within a range from 1 nM (nmol/L) to 200 nM (nmol/L), and preferably within a range from 5 nM to 200 nM, from 5 nM to 150 nM, from 10 nM to 200 nM, from 10 nM to 150 nM, from 20 nM to 200 nM, from 20 nM to 150 nM, or from 20 nM to 100 nM, although the invention is not limited to these ranges. The rox sequence that is recognized by DreO maybe a sequence containing 5'-TAACTTTAAATAATGCCAATTATTTAAAGTTA-3' (SEQ ID NO: 54) or a complementary sequence thereto.

The Zygosacchromyces rouxii-derived recombinant enzyme R may be included in the reaction solution in a concentration, relative to the total volume of the reaction solution, within a range from 1 nM (nmol/L) to 200 nM (nmol/L), and preferably within a range from 5 nM to 200 nM, from 5 nM to 150 nM, from 10 nM to 200 nM, from 10 nM to 150 nM, from 20 nM to 200 nM, from 20 nM to 150 nM, or from 20 nM to 100 nM, although the invention is not limited to these ranges. The RS sequence that is recognized by the enzyme R may be a sequence containing the sequence disclosed in Araki, H. et al. (J. Mol. Biol., 1985, 182(2): 191 to 203) or a complementary sequence thereto.

The serine recombinant enzyme family (γδ, Tn3, Gin, and Hin) may be included in the reaction solution in a concentration, relative to the total volume of the reaction solution, within a range from 1 nM (nmol/L) to 200 nM (nmol/L), and preferably within a range from 5 nM to 200 nM, from 5 nM to 150 nM, from 10 nM to 200 nM, from 10 nM to 150 nM, from 20 nM to 200 nM, from 20 nM to 150 nM, or from 20 nM to 100 nM, although the invention is not limited to these ranges. Further, γδ and Tn3, and their recognition sequence res may each be a sequence containing a sequence disclosed in Grindley N. D. F. et al. (Cell, 1982, 30: 19 to 27) or a complementary sequence thereto. Furthermore, Gin and the recognition sequence therefor may each be a sequence containing a sequence disclosed in Kahmann. R. et al. (Cell, 1985, 41: 771 to 780) or a complementary sequence thereto. Moreover, Hin and the recognition sequence therefor may each be a sequence containing a sequence disclosed in Glasgow. A. C. et al. (J. Biol. Chem., 1989, 264: 10072 to 10082) or a complementary sequence thereto.

The sequence that is recognized by the DNA multimer separation enzyme may exist at any position on the circular DNA. For example, the sequence recognized by the DNA multimer separation enzyme may exist in a region close to or adjacent to oriC, or may exist in a region far removed from oriC.

### <RCR Method V]

In one embodiment, the method of the present invention includes the following steps:
(V-1) a step of preparing a circular DNA containing oriC by:
   adding an oriC transposon and transposase to a buffer solution to form an oriC transposome, wherein the oriC transposon is a linear DNA containing a replication origin sequence (origin of chromosome (oriC)) that can bind to an enzyme having DnaA activity and outside end (OE) sequences at both ends of the replication origin sequence;
   and reacting the oriC transposome with circular DNA that does not contain oriC in a buffer solution to effect a transfer reaction;
(V-2) a step of forming a reaction mixture of the circular DNA containing oriC obtained in step (V-1), and a reaction solution containing:
   a first enzyme group that catalyzes replication of the circular DNA,
   a second enzyme group that catalyzes an Okazaki fragment ligation reaction and synthesizes two sister circular DNAs that form a catenane, and
   a third enzyme group that catalyzes a separation reaction of the two sister circular DNAs; and
(V-3) a step of reacting the reaction mixture formed in step (V-2) (see Patent Document 7).

Although not constrained by theory, the RCR method V is a method for preparing a circular DNA containing oriC by using a transposon to introduce oriC into a circular DNA that does not contain oriC, and then replicating or amplifying that circular DNA containing oriC. The definitions relating to replication and amplification of the circular DNA are as described above in relation to the RCR method III.

The circular DNA containing oriC that is mixed with the reaction solution is as described above in the section entitled "1. Circular DNA". The amount of the circular DNA containing oriC that is used per reaction is as described above for the amount of the template DNA in the RCR method III.

Further, descriptions of the enzyme groups included in the reaction solution and the other components that may be include in the reaction solution are the same as the RCR method III. Moreover, the above step (V-3) is conducted in the same manner as step (III-2) in the RCR method III. The fact that the RCR method V may also include a step of purifying the replicated product or amplified product of the circular DNA, and use of the circular DNA that has been replicated or amplified using the RCR method V are also the same as described for the RCR method III.

The OE sequences at both ends of the oriC transposon may be any sequences known to those skilled in the art as sequences that are recognized by transposase and can be used as OE sequences. In a preferred embodiment, the OE sequence contains the sequence shown in SEQ ID NO: 55 (5'-CTGTCTCTTATACACATCT-3') or a complementary sequence thereto, the OE sequence containing the sequence shown in SEQ ID NO: 55 is inserted at the 5' end of the linear DNA in step (1), and an OE sequence containing a complementary sequence to the sequence shown in SEQ ID NO: 55 is inserted at the 3' end of the linear DNA.

The concentration of the oriC transposon used in the oriC transposome formation in the above step (V-1), relative to the total volume of the reaction solution, may be within a range from 20 nM (nmol/L) to 200 nM (nmol/L), and is preferably from 40 nM to 160 nM.

There are no particular limitations on the biological origin of the transposase, provided it is an enzyme that recognizes the OE sequence, forms a transposome, and transfers transposon DNA into the circular DNA, but for example, E. coli-derived transposase can be used favorably. A highly active Tn5 mutant (E54K, L372P) protein is particularly preferred (Goryshin, I. Y., and Reznikoff, W. S., J. Biol. Chem., 1998, 273: 7367 to 7374). The transposase may use a commercially available product, or may be extracted from microorganisms or the like and then purified as required. Extraction from microorganisms and subsequent purification may be conducted appropriately using techniques widely usable by those skilled in the art. In those cases where the highly active Tn5 mutant (E54K, L372P) protein is used as the transposase, the concentration of the protein used in the oriC transposome formation in the above step (V-1, relative to the total volume of the reaction solution, may be within a range from 50 nM (nmol/L) to 200 nM (nmol/L), and is preferably within a range from 80 nM to 150 nM.

There are no particular limitations on the buffer solution used in step (V-1), provided it is suitable for use within a range from pH 6 to 9, and preferably at pH 7.5. Examples of the buffer include Tris-acetate (Tris-OAc), Tris-HCl, Hepes-KOH, phosphate buffer, MOPS-NaOH, and Tricine-HCl. Preferred buffers include Tris-OAc and Tris-HCl. There are no particular limitations on the concentration of the buffer solution, and the concentration may be selected appropriately by a person skilled in the art, but in the case of Tris-OAc or Tris-HCl, for example, the concentration of the buffer solution, relative to the total volume of the reaction solution, may be selected from within a range from 10 mM (mmol/L) to 100 mM, or from 10 mM to 50 mM, or may be 20 mM.

In step (V-1), the step of forming the oriC transposome is conducted by holding the temperature at about 30°C for about 30 minutes.

The transfer reaction of step (V-1) is carried out at the optimal temperature for the transposase, for example, 37°C. The time required for the transfer reaction may be selected appropriately by a person skilled in the art, and for example, may be about 15 minutes. Further, in the transfer reaction in step (V-1), tRNA may be added. The concentration of added tRNA in the transfer reaction in step (v-1), for example, relative to the total volume of the reaction solution, may be selected from within a range from 10 ng/µL to 200 ng/µL, or from 30 ng/µL to 100 ng/µL, or may be 50 ng/µL.

In one aspect, the circular DNA containing oriC in step (V-2) may further contain at least one type of sequence selected from the group consisting of a pair of ter sequences that are each inserted facing outward relative to oriC, and a base sequence that is recognized by a DNA multimer separation enzyme such as XerCD or Cre. In such cases, when the circular DNA has the ter sequences, the reaction solution in the above step (V-2) also contains a protein having activity that binds to the ter sequences and inhibits replication, whereas when the circular DNA has a base sequence that is recognized by a DNA multimer separation enzyme such as XerCD or Cre, the reaction solution of step (V-2) also contains a DNA multimer separation enzyme such as XerCD or Cre.

Alternatively, in another aspect, at least one type of sequence selected from the group consisting of a pair of ter sequences that are each inserted facing outward relative to oriC, and a base sequence that is recognized by a DNA multimer separation enzyme such as XerCD or Cre may be prepared so as to comprise a portion of the oriC transposon, meaning the at least one type of sequence selected from the group consisting of a pair of ter sequences and a base sequence that is recognized by a DNA multimer separation enzyme such as XerCD or Cre may also be introduced into the circular DNA using the transposon. In other words, in this aspect, the linear DNA in step (V-1) also contains at least one type of sequence selected from the group consisting of a pair of ter sequences that are each inserted facing outward relative to oriC, and a base sequence that is recognized by a DNA multimer separation enzyme such as XerCD or Cre, wherein when the linear DNA has the ter sequences, the reaction solution in step (V-2) also contains a protein having activity that binds to the ter sequences and inhibits replication, whereas when the circular DNA has a base sequence that is recognized by a DNA multimer separation enzyme such as XerCD or Cre, the reaction solution of step (V-2) also contains an XerCD protein.

Here, the definitions and descriptions for the at least one type of sequence selected from the group consisting of a pair of ter sequences that are each inserted facing outward relative to oriC and a base sequence that is recognized by a DNA multimer separation enzyme such as XerCD or Cre, and the at least one type of material selected from the group consisting of a protein having activity that binds to the ter sequences and inhibits replication and a DNA multimer separation enzyme such as XerCD or Cre are as detailed above in relation to the RCR method III or the RCR method IV.

In one aspect, the RCR method V may also include a step (V-4) of removing the oriC transposon from the replicated or amplified circular DNA in the reaction product of step (V-3).

The step of removing the oriC transposon may include a treatment with a transposase in a concentration, relative to the total volume of the reaction solution, within a range from 0.1 nM (nmol/L) to 30 nM (nmol/L), preferably from 1 nM to 20 nM, and more preferably from 3 nM to 10 nM, and a treatment for converting a DNA end to a single strand using a linear double-stranded DNA dependent single-stranded DNA exonuclease such as ExoIII. The buffer solution used in the treatment with transposase may be the buffer solution used in step (V-1). The buffer solution used in the treatment with the single-stranded DNA exonuclease may be a buffer solution of any composition that provides conditions in which the single-stranded DNA exonuclease can act.

Furthermore, the step of removing the oriC transposon may also include a treatment using a restriction enzyme that corresponds with a restriction enzyme site contained in the sequence of the oriC transposon. The purpose of this treatment is to specifically cleave the oriC transposon. Accordingly, in this case, a restriction enzyme is selected which corresponds with a restriction enzyme site that is included in the oriC transposon but is not included in any region outside the oriC transposon region in the replicated or amplified circular DNA. CRISPR-Cas9 may be used instead of a restriction enzyme for double strand cleavage that is specific to the region contained in the oriC transposon. In such a case, a sequence specific to the region contained in the oriC transposon is designated as a guide RNA.

In another embodiment, step (2) of the present invention may be conducted by rolling circle amplification (also referred to as the "RCA method").

The RCA method is a method of amplifying a circular DNA using a DNA polymerase having strand substitution activity, and a first and second primer. The RCA method may be conducted using conventional methods, and for example, may be conducted in accordance with the following procedure. First, the first primer is hybridized with the circular DNA, and then used as an origin for conducting an elongation reaction using the DNA polymerase having strand substitution activity. Even when the DNA polymerase having strand substitution activity reaches the polymerase reaction origin of the circular DNA, the synthesis proceeds while the already synthesized strand is detached. As a result, single-stranded DNA in which the DNA sequence of a single coil of the original circular DNA is repeatedly joined to itself is obtained as an amplified product. The second primer is hybridized with this single-stranded DNA. A plurality of the second primers may be hybridized with one single-stranded DNA. By conducting elongation reactions from this plurality of second primers using the DNA polymerase having strand substitution activity, synthesis of a complementary strand proceeds while the DNA synthesized from a second primer hybridized at an upstream location detaches the DNA synthesized from a second primer hybridized at a downstream location. The first primer then hybridizes with the single-stranded DNA synthesized from the second primer, and a new replication reaction occurs. In this manner, the sequence of the original circular DNA is amplified exponentially, and DNA multimers are obtained in which the original circular DNA sequence is joined repeatedly. By cleaving these multimers with a suitable DNA cleavage enzyme, and conducting circularization using a DNA ligase, monomeric replicated products of the original circular DNA can be obtained. The monomeric replicated products of the original circular DNA may also be obtained by circularization of the DNA multimers using a site-specific recombination system such as Cre-loxP.

The "first primer" is a primer that binds to the target circular DNA, whereas the "second primer" is a primer that binds to a sequence that is complementary to the circular DNA to which the first primer binds. Random primers may be used as the first and second primers. The length of the primers is typically about 6 to 9 bases.

Conventional enzymes may be used as the DNA polymerase having strand substitution activity, and examples include Phi29 bacteriophage DNA polymerase, Bst DNA polymerase, Csa DNA polymerase, and 96-7 DNA polymerase. Commercially available enzymes may also be used. The reaction conditions may be set appropriately in accordance with the DNA polymerase that is used.

### II. Kit for Editing DNA in Cell-Free System

The present invention also provides a kit containing components required for introducing a deletion, substitution or addition at a target site of a DNA in a cell-free system, and components required for amplifying, in a cell-free system, the DNA into which a deletion, substitution or addition has been introduced.

The components required for introducing a deletion, substitution or addition at a target site of the DNA vary depending on the techniques used for implementing the method of the present invention, but for example, may include a single-stranded DNA (such as a chemically synthesized oligonucleotide) containing the desired mutation (for example, a substitution, deletion or addition), and a DNA polymerase and the like. Further, in those cases where the RA method is used, examples of the components included in the kit of the present invention include a RecA family recombinase protein, an exonuclease, a nucleoside triphosphate or deoxynucleotide triphosphate regenerating enzyme and a substrate therefor, a nucleoside triphosphate, a deoxynucleotide triphosphate, a magnesium ion source, an alkali metal ion source, dimethyl sulfoxide, tetramethylammonium chloride, polyethylene glycol, dithiothreitol, and a buffer solution.

The components required for amplifying, in a cell-free system, the DNA into which a deletion, substitution or addition has been introduced vary depending on the techniques used for implementing the method of the present invention. For example, in those cases where the RCR method is used, examples of the components contained in the kit of the present invention include one or more enzymes or enzyme groups selected from the group consisting of replication initiation proteins, one or more types of nucleoid proteins, enzymes or enzyme groups having DNA gyrase activity, single-stranded DNA binding proteins (SSB), enzymes having DNA helicase activity (such as enzymes having DnaB helicase activity), enzymes having DNA helicase loader activity, enzymes having DNA primase activity, enzymes having DNA clamp activity, and enzymes or enzyme groups having DNA polymerase III* activity.

The kit of the present invention may also include additional constituent components such as a reaction buffer or a concentrate thereof, depending on the technique used for implementing the method of the present invention. Examples of these additional constituent components include components required for implementing the RCR methods I to V described above.

The kit of the present invention may also include an artificial DNA cleavage enzyme for specifically cleaving DNA into which a deletion, substitution or addition has not been introduced.

The kit of the present invention may also include documentation describing the protocol for implementing the method or the present invention using the kit. The protocol may be recorded on the surface of the container that houses the kit.

### EXAMPLES

The present invention is described below in further detail based on a series of examples. However, the present invention is not limited by the scope of the following examples.

### [Example 1] Confirmation of Sequence Specificity of CRISPR-Cas9

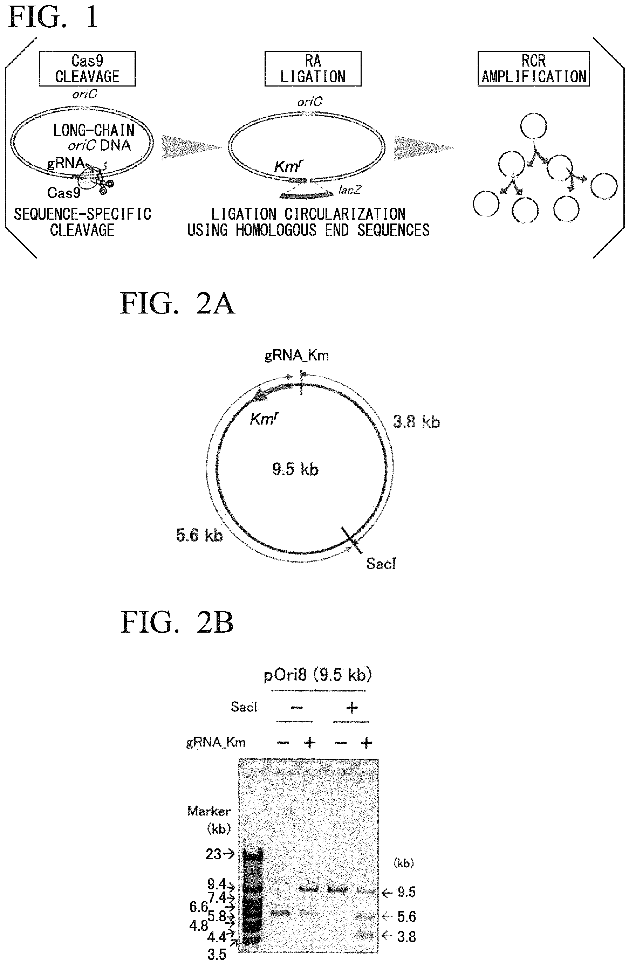

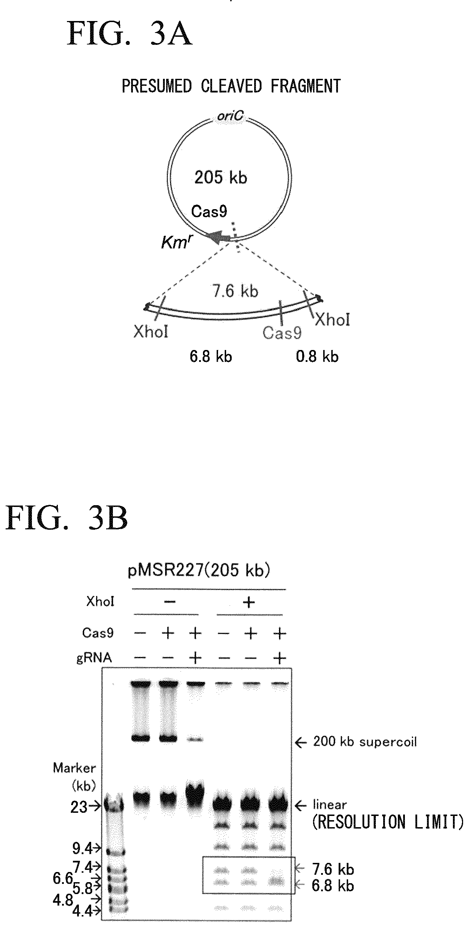

Using a 9.5 kb plasmid (pOri8) or a 205 kb plasmid (pMSR227), the sequence specificity of cleavage by CRISPR-Cas9 was confirmed. The plasmids pOri8 (FIG. 2A) and pMSR227 (FIG. 3A) are plasmids disclosed in Su'etsugu et al, Nucleic Acids Res. 2017 Nov 16;45(20): 11525 to 11534.

Ten ng/µL of pOri8 or pMSR227, 100 nM of gRNA_Km (a guide RNA prepared in accordance with the Alt-R (a registered trademark) CRISPR-Cas9 system from Integrated DNA Technologies, Inc., recognition sequence: UGGUUAAUUGGUUGUAACAC (SEQ ID NO: 1)), 20 nM of Cas9 (Alt-R (a registered trademark) S.p. HiFi Cas9 Nuclease 3NLS, Integrated DNA Technologies, Inc.), 0.8 U/µL of RNase inhibitor murine (M0314, New England Biolabs Ltd.), and an R8 buffer (added to bring the total volume of the mixture up to 10 µL) (composition as shown in Table 2) were mixed, and the resulting mixture was incubated at 30°C for 30 minutes. Subsequently, 10 U of the restriction enzyme SacI (in the case of pOri8) or XhoI (in the case of pMSR227) was added, and the mixture was incubated at 37°C for one hour. The concentration of each of the components in the reaction solution represents a concentration relative to the total volume of the reaction solution. Next, 0.5% agarose gel electrophoresis was conducted, and DNA fragments were detected by SYBR green I staining.

As illustrated in FIG. 2B, the pOri8 supercoiled DNA was cleaved and linearized dependent on the gRNA_Km having a recognition sequence downstream from a kanamycin resistance gene. The result of the SacI treatment revealed that, as expected, cleavage of the 9.5 kb fragment produced fragments of 5.6 kb and 3.8 kb.

Further, as illustrated in FIG. 3B, the pMSR227 supercoiled DNA was cleaved and linearized dependent on Cas9 and the gRNA_Km. The result of the XhoI treatment revealed that, as expected, the 7.6 kb fragment had disappeared. The 6.8 kb fragment produced by cleavage of the 7.6 kb fragment by XhoI overlapped with another Xhol fragment, but the band was confirmed as having broadened.

### [Example 2] CRISPR-RCR

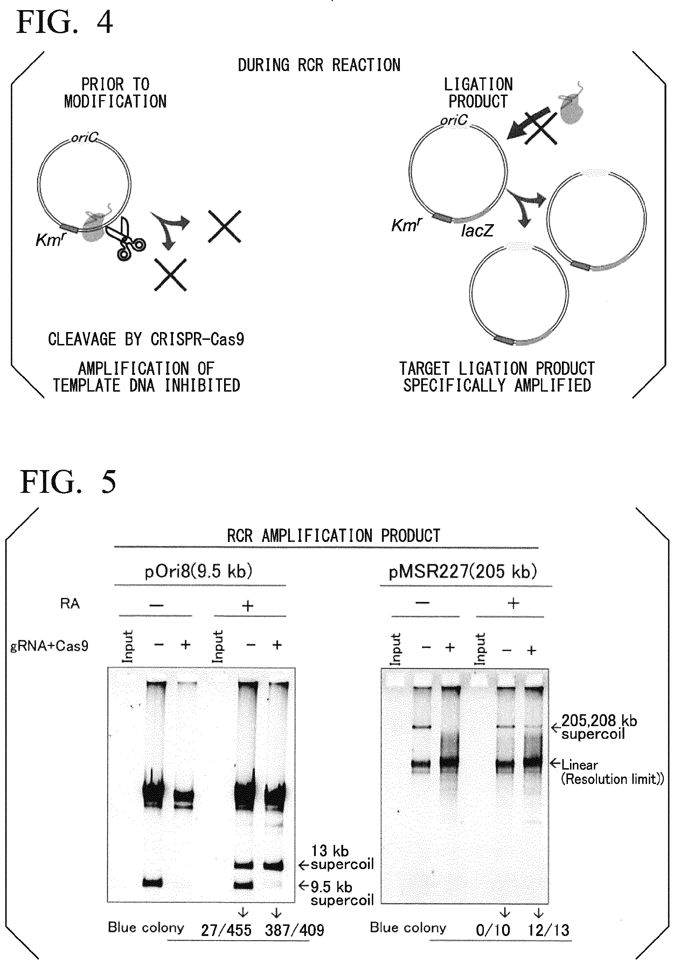

For the DNA amplification reaction following the RA reaction, by conducting RCR in the presence of CRISPR-Cas9, it was confirmed that amplification of the unmodified template DNA was inhibited, and the target ligation product was able to be specifically amplified (FIG. 4).

First, the template DNA (pOri8 or pMSR227) was cleaved and linearized by Cas9 using the reaction described below. Namely, 10 ng/µL of pOri8 (9.5 kb) or pMSR227 (205 kb), 100 nM of gRNA_Km, 20 nM of Cas9, 0.8 U/µL of RNase inhibitor murine and an R8 buffer (added to bring the total volume of the mixture up to 5 µL) were mixed, and the resulting mixture was incubated at 30°C for 30 minutes. The concentration of each of the components in the reaction solution represents a concentration relative to the total volume of the reaction solution.

Next, using the RA reaction described below, the Cas9-cleaved fragment and an lacZ fragment (prepared using the method described below) were joined. The Cas9cleavage reaction solution described above (0.5 µL) containing 5 ng of the Cas9-cleaved fragment, a lacZ fragment (1.7 ng in the case of pOri8, 0.09 ng in the case of pMSR227), 50 ng/µL of tRNA (R8759, Sigma-Aldrich Corporation), and an RA reaction solution (of the composition described below, added to bring the total volume of the mixture up to 5 µL) were mixed, and following incubation at 42°C for one hour and then incubation at 65°C for 2 minutes, the mixture was left to stand on ice. The concentration of each of the components in the reaction solution represents a concentration relative to the total volume of the reaction solution.

Preparation of the lacZ fragment: using pPKOZ (Su'etsugu et al, Nucleic Acids Res. 2017 Nov 16;45(20): 11525 to 11534) as the template, and using primers SUE1510 and SUE1511, PCR was conducted to prepare a lacZ prefragment. Using this lacZ prefragment as a template, and using primers SUE1638 and SUE1639, PCR was conducted to prepare a lacZ fragment having and end homologous with the 60 base pairs (a sequence common to both pOri8 and pMSR227) at the CRISPR-Cas9-cleaved end downstream from the kanamycin resistance gene.

**[Table 1]**

| Primer Sequences: Homologous End Sequences shown as Uppercase | | | |
|---|---|---|---|
| Name | Sequence | SEQ ID NO: | Remarks |
| SUE1510 | CGATGAGTTTTTCTAATCAGAATTGGTTAA TTGGTTGTAActttagttacaacatactacgacaggtttcccgactg | 3 | lacZ prefragment production (overlap 40 bp) |
| SUE1511 | AGCCGCCGTCCCGTCAAGTCAGCGTAATGC TCTGCCAGTGctttagttacaacatacttttccttacgcgaaatacgg | 4 | lacZ prefragment production (overlap 40 bp) |
| SUE1638 | TTGCAGTTTCATTTGATGCTCGATGAGTTT TTCTAATCAGAATTGGTTAATTGG | 5 | lacZ fragment production (overlap 60 bp) |
| SUE1639 | AAGTTCGATTTATTCAACAAAGCCGCCGTC CCGTCAAGT | 6 | lacZ fragment production (overlap 60 bp) |
| SUE1753 | TTGCAGTTTCATTTGATGCTCGATGAGTTT TTCTAATCAGAATTGGTTAATTGGTTGTAA atatgagtaaacttggtctgacag | 7 | Long-chain DNA ligation adapter (Amp) |
| SUE1756 | AAGTTCGATTTATTCAACAAAGCCGCCGTC CCGTCAAGTCAGCGTAATGCTCTGCCAGTG tttccttacgcgaaatacgg | 8 | Long-chain DNA ligation adapter (lacZ) |
| SUE1822 | CGCATCCGGCAGGGTTTATTATTGTTTTTT CGCTCTAGGTCGGCTTGAGAAAGACCTGAG cacttttcggggaaatgtgc | 9 | Long-chain DNA ligation adapter (Amp) |
| SUE1823 | TTTAATACCCAGGATCCATTTAACATAATA TACATTATGCGCACCTTTAGTTACAACATA acgacaggtttcccgactg | 10 | Long-chain DNA ligation adapter (lacZ) |

RA reaction solution composition: 1 µM of a wild-type RecA (prepared from a RecA E. coli expression strain, and purified by steps including polyethyleneimine precipitation, ammonium sulfate precipitation and affinity column chromatography), 80 mU/µL of an exonuclease III (2170A, Takara Bio Inc.), 1 U/µL of an exonuclease I (M0293, New England Biolabs Ltd.), 20 mM of Tris-HCl (pH 8.0), 4 mM of DTT, ImM of magnesium acetate, 50 mM of potassium glutamate, 100 µM of ATP, 150 mM of tetramethylammonium chloride (TMAC), 5% by mass of PEG8000, 10% by volume of DMSO, 20 ng/µL of creatine kinase (10127566001, Sigma-Aldrich Corporation), and 4 mM of creatine phosphate. The concentration of each of the components in the RA reaction solution represents a concentration relative to the total volume of the RA reaction solution.

The RA reaction product was subjected to an RCR amplification reaction in the following manner. Namely, a RCR reaction solution ver. 2 (of the composition described below, wherein the concentration of each of the components in the RCR reaction solution ver. 2 represents a concentration relative to the total volume of the RCR reaction solution ver. 2, added to bring the total volume of the mixture up to 4.5 µL), 100 nM of gRNA_Km, and 1 nM of Cas9 were mixed, and the resulting mixture was preincubated at 30°C for 30 minutes. The concentrations of the gRNA_Km and the Cas9 in the reaction solution represent concentrations relative to the total volume of the reaction solution. When the reaction was conducted in the absence of CRISPR-Cas9, the 100 nM of gRNA_Km, and 1 nM of Cas9 were not added, and additional RCR reaction solution ver. 2 was added to make up the volume. Subsequently, 0.5 µL of the RA reaction product was added, a cycle that involved incubation at 37°C for one minute and then incubation at 24°C for 30 minutes was repeated 40 times, and then the resultant was diluted 5-fold with R8 buffer and an additional incubation was conducted at 30°C for 30 minutes. Subsequently, 1 µL of the RCR product was supplied to 0.5% agarose gel electrophoresis and detected by SYBR Green I staining.

**[Table 2]**

| RCR Reaction Solution ver. 2 | |
|---|---|
| R8 buffer | Final concentration |
| Tris-Hcl (pH 8.0) | 20 mM |
| KOAc | 150 mM |
| Mg(OAc)₂ | 10 mM |
| DTT | 4 mM |
| Creatine phosphate | 4 mM |
| Ammonium sulfate | 4 mM |
| ATP | 1 mM |
| G, C, UTPs | each 1 mM |
| tRNA | 50 ng/µL |
| NAD | 0.25 mM |
| dNTPs | each 0.25 mM |
| Tiron | 0.25 mM |

| Enzyme mixture | Final concentration |
|---|---|
| BSA | 0.5 mg/mL |
| Creatine kinase | 20 ng/µL |
| SSB | 400 nM |
| IHF | 20 nM |
| DnaG | 400 nM |
| Clamp | 40 nM |
| PolIII* | 5 nM |
| DnaB-DnaC | 20 nM |
| DnaA | 100 nM |
| RNaseH | 10 nM |
| Ligase | 50 nM |
| Poll | 50 nM |
| GyrA, GyrB | 50 nM |
| Topo IV | 5 nM |
| Topo III | 50 nM |
| RecQ | 50 nM |
| RecG | 60 nM |
| RecJ | 0.5 U/µL |
| ExoI | 0.2 U/µL |
| ExoIII | 60 mU/µL |
| Lambda DNA | 3 ng/µL |

In the table, SSB represents E. coli-derived SSB, IHF represents an E. coli-derived complex of IhfA and IhfB, DnaG represents E. coli-derived DnaG, Clamp represents E. coli-derived DnaN, PolIII* represents a DNA polymerase III* complex that is a complex formed from E. coli-derived DnaX, HolA, HolB, HolC, HolD, DnaE, DnaQ and HolE, DnaB represents E. coli-derived DnaB, DnaC represents E. coli-derived DnaC, DnaA represents E. coli-derived DnaA, RNaseH represents E. coli-derived RNaseH, Ligase represents E. coli-derived DNA ligase, Poll represents E. coli-derived DNA polymerase 1, GyrA represents E. coli-derived GyrA, GyrB represents E. coli-derived GyrB, Topo IV represents a complex of E. coli-derived ParC and ParE, Topo III represents E. coli-derived topoisomerase III, RecQ represents E. coli-derived RecQ, RecG represents E. coli-derived RecG, RecJ represents E. coli-derived RecJ, ExoI represents E. coli-derived ExoI, and ExoIII represents E. coli-derived ExoIII.

SSB was prepared from an SSB E. coli expression strain, and purified by steps including ammonium sulfate precipitation and ion exchange column chromatography.

IHF was prepared from an IhfA and IhfB E. coli expression strain, and purified by steps including ammonium sulfate precipitation and affinity column chromatography.

DnaG was prepared from an DnaG E. coli expression strain, and purified by steps including ammonium sulfate precipitation, anion exchange column chromatography and gel permeation column chromatography.

Clamp was prepared from a DnaN (Clamp) E. coli expression strain, and purified by steps including ammonium sulfate precipitation and anion exchange column chromatography.

PolIII* was prepared from a DnaX, HolA, HolB, HolC, HolD, DnaE, DnaQ and HolE E. coli expression strain, and purified by steps including ammonium sulfate precipitation, affinity column chromatography and gel permeation column chromatography.

DnaB and DnaC were prepared from a DnaB and DnaC E. coli expression strain, and purified by steps including ammonium sulfate precipitation, affinity column chromatography and gel permeation column chromatography.

DnaA was prepared from a DnaA E. coli expression strain, and purified by steps including ammonium sulfate precipitation, dialysis precipitation and gel permeation column chromatography.

GyrA and GyrB were prepared from a mixture of a GyrA E. coli expression strain and a GyrB E. coli expression strain, and purified by steps including ammonium sulfate precipitation, affinity column chromatography and gel permeation column chromatography.

Topo IV was prepared from a mixture of a ParC E. coli expression strain and a ParE E. coli expression strain, and purified by steps including ammonium sulfate precipitation, affinity column chromatography and gel permeation column chromatography.

Topo III was prepared from a Topo III E. coli expression strain, and purified by steps including ammonium sulfate precipitation and affinity column chromatography.

RecQ was prepared from a RecQ E. coli expression strain, and purified by steps including ammonium sulfate precipitation, affinity column chromatography and gel permeation column chromatography.

RecG was prepared from a RecG E. coli expression strain, and purified by steps including ammonium sulfate precipitation and affinity column chromatography.

RecJ used a commercially available enzyme (M0264, New England Biolabs Ltd.)

ExoI used a commercially available enzyme (see the composition of the above RA reaction solution).

ExoIII used a commercially available enzyme (see the composition of the above RA reaction solution).

RNaseH, Ligase and Poll each used a commercially available E. coli-derived enzyme (Takara Bio Inc.).

Further, by transforming the RCR product into E. coli and conducting a blue-white determination of the colonies, the proportion of lacZ-inserted product within the RCR product was investigated. In the case of pOri8, 1 µL of the RCR product was diluted 10-fold with TE buffer, and 1 µL of the diluted solution was then used to transform an E. coli DH5α strain using a chemical method. In the case of pMSR227, 1 µL of the RCR product was diluted 1 0-fold with TE buffer, and 2 µL of the diluted solution was then used to transform an E. coli HST08 strain using an electroporation method. Following transformation, each E. coli was seeded onto an LB plate containing 50 µg/mL of kanamycin, 0.1 mM (mmol/L) of IPTG and 40 µg/mL of X-gal relative to the total volume of the LB plate, and the resulting mixture was incubated overnight at 37°C. The proportion of the number of blue colonies relative to the total number of colonies was calculated.

The results are illustrated in FIG. 5. In the case of pOri8, the 13 kb supercoiled DNA that represented the target lacZ-inserted product underwent RA-dependent RCR amplification. Amplification of the premodification 9.5 kb supercoiled DNA was suppressed in the presence of the gRNA and Cas9. The results of the blue-white determination of the transformant colonies also revealed a proportion of blue colonies (shown as "Blue colony" in the figure) of less than 6% in the absence of gRNA and Cas9, which increased to 95% in the presence of the gRNA and Cas9. In the figure, "Input" represents a sample that has not undergone RCR amplification.

In the case of pMSR227, a 208 kb supercoiled DNA that is thought to represent the target lacZ-inserted product was amplified by RCR in the presence of gRNA and Cas9. Further, the results of the blue-white determination of the transformant colonies revealed a proportion of blue colonies of 0% in the absence of gRNA and Cas9, which increased to 12 colonies among 13 in the presence of the gRNA and Cas9, confirming that the target lacZ-inserted product had been obtained with high efficiency.

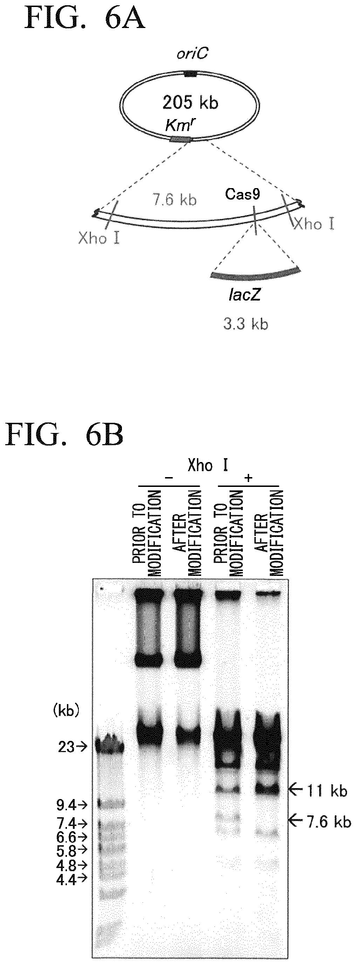

Moreover, the plasmid was extracted from a blue colony obtained from the sample that had undergone insertion modification of lacZ (3.3 kb) into pMSR227, and a structural confirmation was conducted by cleavage with the restriction enzyme XhoI (FIG. 6A). As expected, the 7.6 kb fragment observed prior to modification disappeared and the band near 11 kb darkened, confirming formation of the lacZ-inserted product (FIG. 6B).

### [Example 3] Cell-Free Synthesis of Long-Chain DNA

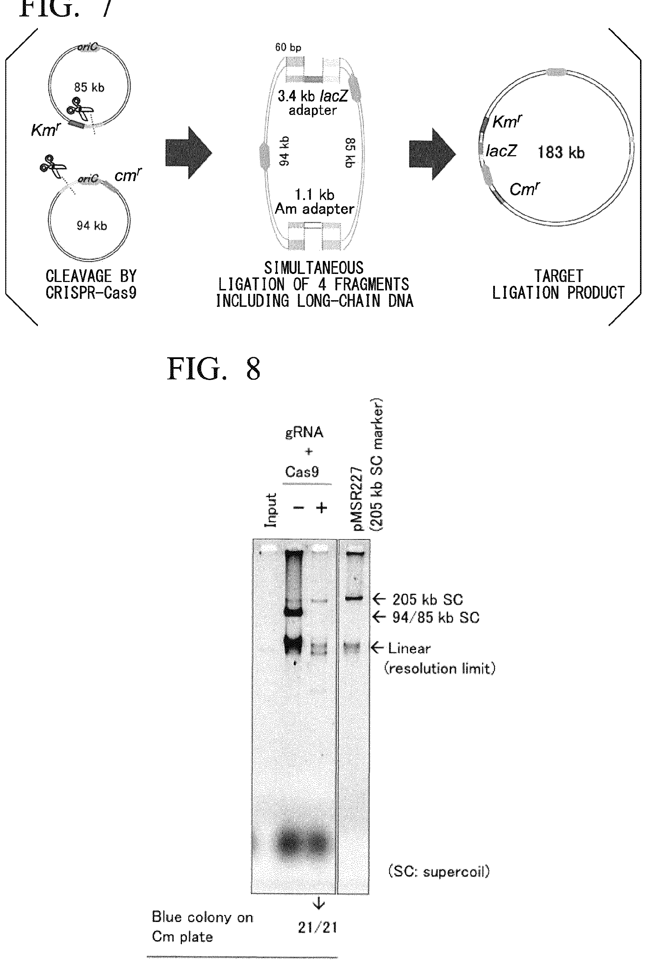

Using the RCR amplification method and the RA ligation method, two long-chain circular DNAs were joined, and the resulting longer chain circular DNA was amplified (FIG. 7).

First, pOri80 (85 kb) and pOri93Cm (94 kb) were cleaved and linearized at specific sites by CRISPR-Cas9 using the following method. Namely, 10 ng/µL of pOri80 (85 kb) or pOri93Cm (94 kb), 100 nM of gRNA_Km (in the case of pOri80) or gRNA_007 (in the case of pOri93Cm, a guide RNA prepared in accordance with the Alt-R (a registered trademark) CRISPR-Cas9 system from Integrated DNA Technologies, Inc., recognition sequence: CCUUUAGUUACAACAUACUC (SEQ ID NO: 2)), 20 nM of Cas9, 0.8 U/µL of RNase inhibitor murine, and an R8 buffer (added to bring the total volume of the mixture up to 5 µL) were mixed, and the resulting mixture was incubated at 30°C for 30 minutes. The concentration of each of the components in the reaction solution represents a concentration relative to the total volume of the reaction solution.

The pOri93Cm was prepared by the following method. Namely, the 93 kb genome fragment from the XbaI digestion product of the genome DNA from an E. coli strain (DGF-298WΔ100::revΔ234::SC) was joined and circularized with a ligation fragment containing oriC and a chloramphenicol resistance gene (Cm-oriC fragment, 1.3 kb, SEQ ID NO: 11) using the RA method. The sequences of 60 base pairs at the two ends of the Cm-oriC fragment are homologous with the two respective ends of the 93 kb genome fragment. Following RCR amplification of the circular DNA, an E. coli transformation was conducted, and the purified supercoiled plasmid from the obtained E. coli colony was used as the pOri93Cm.

The obtained two long-chain DNA fragments were joined by the following RA reaction using an lacZ adapter (3.4 kb) and an Am adapter (1.1 kb). Specifically, 5 ng (0.5 µL) of the Cas9-cleaved fragment of pOri80, 5 ng (0.5 µL) of the Cas9-cleaved fragment of pOri93Cm, the lacZ adapter (3.4 kb, 27 pg), the Am adapter (1.1 kb, 86 ng), 50 ng/µL of tRNA, and the RA reaction solution (see Example 2, added to bring the total volume of the mixture up to 5 µL) were mixed, and following incubation at 42°C for 3 hours and then incubation at 65°C for 2 minutes, the mixture was left to stand on ice. The concentration of each of the components in the reaction solution represents a concentration relative to the total volume of the reaction solution.

The sequences of 60 base pairs at both ends of each adapter are homologous sequences with the two ends of the two long-chain DNA fragments, and by ligation and circularization of the four fragments by RA, a DNA with a total length of 183 kb was formed (FIG. 7).

The lacZ adapter was prepared by conducting PCR using pPKOZ as the template, and using primers SUE1756 and SUE1823.

The Am adapter was prepared by conducting PCR using a pUC4K plasmid (GE Healthcare Inc.) as the template, and using primers SUE1753 and SUE1822.

Following the ligation circularization, an RCR amplification reaction was conducted in the following manner. The RCR reaction solution ver. 2 (added to bring the total volume of the mixture up to 4.5 µL), 100 nM of gRNA_Km, 100 nM of gRNA_007 and 2 nM of Cas9 were mixed, and the resulting mixture was preincubated at 30°C for 30 minutes. The concentrations of the gRNA_Km, gRNA_007 and Cas9 in the reaction solution each represent a concentration relative to the total volume of the reaction solution. When the reaction was conducted in the absence of CRISPR-Cas9, the 100 nM of gRNA_Km, 100 nM of gRNA-007 and 2 nM of Cas9 were not added, and additional RCR reaction solution ver. 2 was added to make up the volume. Subsequently, 0.5 µL of the RA reaction product was added, a cycle that involved incubation at 37°C for one minute and then incubation at 24°C for 30 minutes was repeated 40 times, and then the resultant was diluted 5-fold with R8 buffer and an additional incubation was conducted at 30°C for 30 minutes. Subsequently, 1 µL of the RCR product was supplied to 0.5% agarose gel electrophoresis and detected by SYBR Green I staining.

Further, by transforming the RCR product into E. coli and conducting a blue-white determination of the colonies, the proportion of target joined product within the RCR product was investigated. Specifically, 1 µL of the RCR product was diluted 10-fold with TE buffer, and 2 µL of the diluted solution was then used to transform an E. coli HST08 strain using an electroporation method. Following transformation, the E. coli was seeded onto an LB plate containing 12.5 µg/mL of chloramphenicol, 0.1 mM of IPTG and 40 µg/mL of X-gal relative to the total volume of the LB plate, and the resulting mixture was incubated overnight at 37°C. The proportion of the number of blue colonies relative to the total number of colonies was calculated (blue colony).

The results are illustrated in FIG. 8. In the case where RCR was conducted in the absence of the gRNA and Cas9, the premodification 94 kb and 85 kb supercoiled DNAs were mainly amplified. In contrast, when the RCR was conducted in the presence of the gRNA and Cas9, amplification of the 94 kb and 85 kb DNAs was suppressed, and DNA amplification was observed at a size close to a 205 kb supercoiled DNA that was subjected to RCR amplification as a control (pMSR227). In the figure, "Input" represents a sample prior to RCR amplification.

An E. coli transformation was conducted using this amplified product, and the results of using a blue-white determination to detect those colonies having the lacZ gene among all of the chloramphenicol-resistant colonies revealed that all 21 colonies were the target blue colonies (in the figure, labeled as "Blue colony on Cm plate").

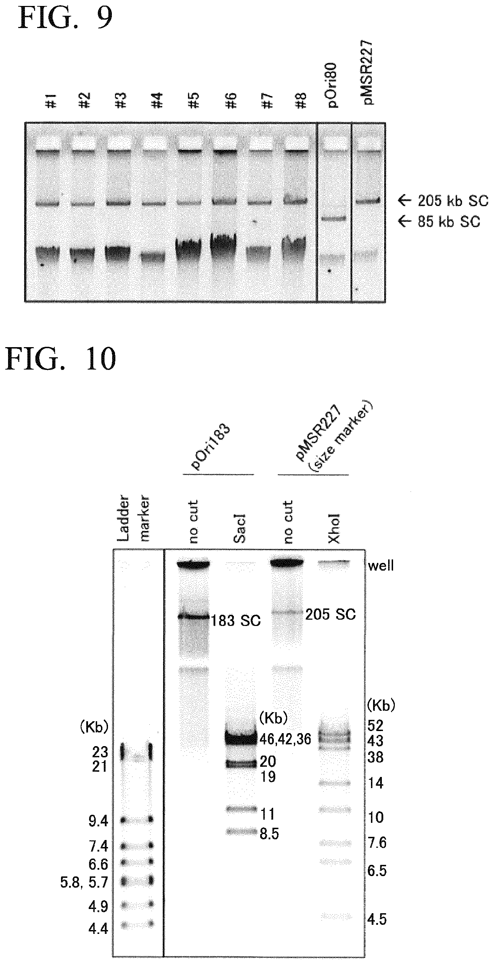

Eight of the obtained blue colonies were selected, the plasmid was extracted from each of the colonies, and when the size was confirmed by agarose gel electrophoresis, all of the colonies exhibited a size close to the expected 205 kb supercoiled DNA (FIG. 9).

Moreover, one of the obtained plasmids (named pOri183) was cleaved with the restriction enzyme SacI, and the structure was confirmed by pulsed-field gel electrophoresis (FIG. 10). A result that substantially matched the cleavage pattern (sizes shown to the right of the bands) expected from the base sequence was obtained. For size markers, not only was a Ladder marker used, but the supercoiled (SC) product of pMSR227 and the XhoI-cleaved product thereof, for which the structures are known, were also subjected to simultaneous electrophoresis.

### [Example 4] Construction of Base Substitution Introduction Method Combined with RCR

First, an investigation was conducted as to whether a base substitution introduction method using an oligo DNA could be applied to RCR.

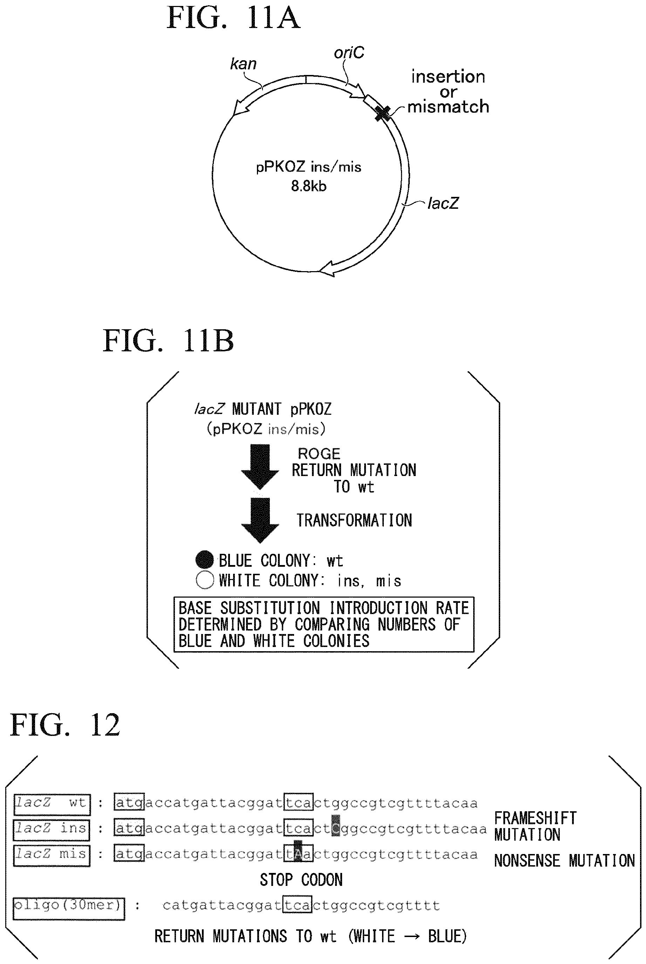

The test system is illustrated in FIG. 11B and FIG. 12. The lacZ-modified pPKOZ (pPKOZins and pPKOZmis, FIG. 11A) was prepared by the QuikChange PCR method using pPKOZ (8.8 kb) as a template.

In the preparation of pPKOZins, SUE818 and SUE819 were used as primers. pPKOZins is a plasmid having one base inserted in the coding region of lacZ (FIG. 12). Because of the frameshift mutation, wild-type lacZ is not expressed, and therefore E. coli colonies transformed with pPKOZins do not appear blue in the blue-white determination (FIG. 11B).

In the preparation of pPKOZmis, SUE1415 and SUE1416 were used as primers. pPKOZmis is a plasmid having one base substituted in the coding region of lacZ (FIG. 12). Because of the nonsense mutation, wild-type lacZ is not expressed, and therefore E. coli colonies transformed with pPKOZmis do not appear blue in the blue-white determination (FIG. 11B).

SUE1354 to SUE1357 were used as oligo DNAs for returning these mutant lacZ to wild-type lacZ.

**[Table 3]**

| Primer Sequences: Substituted (Inserted) Bases shown as Uppercase | | | |
|---|---|---|---|
| Name | Sequence | SEQ ID NO: | Remarks |
| SUE818 | gattac ggattcactC ggccgtcgttttac | 12 | pPKOZins production |
| SUE819 | gtaaaacgacggccGagtgaatccgtaatc | 13 | pPKOZins production |
| SUE1415 | ccatgattacggattAactggccgtcgttt | 14 | pPKOZmis production |
| SUE1416 | aaacgacggccagtTaatccgtaatcatgg | 15 | pPKOZmis production |
| SUE1354 | ttacggattcactggccgtc | 16 | Modification oligo DNA (20 mer) |
| SUE1355 | catgattacggattcactggccgtcgtttt | 17 | Modification oligo DNA (30 mer) |
| SUE1356 | atgaccatgattacggattcactggccgtcgttttacaac | 18 | Modification oligo DNA (40 mer) |
| SUE1357 | ggaaacagctatgaccatgattacggattcactggccgtcg ttttacaacgtcgtgactg | 19 | Modification oligo DNA (60 mer) |

Using the pPKOZins having a lacZ frameshift mutation as a template, an RCR reaction was conducted in the presence of 20 mer to 60 mer modification oligo DNAs in the following manner. Namely, an RCR reaction solution ver. 1 (of the composition described below, wherein the concentration of each of the components in the RCR reaction solution ver. 1 represents a concentration relative to the total volume of the RCR reaction solution ver. 1, added to bring the total volume of the mixture up to 5 µL), 1 or 3 µM of the modification oligo DNAs (20 to 60 mer), and 75 pg/µL of pPKOZins were mixed, and the resulting mixture was incubated at 30°C for 18 hours. The concentration of each of the components in the reaction solution represents a concentration relative to the total volume of the reaction solution. Subsequently, 1 µL of the RCR product was supplied to 0.5% agarose gel electrophoresis and detected by SYBR Green I staining.

**[Table 4]**

| RCR Reaction Solution ver. 1 (not including promotion factors) | |
|---|---|
| R8 buffer | Final concentration |
| Tris-Hcl (pH 8.0) | 20 mM |
| KOAc | 150 mM |
| Mg(OAc)₂ | 10 mM |
| DTT | 4 mM |
| Creatine phosphate | 4 mM |
| Ammonium sulfate | 4 mM |
| ATP | 1 mM |
| G, C, UTPs | each 1 mM |
| tRNA | 50 ng/µL |
| NAD | 0.25 mM |
| dNTPs | each 0.25 mM |
| Tiron | 0.25 mM |

| Enzyme mixture | Final concentration |
|---|---|
| BSA | 0.5 mg/mL |
| Creatine kinase | 20 ng/µL |
| SSB | 400 nM |
| IHF | 20 nM |
| DnaG | 400 nM |
| Clamp | 40 nM |
| PolIII* | 5 nM |
| DnaB-DnaC | 20 nM |
| DnaA | 100 nM |
| RNaseH | 10 nM |
| Ligase | 50 nM |
| Poll | 50 nM |
| GyrA, GyrB | 50 nM |
| Topo IV | 5 nM |
| Topo III | 50 nM |
| RecQ | 50 nM |

The components in the RCR reaction solution ver. 1 are the same as those described above for the RCR reaction solution ver. 2.

Furthermore, by transforming the RCR product into E. coli and conducting a blue-white determination of the colonies, the proportion of the lacZ mutation modified to the wild-type form within the RCR product was investigated. Specifically, 0.25 µL of the RCR product was used to transform an E. coli DH5α strain using a chemical method. Following transformation, the E. coli was seeded onto an LB plate containing 25 µg/mL of kanamycin, 0.1 mM of IPTG and 40 µg/mL of X-gal, and the resulting mixture was incubated overnight at 37°C. The number of blue colonies relative to the total number of colonies was calculated.

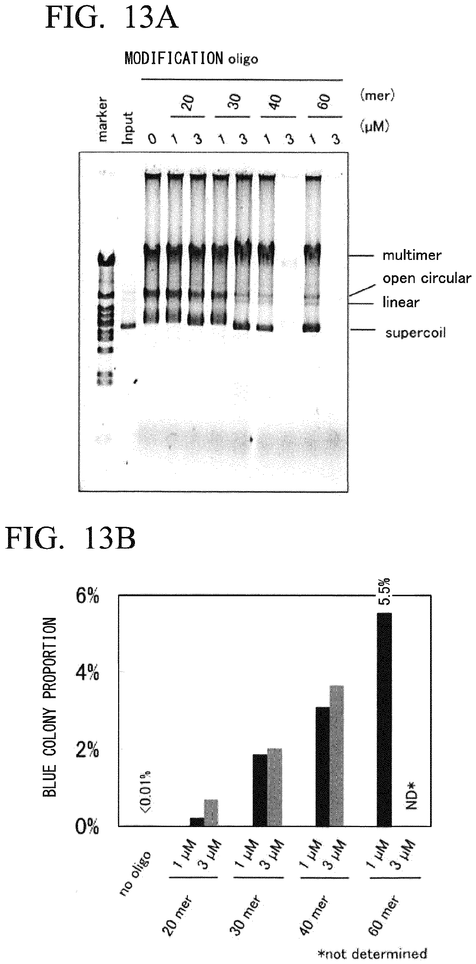

The results are illustrated in FIG. 13A and FIG. 13B. With the exception of the cases where 30 µM of the 40 mer or 60 mer modification oligo DNA was added, satisfactory amplification was observed (FIG. 13A). In the figure, "Input" represents the case that was flushed with 3.8 ng of the template DNA.

Further, conducting a blue-white determination of the E. coli transformant colonies confirmed that modification of the lacZ mutation to the wild-type occurred with the highest efficiency of 5.5% in the sample to which 1 µM of the 60 mer modification oligo DNA had been added.

### [Example 5] Concentration Investigation for Oligo DNA 60 mer (ins/mis)

In order to improve the modification efficiency, the usage concentration of the oligo DNA was investigated.

Using the following method, pPKOZins or pPKOZmis was used as a template, and RCR reactions were conducted in the presence of different concentrations of the 60 mer modification oligo DNA. The RCR reaction solution ver. 1 (added to bring the total volume of the mixture up to 5 µL), 0 to 3 µM of the 60 mer modification oligo DNA (SUE1357), and 75 pg/µL of pPKOZins or 14 pg/µL of pPKOZmis were mixed, and the resulting mixture was incubated at 30°C for 19 hours. Subsequently, 1 µL of the RCR product was supplied to 0.5% agarose gel electrophoresis and detected by SYBR Green I staining.

Further, by transforming the RCR product into E. coli and conducting a blue-white determination of the colonies, the proportion of the lacZ mutation modified to the wild-type form within the RCR product was investigated. Specifically, 0.25 µL of the RCR product was used to transform an E. coli DH5α strain using a chemical method. Following transformation, the E. coli was seeded onto an LB plate containing 25 µg/mL of kanamycin, 0.1 mM of IPTG and 40 µg/mL of X-gal relative to the total volume of the LB plate, and the resulting mixture was incubated overnight at 37°C. The number of blue colonies relative to the total number of colonies was calculated.

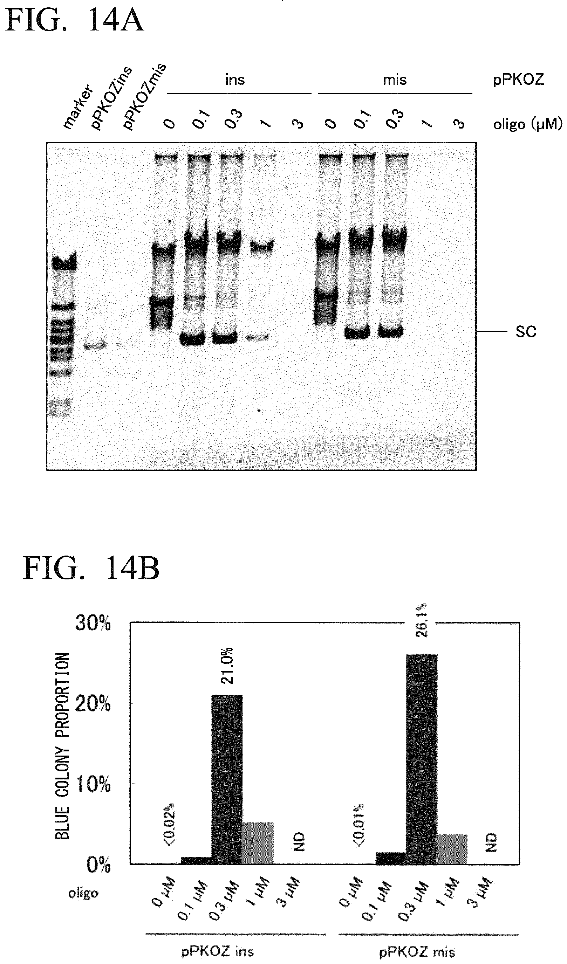

The results are illustrated in FIG. 14A and FIG. 14B. In FIG. 14A, "Input" represents the cases flushed with 3.8 ng of pPKOZins or 0.72 ng of pPKOZmis. The results of conducting blue-white determinations of the E. coli transformant colonies confirmed that, for either of pPKOZins or pPKOZmis, modification of the lacZ mutation to the wild-type occurred with the highest efficiency of 20% or higher in the sample to which 0.3 µM of the modification oligo DNA had been added (FIG. 14B).

### [Example 6] Increasing Efficiency of ROGE using CRISPR-Cas9

Using the same test system as Example 5, RCR was conducted in the presence of CRISPR-Cas9 to investigate whether the efficiency of the modification of the lacZ mutation to the wild-type could be increased.

In the following manner, using pPKOZins as the template, an RCR reaction was conducted in the presence of 0.3 µM of the 60 mer modification oligo DNA. Specifically, the RCR reaction solution ver. 1 (added to bring the total volume of the mixture up to 5 µL), 0.3 µM of the 60 mer modification oligo DNA (SUE1357), 0 to 20 nM of Cas9, 100 nM of gRNA_Zins (a guide RNA prepared in accordance with the Alt-R (a registered trademark) CRISPR-Cas9 system from Integrated DNA Technologies, Inc., recognition sequence: ACCAUGAUUACGGAUUCACU (SEQ ID NO: 20)), and 7.5 fg/µL of pPKOZins were mixed, and the resulting mixture was incubated at 30°C for 21 hours. The concentration of each of the components in the reaction solution represents a concentration relative to the total volume of the reaction solution. The resultant was diluted 5-fold with R8 buffer and an additional incubation was conducted at 30°C for 30 minutes. Subsequently, 2.5 µL of the RCR product was supplied to 0.5% agarose gel electrophoresis and detected by SYBR Green I staining.

Here, gRNA_Zins is a guide RNA that specifically recognizes the lacZins sequence prior to modification.

Further, by transforming the RCR product into E. coli and conducting a blue-white determination of the colonies, the proportion of the lacZ mutation modified to the wild-type form within the RCR product was investigated. Specifically, 0.25 µL of the RCR product was used to transform an E. coli DH5α strain using a chemical method. Following transformation, the E. coli was seeded onto an LB plate containing 25 µg/mL of kanamycin, 0.1 mM of IPTG and 40 µg/mL of X-gal relative to the total volume of the LB plate, and the resulting mixture was incubated overnight at 37°C. The number of blue colonies relative to the total number of colonies was calculated.

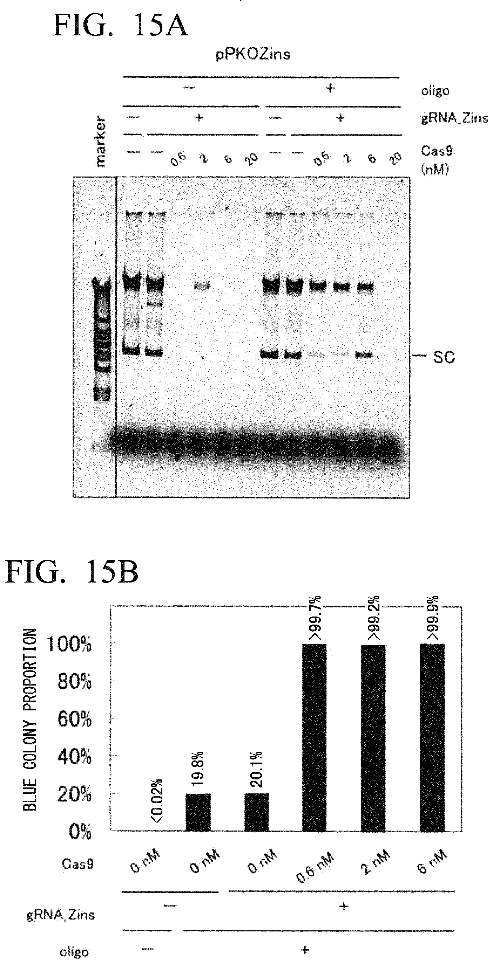

The results are illustrated in FIG. 15A and FIG. 15B. In the absence of the modification oligo DNA, addition of both Cas9 and gRNA_Zins dramatically suppressed the amplification of the supercoiled DNA by RCR (FIG. 15A). In contrast, in the presence of the modification oligo DNA, even when CRISPR-Cas9 was added, an amplified supercoiled DNA product was detected (FIG. 15A). Further, the results of blue-white determination of the transformant colonies confirmed that by addition of Cas9 and gRNA_Zins, in the presence of the modification oligo DNA, almost all of the amplified product was modified to the lacZ wild-type form (FIG. 15B).

### [Example 7] Long-Chain DNA ROGE

Using the same test system as Example 5, an investigation was conducted as to whether the base substitution introduction method using oligo DNA could be applied to RCR of long-chain DNA.

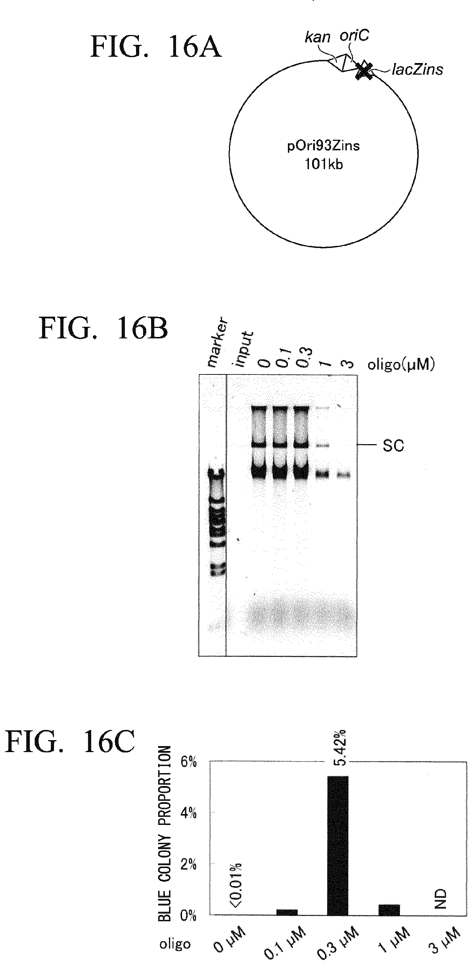

In the following manner, an RCR reaction was conducted using a 101 kb oriC-containing circular DNA having a lacZins mutation (pOri93Zins) (FIG. 16A) as the template. Specifically, the RCR reaction solution ver. 1 (added to bring the total volume of the mixture up to 5 µL), 0 to 3 µM of the 60 mer modification oligo DNA (SUE1357), 30 ng/µL of lambda DNA, and 0.67 pM of pOri93Zins were mixed, the resulting mixture was incubated at 33°C for 18 hours, and then the resultant was diluted 5-fold with R8 buffer and an additional incubation was conducted at 30°C for 30 minutes. The concentration of each of the components in the reaction solution represents a concentration relative to the total volume of the reaction solution. Subsequently, 2.5 µL of the RCR product was supplied to 0.5% agarose gel electrophoresis and detected by SYBR Green I staining.

Here, pOri93Zins was prepared using the following method. Namely, the 93 kb genome fragment from the XbaI digestion product of the genome DNA from an E. coli strain (DGF-298WΔ100::revΔ234::SC) was joined and circularized with a ligation fragment containing oriC, a kanamycin resistance gene and a lacZins mutant gene (KOZins fragment) using the RA method. The KOZins fragment (8.6 kb) was prepared by PCR using pKOZins as a template and using the primers SUE1745 and SUE1746. The sequences at the two ends of the KOZins fragment are homologous with the two respective ends of the 93 kb genome fragment. After the RA reaction, the circular DNA was amplified by RCR and transformed into E. coli, and the purified supercoiled plasmid from the obtained E. coli colony was used as the pOri93Zins.

**[Table 5]**

| Primer Sequences: Homologous End Sequences shown as Uppercase | | | |
|---|---|---|---|
| Name | Sequence | SEQ ID NO: | Remarks |
| SUE1745 | GTTGAGAAAGGTGATTATCACCATGCGAAT TAACGAAGTTTTTACGGAGGGAAACAATCT caaccaattaaccaattctgattag | 21 | pOri93Zins production |
| SUE 1746 | GCCGGATAAGATGCGTCAGCATCGCATCCG GCAGGGTTTATTATTGTTTTTCGCTCTAG acatgtcaatacttgccccttg | 22 | pOri93ins production |

Further, by transforming the RCR product into E. coli and conducting a blue-white determination of the colonies, the proportion of the lacZ mutation that had been modified to the wild-type form within the RCR product was investigated. Specifically, 0.2 µL of the RCR product was used to transform an E. coli HST08 strain using an electroporation method. Following transformation, the E. coli was seeded onto an LB plate containing 25 µg/mL of kanamycin, 0.1 mM of IPTG and 40 µg/mL of X-gal relative to the total volume of the LB plate, and the resulting mixture was incubated overnight at 37°C. The number of blue colonies relative to the total number of colonies was calculated.

The results are illustrated in FIG. 16B and FIG. 16C. When the concentration of the modification oligo DNA was 1 µM or higher, inhibition of the amplification reaction was detected (FIG. 16B). In FIG. 16B, "Input" represents the case that was flushed with 0.02 ng of the template DNA.

Further, the results of blue-white determination of the transformant colonies indicated that in the presence of 0.3 µM of the modification oligo DNA, the lacZins mutation had been modified to the lacZ wild-type in 5.4% of the amplified product (FIG. 16C).

### [Example 8] Increasing Efficiency of Long-Chain DNA ROGE using CRISPR-Cas9

Using the same test system as Example 6, an investigation was conducted as to whether the increase in modification efficiency provided by CRISPR-Cas9 could be applied to RCR of long-chain DNA.

Using pOri93Zins circular DNA that had undergone treatment to remove unnecessary linear DNA as the template, an RCR reaction was conducted in the presence of 0.3 µM of the 60 mer modification oligo DNA, in the manner described below. Specifically, the RCR reaction solution ver. 1 (added to bring the total volume of the mixture up to 5 µL), 0.3 µM of the 60 mer modification oligo DNA (SUE1357), 3 ng/µL of lambda DNA, 10 nM of Cas9, 100 nM of gRNA_Zins (a guide RNA prepared in accordance with the Alt-R (a registered trademark) CRISPR-Cas9 system from Integrated DNA Technologies, Inc., recognition sequence: ACCAUGAUUACGGAUUCACU (SEQ ID NO: 20)), and 1 pM of the pOri93Zins were mixed. The concentration of each of the components in the reaction solution represents a concentration relative to the total volume of the reaction solution. Subsequently, a 40-cycle incubation was conducted using a temperature cycle that involved incubation at 37°C for one minute and then incubation at 24°C for 30 minutes, and then the resultant was diluted 5-fold with R8 buffer and an additional incubation was conducted at 30°C for 30 minutes. Subsequently, 2.5 µL of the RCR product was supplied to 0.5% agarose gel electrophoresis and detected by SYBR Green I staining.

Further, by transforming the RCR product into E. coli and conducting a blue-white determination of the colonies, the proportion of the lacZ mutation that had been modified to the wild-type form within the RCR product was investigated. Specifically, 0.2 µL of the RCR product was used to transform an E. coli HST08 strain using an electroporation method. Following transformation, the E. coli was seeded onto an LB plate containing 25 µg/mL of kanamycin, 0.1 mM of IPTG and 40 µg/mL of X-gal relative to the total volume of the LB plate, and the resulting mixture was incubated overnight at 37°C. The number of blue colonies relative to the total number of colonies was calculated.

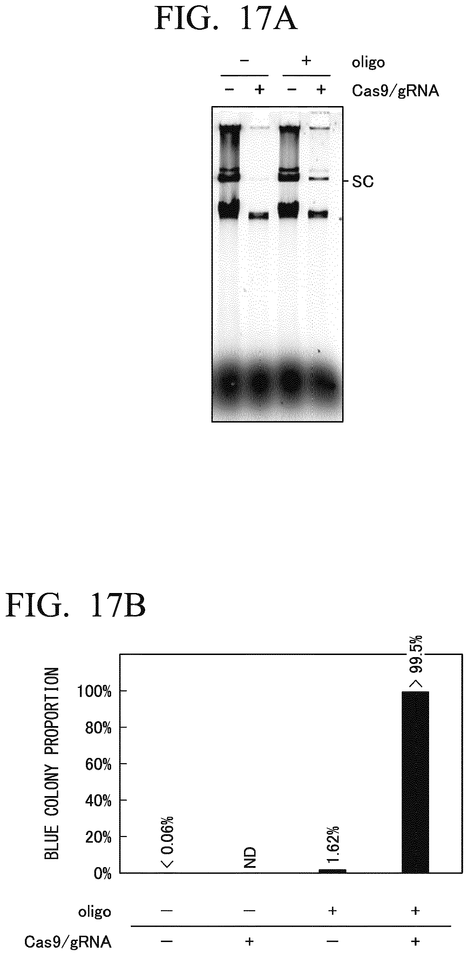

The results are illustrated in FIG. 17A and FIG. 17B. In the absence of the modification oligo DNA, addition of both Cas9 and gRNA_Zins dramatically suppressed the amplification of the supercoiled DNA by RCR (FIG. 17A). In contrast, in the presence of the modification oligo DNA, even when CRISPR-Cas9 was added, an amplified supercoiled DNA product was detected (FIG. 17A).

Further, the results of blue-white determination of the transformant colonies confirmed that by addition of Cas9 and gRNA_Zins, in the presence of the modification oligo DNA, almost all of the amplified product was modified to the lacZ wild-type form (FIG. 17B).

### [Example 9] Substitution and Insertion of a Plurality of Bases

Using the same test system as Example 5, an investigation was conducted as to whether a plurality of bases could be substituted or inserted using modification oligo DNA.

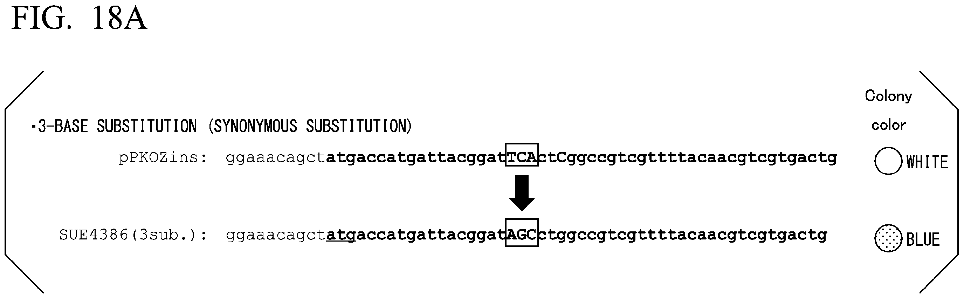

In the case of substitution of a plurality of bases, pPKOZins was used as the template, and a modification oligo DNA with a synonymous codon due to a 3-base substitution was used. pPKOZins has a frameshift mutation due to the insertion of one base in the lacZ coding region, and the E. coli colonies appear white. The modification oligo DNA includes, near the site of the 3-base substitution, a sequence that returns this one-base insertion to the wild-type form. It is expected that the plasmid converted to wild-type (blue E. coli colony) by the modification will have also undergone a simultaneous introduction of the 3-base substitution (FIG. 18A).

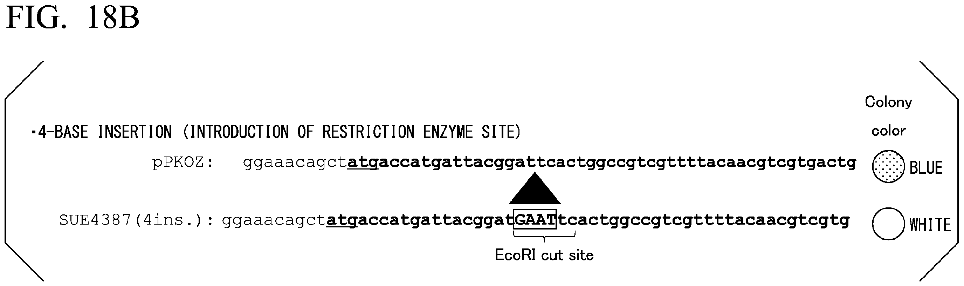

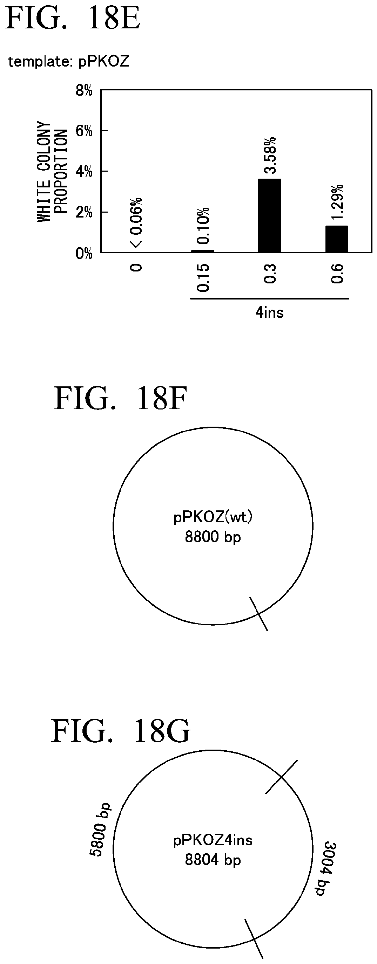

On the other hand, in the case of insertion of a plurality of bases, pPKOZ was used as the template, and a modification oligo that develops an EcoRI recognition sequence due to a 4-base insertion was used (FIG. 18B). The 4-base insertion introduces a frameshift mutation, and because the lacZ gene is not synthesized, it is expected that the E. coli colonies will appear white. Because pPKOZ already has an EcoRI site at one location (FIG. 18F), the 4-base insertion yields a plasmid that has EcoRI sites at two locations (FIG. 18G).

Using pPKOZins or pPKOZ as a template, RCR reactions were conducted in the presence of different concentrations of the modification oligo DNA in the manner described below. Specifically, the RCR reaction solution ver. 1 (added to bring the total volume of the mixture up to 5 µL), 0 µM to 0.6 µM of the 60 mer modification oligo DNA (SUE4386 or SUE4387), and 50 pg/µL of pPKOZins or pPKOZ were mixed. The concentration of each of the components in the reaction solution represents a concentration relative to the total volume of the reaction solution. Subsequently, the reaction solution was incubated at 33°C for 18 hours, and the resultant was then diluted 5-fold with R8 buffer, and an additional incubation was conducted at 30°C for 30 minutes. Subsequently, 2.5 µL of the RCR product was supplied to 0.5% agarose gel electrophoresis and detected by SYBR Green I staining.

**[Table 6]**

| Primer Sequences: Homologous End Sequences shown as Uppercase | | | |
|---|---|---|---|
| Name | Sequence | SEQ ID NO: | Remarks |
| SUE4386 | GGAAACAGCTATGACCATGATTACGGATAG CCTGGCCGTCGTTTTACAACGTCGTGACTG | 56 | Modification oligo DNA (3-base substitution) |
| SUE4387 | GGAAACAGCTATGACCATGATTACGGAT gaatTCACTGGCCGTCGTTTTACAACGTCGTG | 57 | Modification oligo DNA (4-base insertion) |

Further, by transforming the RCR products into E. coli and conducting a blue-white determination of the colonies, the proportion of blue colonies in the case where pPKOZins was used, and the proportion of white colonies in the case where pPKOZ was used were investigated. Specifically, 0.2 µL of each RCR product was used to transform an E. coli DH5α strain using a chemical method. Following transformation, the E. coli was seeded onto an LB plate containing 25 µg/mL of kanamycin, 0.1 mM of IPTG and 40 µg/mL of X-gal relative to the total volume of the LB plate, and the resulting mixture was incubated overnight at 37°C. The number of blue colonies or white colonies relative to the total number of colonies was calculated.

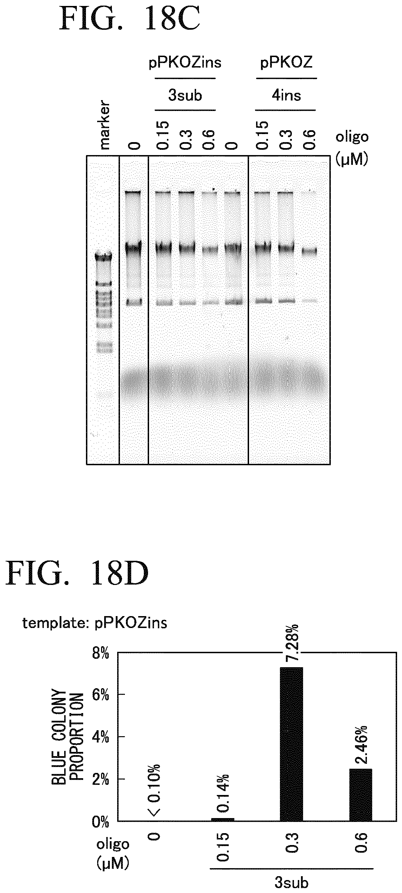

The results are illustrated in FIG. 18C, FIG. 18D and FIG. 18E. The results of the blue-white determination of the E. coli transformant colonies confirmed that, for both pPKOZins and pPKOZ, lacZ was modified with the highest efficiency (7.3% and 3.6% respectively) in the sample to which 0.3 µM of the modification oligo DNA had been added (FIG. 18D and FIG. 18E).

In order to ascertain whether modification to the target sequence had actually been achieved, four of the plasmids obtained from the blue colonies in the modification tests using pPKOZins as the template were subjected to sequence analysis, and in all four cases, the target 3-base substitution had been inserted.

Further, seven of the plasmids obtained from the white colonies in the 4-base insertion tests using pPKOZ as the template were subjected to a restriction enzyme treatment in the following manner to confirm the 4-base insertion. Specifically, 10×H buffer (added to bring the total volume of the mixture up to 20 µL), 0.75 U/µL of EcoRI, and 2 µL of each plasmid were mixed, and each resulting mixture was incubated at 37°C for one hour. The same restriction enzyme treatment was conducted for the plasmids obtained from the blue colonies which were assumed to be unmodified. Following the treatment, 4 µL of the restriction enzyme treatment product was supplied to 0.5% agarose gel electrophoresis and detected by SYBR Green I staining.

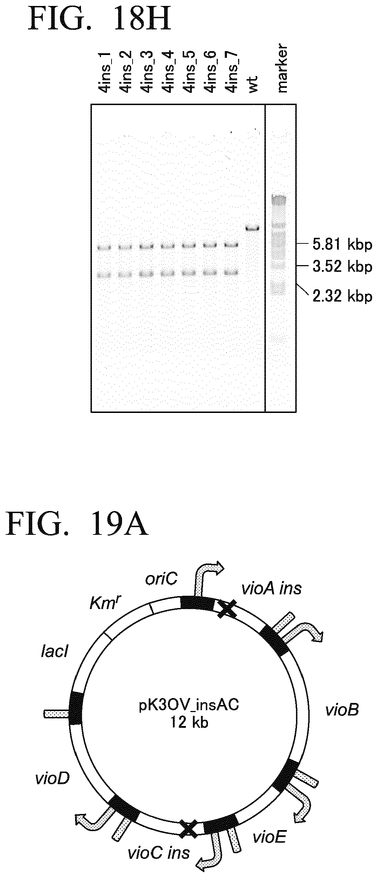

The results are illustrated in FIG. 18H. In all of the seven modified plasmids that were subjected to the restriction enzyme treatment, two fragments were detected from the expected cleavages at two locations, confirming that modification with the target 4-base insertion sequence had been achieved. In the unmodified plasmids (wt), cleavage was only detected at one location (FIG. 18H).

### [Example 10] Simultaneous Base Substitution at a Plurality of Locations

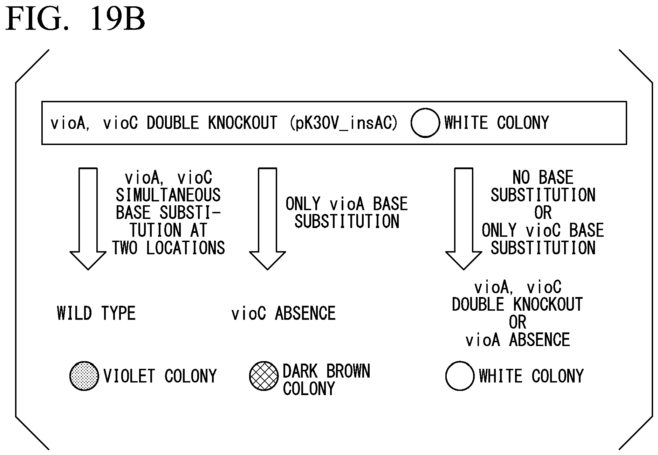

Testing was conducted in the following manner using the violacein biosynthetic pathway. Violacein is a secondary metabolite produced based on tryptophan using five gene products (vioA, vioB, vioC, vioD and vioE). In E. coli, in those cases where the five genes all exhibit functionality, the colony exhibits a violet color (FIG. 19B). In the absence of vioC, production proceeds as far as a violacein biosynthetic intermediate product, and the colony exhibits a dark brown color (FIG. 19B). Further, in the absence of vioA or in the case of double knockout of vioA and vioC, no colored product is synthesized, and the colony remains white (FIG. 19B). By using this variation in the colony color, a test was conducted as to whether a vioA, vioC double knockout mutant plasmid could be returned to a vioA, vioC wild-type plasmid by simultaneous base substitution at two separated regions for vioA and vioC, using the appearance of a violet colony as a detection indicator.

The plasmid pK30V_insAC that was used had one base inserted at the coding region of each of vioA and vioC (FIG. 19A). This pK30V_insAC was prepared using pK30V as a template, by conducting two consecutive 1-base insertions in combination with the RCR reaction. The first insertion was conducted using SUE4384 (SEQ ID NO: 64) as the oligo DNA to achieve a 1-base insertion into vioC, with a plasmid pK30V_insC prepared from the thus produced dark brown colony, and the second insertion was then conducted using the pK30V_insC as a template, using SUE4579 (SEQ ID NO: 63) to introduce a 1-base insertion into vioA, with the plasmid pK30V_insAC prepared from the thus produced white colony. Because the vioA and vioC do not express due to the frameshift mutations, E. coli colonies that have been transformed with pK30V_insAC are white, and exhibit no color.

The pK30V was prepared by subjecting the DNA region of pETm6-vioABECD (Jones et al., Sci. Rep. (2015) 5, 11301) that encodes the vioABECD gene to ligation and circularization by the RA method with a 3.5 kb DNA fragment containing an oriC represented by SEQ ID NO: 67, a kanamycin resistance gene and LacI.

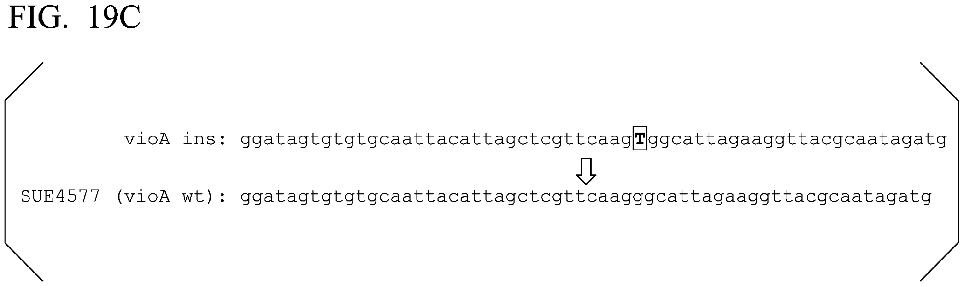

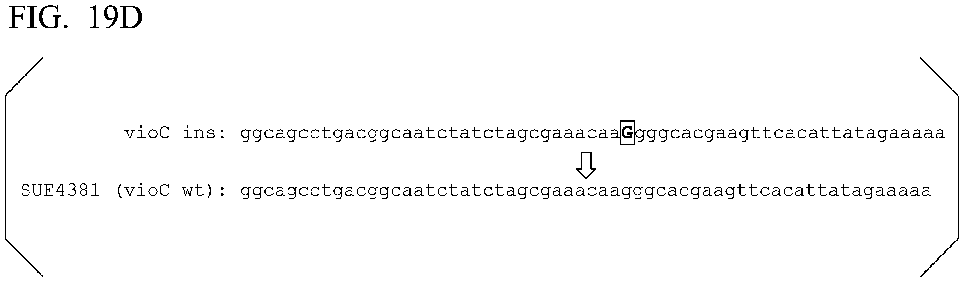

SUE4577 (SEQ ID NO: 65) and SUE4381 (SEQ ID NO: 66) were used as the oligo DNAs for returning the mutant vioA and vioC to wild-type forms (FIG. 19C and FIG. 19D).

Using the pK30V_insAC having frameshift mutations in vioA and vioC as a template, an RCR reaction was conducted in the following manner. Specifically, the RCR reaction solution ver. 1 (added to bring the total volume of the mixture up to 5 µL), 0.15 µM of the 60 mer modification oligo DNAs (SUE4577 and SUE4381), and 50 pg/µL of pK30V_insAC were mixed, and the resulting mixture was incubated at 33°C for 6 hours, 12 hours or 18 hours. The concentration of each of the components in the reaction solution represents a concentration relative to the total volume of the reaction solution.

**[Table 7]**

| Primer Sequences: Homologous End Sequences shown as Uppercase | | | |
|---|---|---|---|
| Name | Sequence | SEQ ID NO: | Remarks |
| SUE4579 | GTGTGTGCAATTACATTAGCTCGTTCAAG tGGCATTAGAAGGTTACGCAATAGATGTCTA | 63 | oligo DNA for production of pK30V_insAC |
| SUE4384 | GCCTGACGGCAATCTATCTAGCGAAACAA gGGGCACGAAGTTCACATTATAGAAAAACGT | 64 | oligo DNA for production of pK30V_insAC |
| SUE4577 | GGATAGTGTGTGCAATTACATTAGCTCGT TCAAGGGCATTAGAAGGTTACGCAATAGATG | 65 | Modification oligo DNA |
| SUE4381 | GGCAGCCTGACGGCAATCTATCTAGCGAA ACAAGGGCACGAAGTTCACATTATAGAAAAA | 66 | Modification oligo DNA |

Subsequently, by transforming the RCR products into E. coli and conducting a color determination of the colonies, the proportion of colonies in which the vioA and vioC mutations had been modified to the wild-type forms in the RCR product was investigated. Specifically, 1 µL of the RCR product was used directly to transform an E. coli BL21 Star (DE3) strain (Thermo Fisher Scientific Inc.) using a chemical method. Following transformation, the E. coli was seeded onto an LB plate containing 25 µg/mL of kanamycin and 10 µM of IPTG relative to the total volume of the LB plate, and the resulting mixture was incubated overnight at 30°C. The number of violet colonies relative to the total number of colonies was calculated.

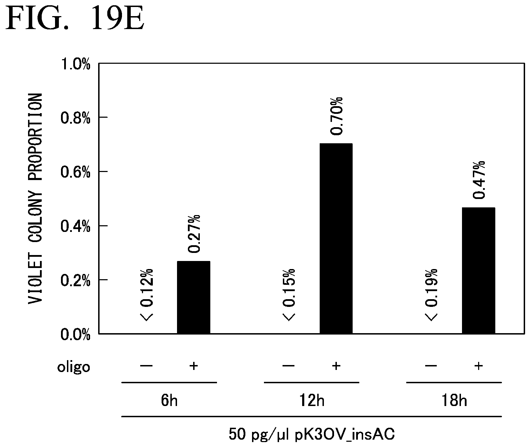

The results are shown in FIG. 19E. The results of the E. coli transformation by the RCR product at each time revealed the detection of violet colonies at an efficiency of about 0.3 to 0.7%. These results confirmed that the two locations of the vioA mutation and the vioC mutation had been simultaneously modified to the wild-type forms (FIG. 19E).

### INDUSTRIAL APPLICABILITY

By using the method of an embodiment of the present invention, a method for amplifying DNA edited products, and particularly long-chain DNA edited products, without using cells can be provided.

### SEQUENCE LISTINGS FREE TEXT

SEQ ID NO: 1 gRNA_Km
SEQ ID NO:2 gRNA_007
SEQ ID NO: 3 SUE1510
SEQ ID NO: 4 SUE1511
SEQ ID NO: 5 SUE1638
SEQ ID NO: 6 SUE1639
SEQ ID NO:7 SUE1753
SEQ ID NO: 8 SUE1756
SEQ ID NO: 9 SUE1822
SEQ ID NO: 10 SUE1823
SEQ ID NO: 11 Cm-oriC fragment
SEQ ID NO: 12 SUE818
SEQ ID NO: 13 SUE819
SEQ ID NO: 14 SUE1415
SEQ ID NO: 15 SUE1416
SEQ ID NO: 16 SUE1354
SEQ ID NO: 17 SUE1355
SEQ ID NO: 18 SUE1356
SEQ ID NO: 19 SUE1357
SEQ ID NO: 20 gRNA_Zins
SEQ ID NO: 21 SUE1745
SEQ ID NO: 22 SUE1746
SEQ ID NO: 23 ter sequence (consensus)
SEQ ID NO: 24 ter sequence (consensus)
SEQ ID NO: 25 ter sequence (terA, B, D, E or H)
SEQ ID NO: 26 ter sequence (terA, B, D, E or H)
SEQ ID NO: 27 ter sequence (terC)
SEQ ID NO: 28 ter sequence (terF)
SEQ ID NO: 29 ter sequence (terG)
SEQ ID NO: 30 ter sequence (terI)
SEQ ID NO: 31 ter sequence (terJ)
SEQ ID NO: 32 ter sequence (Bacillus consensus)
SEQ ID NO: 33 ter sequence (Bacillus subtilis consensus)
SEQ ID NO: 34 ter sequence (Bacillus subtilis consensus)
SEQ ID NO: 35 ter sequence (terVII)
SEQ ID NO: 36 ter sequence (terIX)
SEQ ID NO: 37 sequence recognized by XerCD (consensus)
SEQ ID NO: 38 sequence recognized by XerCD (consensus, dif and cer)
SEQ ID NO: 39 sequence recognized by XerCD (consensus, dif and psi)
SEQ ID NO: 40 sequence recognized by XerCD (consensus, cer and psi)
SEQ ID NO: 41 dif sequence
SEQ ID NO: 42 cer sequence
SEQ ID NO: 43 psi sequence
SEQ ID NO: 44 dif sequence
SEQ ID NO: 45 cer sequence
SEQ ID NO: 46 psi sequence
SEQ ID NO: 47 loxP consensus sequence
SEQ ID NO: 48 lox511 sequence
SEQ ID NO: 49 lox2272 sequence
SEQ ID NO: 50 loxFAS sequence
SEQ ID NO: 51 lox RE sequence
SEQ ID NO: 52 lox LE sequence
SEQ ID NO: 53 FRT sequence
SEQ ID NO: 54 rox sequence
SEQ ID NO: 55 Outside End (OE) sequence
SEQ ID NO: 56 SUE4386
SEQ ID NO: 57 SUE4387
SEQ ID NO: 58 partial sequence downstream of start codon of lacZ wt
SEQ ID NO: 59 partial sequence downstream of start codon of lacZ ins
SEQ ID NO: 60 partial sequence downstream of start codon of lacZ mis
SEQ ID NO: 61 partial sequence in vicinity of start codon of pPKOZins
SEQ ID NO: 62 partial sequence in vicinity of start codon of pPKOZ
SEQ ID NO: 63 SUE4579
SEQ ID NO: 64 SUE4384
SEQ ID NO: 65 SUE4577
SEQ ID NO: 66 SUE4381
SEQ ID NO: 67 DNA fragment (3.5 kb) for production of pK30V
SEQ ID NO: 68 vioA ins
SEQ ID NO: 69 vioC ins

## Claims

1. A method for editing DNA in a cell-free system, the method comprising the following steps:
(1) a step of introducing a deletion, substitution or addition at a target site of a DNA in a cell-free system, and
(2) a step of amplifying, in a cell-free system, the DNA into which a deletion, substitution or addition has been introduced in step (1), wherein the DNA is amplified under temperature conditions that incubate at a temperature within a range from 20°C to 80°C.

2. The method for editing DNA in a cell-free system according to Claim 1, the method comprising the following steps:
(1) a step of introducing a deletion, substitution or addition at a target site of a DNA in a cell-free system, and
(2) a step of amplifying, in a cell-free system, the DNA into which a deletion, substitution or addition has been introduced in step (1), wherein the DNA is amplified under temperature conditions that either incubate at a constant temperature, or incubate under a repeating temperature cycle in which incubation is conducted at two temperatures of 65°C or lower.

3. The method for editing DNA in a cell-free system according to Claim 1 or 2, the method comprising the following steps:
(1) a step of introducing a deletion, substitution or addition at a target site of a DNA in a cell-free system, and
(2) a step of amplifying, in a cell-free system, the DNA into which a deletion, substitution or addition has been introduced in step (1), wherein the DNA is amplified under temperature conditions that either incubate at a fixed temperature within a range from 20°C to 80°C, or incubate under a repeating temperature cycle in which incubation is conducted at two temperatures of 65°C or lower.

4. The method according to any one of Claims 1 to 3, wherein step (2) is conducted in presence of an artificial DNA cleavage enzyme that specifically cleaves DNA into which a deletion, substitution or addition has not been introduced.

5. The method according to Claim 4, wherein the artificial DNA cleavage enzyme is an artificial nuclease or an RNA-guided nuclease.

6. The method according to Claim 4, wherein the artificial DNA cleavage enzyme is CRISPR-Cas9.

7. The method according to any one of Claims 1 to 6, wherein the DNA is a circular DNA.

8. The method according to Claim 7, wherein step (2) comprises the following steps:
(2-1) a step of preparing a reaction mixture of: a reaction solution comprising (a) a first enzyme group that catalyzes replication of the circular DNA, (b) a second enzyme group that catalyzes an Okazaki fragment ligation reaction and synthesizes two sister circular DNAs that form a catenane, and (c) a third enzyme group that catalyzes a separation reaction of the two sister circular DNAs; and the circular DNA into which a deletion, substitution or addition has been introduced in step (1); and
(2-2) a step of incubating the reaction mixture prepared in step (2-1), either at a fixed temperature within a range from 20°C to 80°C, or under a repeating temperature cycle in which incubation is conducted at two temperatures of 65°C or lower.

9. The method according to Claim 8, wherein the circular DNA contains a replication origin sequence that can bind to an enzyme having DnaA activity.

10. The method according to Claim 7, wherein in step (2), the circular DNA is amplified by rolling circle amplification.

11. The method according to any one of Claims 1 to 10, wherein step (1) comprises the following steps:
(1-1) a step of cleaving the DNA at a target site to prepare at least one linear DNA by causing an artificial DNA cleavage enzyme to act upon the DNA;
(1-2) a step of preparing a reaction solution containing the linear DNA prepared in step (1-1), one or more types of DNA fragment, and a protein having RecA family recombinase activity; and
(1-3) a step of mutually joining the linear DNA and the one or more types of DNA fragment at regions in which base sequences are homologous or at regions in which base sequences are complementary, thereby forming a DNA in which the one or more types of DNA fragment have each been inserted at a target site of a template DNA.

12. The method according to any one of Claims 1 to 10, wherein step (1) comprises the following step:
a step of conducting a DNA replication reaction in presence of a single-stranded DNA used for introducing a deletion, substitution or addition, wherein the single-stranded DNA can hybridize with a target site of the DNA under the replication reaction conditions.

13. The method according to any one of Claims 1 to 12, wherein in step (2), the DNA is amplified under temperature conditions that incubate under a temperature cycle that repeats incubation at 30°C or higher and incubation at 27°C or lower.

14. The method according to any one of Claims 1 to 13, wherein a size of the DNA into which a deletion, substitution or addition has been introduced is 50 kb or larger.


### SEQUENCE LISTINGS

SEQUENCE LISTING

<110>  OriCiro Genomics, Inc.

<120>  METHOD OF EDITING DNA IN CELl-FREE SYSTEM

<130>  PC28170

<150>  JP2018-142274
<151>  2018-07-30

<160>  69

<170>  PatentIn version 3.5

<210>  1
<211>  20
<212>  RNA
<213>  Artificial Sequence

<220>
<223>  synthetic nucleotide for gRNA_Km

<400>  1
ugguuaauug guuguaacac                                                   20


<210>  2
<211>  20
<212>  RNA
<213>  Artificial Sequence

<220>
<223>  synthetic nucleotide for gRNA_007

<400>  2
ccuuuaguua caacauacuc                                                   20


<210>  3
<211>  77
<212>  DNA
<213>  Artificial Sequence

<220>
<223>  synthetic nucleotide for SUE1510

<400>  3
cgatgagttt ttctaatcag aattggttaa ttggttgtaa ctttagttac aacatactac       60

gacaggtttc ccgactg                                                      77


<210>  4
<211>  78
<212>  DNA
<213>  Artificial Sequence

<220>
<223>  synthetic nucleotide for SUE1511

<400>  4
agccgccgtc ccgtcaagtc agcgtaatgc tctgccagtg ctttagttac aacatacttt       60

tccttacgcg aaatacgg                                                    78

<210>  5
<211>  54
<212>  DNA
<213>  Artificial Sequence

<220>
<223>  synthetic nucleotide for SUE1638

<400>  5
ttgcagtttc atttgatgct cgatgagttt ttctaatcag aattggttaa ttgg            54

<210>  6
<211>  39
<212>  DNA
<213>  Artificial Sequence

<220>
<223>  synthetic nucleotide for SUE1639

<400>  6
aagttcgatt tattcaacaa agccgccgtc ccgtcaagt                             39

<210>  7
<211>  84
<212>  DNA
<213>  Artificial Sequence

<220>
<223>  synthetic nucleotide for SUE1753

<400>  7
ttgcagtttc atttgatgct cgatgagttt ttctaatcag aattggttaa ttggttgtaa      60

atatgagtaa acttggtctg acag                                             84

<210>  8
<211>  80
<212>  DNA
<213>  Artificial Sequence

<220>
<223>  synthetic nucleotide for SUE1756

<400>  8
aagttcgatt tattcaacaa agccgccgtc ccgtcaagtc agcgtaatgc tctgccagtg      60

tttccttacg cgaaatacgg                                                  80

<210>  9
<211>  80
<212>  DNA
<213>  Artificial Sequence

<220>
<223>  synthetic nucleotide for SUE1822

<400>  9
cgcatccggc agggtttatt attgtttttt cgctctaggt cggcttgaga aagacctgag      60

cacttttcgg ggaaatgtgc                                                  80


<210>  10
<211>  79
<212>  DNA
<213>  Artificial Sequence

<220>
<223>  synthetic nucleotide for SUE1823

<400>  10
tttaataccc aggatccatt taacataata tacattatgc gcacctttag ttacaacata      60

acgacaggtt tcccgactg                                                   79


<210>  11
<211>  1298
<212>  DNA
<213>  Artificial Sequence

<220>
<223>  synthetic nucleotide for Cm-oriC fragment

<400>  11
gttgagaaag gtgattatca ccatgcgaat taacgaagtt tttacggagg gaaacaatct      60

cgtaagaggt tccaactttc accataatga aataagatca ctaccgggcg tattttttga      120

gttatcgaga ttttcaggag ctaaggaagc taaaatggag aaaaaaatca ctggatatac     180

caccgttgat atatcccaat ggcatcgtaa agaacatttt gaggcatttc agtcagttgc     240

tcaatgtacc tataaccaga ccgttcagct ggatattacg gcctttttaa agaccgtaaa     300

gaaaaataag cacaagtttt atccggcctt tattcacatt cttgcccgcc tgatgaatgc     360

tcatccggaa ttccgtatgg caatgaaaga cggtgagctg gtgatatggg atagtgttca     420

cccttgttac accgttttcc atgagcaaac tgaaacgttt tcatcgctct ggagtgaata     480

ccacgacgat ttccggcagt ttctacacat atattcgcaa gatgtggcgt gttacggtga     540

aaacctggcc tatttcccta aagggtttat tgagaatatg tttttcgtct cagccaatcc     600

ctgggtgagt ttcaccagtt ttgatttaaa cgtggccaat atggacaact tcttcgcccc     660

cgttttcacc atgggcaaat attatacgca aggcgacaag gtgctgatgc cgctggcgat     720

tcaggttcat catgccgtct gtgatggctt ccatgtcggc agaatgctta atgaattaca     780

acagtactgc gatgagtggc agggcggggc gtaagaagat ccggcagaag aatggagtat     840

gttgtaacta aagataactt cgtataatgt atgctatacg aagttataca gatcgtgcga     900

tctactgtgg ataactctgt caggaagctt ggatcaaccg gtagttatcc aaagaacaac     960

tgttgttcag tttttgagtt gtgtataacc cctcattctg atcccagctt atacggtcca    1020

ggatcaccga tcattcacag ttaatgatcc tttccaggtt gttgatctta aaagccggat 1080

ccttgttatc cacagggcag tgcgatccta ataagagatc acaatagaac agatctctaa 1140

ataaatagat cttcttttta atacccagga tccatttaac ataatataca ttatgcgcac 1200

ctttagttac aacatactca ggtctttctc aagccgacct agagcgaaaa aacaataata 1260

aaccctgccg gatgcgatgc tgacgcatct tatccggc 1298

<210> 12
<211> 30
<212> DNA
<213> Artificial Sequence

<220>
<223> synthetic nucleotide for SUE818

<400> 12
gattacggat tcactcggcc gtcgttttac 30

<210> 13
<211> 30
<212> DNA
<213> Artificial Sequence

<220>
<223> synthetic nucleotide for SUE819

<400> 13
gtaaaacgac ggccgagtga atccgtaatc 30

<210> 14
<211> 30
<212> DNA
<213> Artificial Sequence

<220>
<223> synthetic nucleotide for SUE1415

<400> 14
ccatgattac ggattaactg gccgtcgttt 30

<210> 15
<211> 30
<212> DNA
<213> Artificial Sequence

<220>
<223> synthetic nucleotide for SUE1416

<400> 15
aaacgacggc cagttaatcc gtaatcatgg 30

<210> 16
<211> 20
<212> DNA

<213>  Artificial Sequence

<220>
<223>  synthetic nucleotide for SUE1354

<400>  16
ttacggattc actggccgtc                                                    20


<210>  17
<211>  30
<212>  DNA
<213>  Artificial Sequence

<220>
<223>  synthetic nucleotide for SUE1355

<400>  17
catgattacg gattcactgg ccgtcgtttt                                         30


<210>  18
<211>  40
<212>  DNA
<213>  Artificial Sequence

<220>
<223>  synthetic nucleotide for SUE1356

<400>  18
atgaccatga ttacggattc actggccgtc gttttacaac                              40


<210>  19
<211>  60
<212>  DNA
<213>  Artificial Sequence

<220>
<223>  synthetic nucleotide for SUE1357

<400>  19
ggaaacagct atgaccatga ttacggattc actggccgtc gttttacaac gtcgtgactg        60


<210>  20
<211>  20
<212>  RNA
<213>  Artificial Sequence

<220>
<223>  synthetic nucleotide for gRNA_Zins

<400>  20
accaugauua cggauucacu                                                    20


<210>  21
<211>  85
<212>  DNA
<213>  Artificial Sequence

<220>

<223>  synthetic nucleotide for SUE1745

<400>  21
gttgagaaag gtgattatca ccatgcgaat taacgaagtt tttacggagg gaaacaatct      60

caaccaatta accaattctg attag                                            85


<210>  22
<211>  81
<212>  DNA
<213>  Artificial Sequence

<220>
<223>  synthetic nucleotide for SUE1746

<400>  22
gccggataag atgcgtcagc atcgcatccg gcagggttta ttattgtttt ttcgctctag      60

acatgtcaat acttgccctt g                                                81


<210>  23
<211>  14
<212>  DNA
<213>  Artificial Sequence

<220>
<223>  synthetic nucleotide for ter sequence


<220>
<221>  misc_feature
<222>  (2)..(2)
<223>  n is a, c, g, or t

<220>
<221>  misc_feature
<222>  (3)..(3)
<223>  r is a or g

<220>
<221>  misc_feature
<222>  (4)..(4)
<223>  w is a or t

<220>
<221>  misc_feature
<222>  (13)..(13)
<223>  k is g or t

<400>  23
gnrwgttgta acka                                                        14


<210>  24
<211>  14
<212>  DNA
<213>  Artificial Sequence

<220>
<223>  synthetic nucleotide for ter sequence

<220>
<221>  misc_feature
<222>  (2)..(2)
<223>  k is g or t

<220>
<221>  misc_feature
<222>  (4)..(4)
<223>  w is a or t

<220>
<221>  misc_feature
<222>  (13)..(13)
<223>  k is g or t

<400>  24
gkawgttgta acka                                                         14


<210>  25
<211>  14
<212>  DNA
<213>  Artificial Sequence

<220>
<223>  synthetic nucleotide for ter sequence (ter A, B, D, E, or H)

<400>  25
gtatgttgta acta                                                         14


<210>  26
<211>  18
<212>  DNA
<213>  Artificial Sequence

<220>
<223>  synthetic nucleotide for ter sequence (ter A, B, D, E, or H)

<400>  26
agtatgttgt aactaaag                                                     18


<210>  27
<211>  14
<212>  DNA
<213>  Artificial Sequence

<220>
<223>  synthetic nucleotide for ter sequence (terC)

<400>  27
ggatgttgta acta                                                         14


<210>  28
<211>  14
<212>  DNA
<213>  Artificial Sequence

<220>
<223>  synthetic nucleotide for ter sequence (terF)

<400>  28
gtatgttgta acga                                                         14


<210>  29
<211>  14
<212>  DNA
<213>  Artificial Sequence

<220>
<223>  synthetic nucleotide for ter sequence (terG)

<400>  29
ggatgttgta acta                                                         14


<210>  30
<211>  14
<212>  DNA
<213>  Artificial Sequence

<220>
<223>  synthetic nucleotide for ter sequence (terI)

<400>  30
ggaagttgta acga                                                         14


<210>  31
<211>  14
<212>  DNA
<213>  Artificial Sequence

<220>
<223>  synthetic nucleotide for ter sequence (terJ)

<400>  31
gtaagttgta acga                                                         14


<210>  32
<211>  23
<212>  DNA
<213>  Artificial Sequence

<220>
<223>  synthetic nucleotide for ter sequence (Bacillus)


<220>
<221>  misc_feature
<222>  (3)..(3)
<223>  w is a or t

<220>
<221>  misc_feature
<222>  (4)..(4)
<223>  r is a or g

<220>
<221>  misc_feature
<222>  (6)..(10)

<223>  n is a, c, g, or t

<220>
<221>  misc_feature
<222>  (11)..(11)
<223>  y is t or c

<220>
<221>  misc_feature
<222>  (12)..(12)
<223>  n is a, c, g, or t

<220>
<221>  misc_feature
<222>  (19)..(19)
<223>  n is a, c, g, or t

<400>  32
acwrannnnn ynatgtacna aat                                              23


<210>  33
<211>  23
<212>  DNA
<213>  Artificial Sequence

<220>
<223>  synthetic nucleotide for ter sequence (Bacillus subtilis)


<220>
<221>  misc_feature
<222>  (8)..(8)
<223>  r is a or g

<220>
<221>  misc_feature
<222>  (10)..(10)
<223>  w is a or t

<220>
<221>  misc_feature
<222>  (12)..(12)
<223>  y is c or t

<400>  33
actaattraw cyatgtacta aat                                              23


<210>  34
<211>  28
<212>  DNA
<213>  Artificial Sequence

<220>
<223>  synthetic nucleotide for ter sequence (Bacillus subtilis)


<220>
<221>  misc_feature
<222>  (8)..(8)
<223>  r is a or g

<220>
<221>  misc_feature
<222>  (10)..(10)
<223>  w is a or t

<220>
<221>  misc_feature
<222>  (12)..(12)
<223>  y is c or t

<400>  34
actaattraw cyatgtacta aattttca                                          28


<210>  35
<211>  30
<212>  DNA
<213>  Artificial Sequence

<220>
<223>  synthetic nucleotide for ter sequence (terVII)

<400>  35
gaactaatta aactatgtac taaattttca                                        30


<210>  36
<211>  30
<212>  DNA
<213>  Artificial Sequence

<220>
<223>  synthetic nucleotide for ter sequence (terIX)

<400>  36
atactaattg atccatgtac taaattttca                                        30


<210>  37
<211>  28
<212>  DNA
<213>  Artificial Sequence

<220>
<223>  synthetic nucleotide recognized by XerCD


<220>
<221>  misc_feature
<222>  (7)..(7)
<223>  y is c or t

<220>
<221>  misc_feature
<222>  (8)..(8)
<223>  r is a or g

<220>
<221>  misc_feature
<222>  (9)..(9)
<223>  y is c or t

<220>

<221>  misc_feature
<222>  (12)..(17)
<223>  n is a, c, g, or t

<220>
<221>  misc_feature
<222>  (23)..(23)
<223>  k is g or t

<220>
<221>  misc_feature
<222>  (28)..(28)
<223>  y is c or t

<400>  37
ggtgcgyrya annnnnntta tgktaaay                                          28


<210>  38
<211>  28
<212>  DNA
<213>  Artificial Sequence

<220>
<223>  synthetic nucleotide recognized by XerCD (dif and cer)


<220>
<221>  misc_feature
<222>  (7)..(7)
<223>  y is c or t

<220>
<221>  misc_feature
<222>  (9)..(9)
<223>  y is c or t

<220>
<221>  misc_feature
<222>  (12)..(17)
<223>  n is a, c, g, or t

<220>
<221>  misc_feature
<222>  (23)..(23)
<223>  k is g or t

<400>  38
ggtgcgyaya annnnnntta tgktaaat                                          28


<210>  39
<211>  28
<212>  DNA
<213>  Artificial Sequence

<220>
<223>  synthetic nucleotide recognized by XerCD (dif and psi)


<220>
<221>  misc_feature
<222>  (8)..(8)

<223>  r is a or g

<220>
<221>  misc_feature
<222>  (9)..(9)
<223>  y is c or t

<220>
<221>  misc_feature
<222>  (12)..(17)
<223>  n is a, c, g, or t

<220>
<221>  misc_feature
<222>  (28)..(28)
<223>  y is c or t

<400>  39
ggtgcgcrya annnnnntta tgttaaay                                          28


<210>  40
<211>  28
<212>  DNA
<213>  Artificial Sequence

<220>
<223>  synthetic nucleotide recognized by XerCD (dif and psi)


<220>
<221>  misc_feature
<222>  (7)..(7)
<223>  y is c or t

<220>
<221>  misc_feature
<222>  (8)..(8)
<223>  r is a or g

<220>
<221>  misc_feature
<222>  (12)..(17)
<223>  n is a, c, g, or t

<220>
<221>  misc_feature
<222>  (23)..(23)
<223>  k is g or t

<220>
<221>  misc_feature
<222>  (28)..(28)
<223>  y is c or t

<400>  40
ggtgcgyrca annnnnntta tgktaaay                                          28


<210>  41
<211>  28
<212>  DNA
<213>  Artificial Sequence

<220>
<223>  synthetic nucleotide for dif sequence


<220>
<221>  misc_feature
<222>  (12)..(17)
<223>  n is a, c, g, or t

<400>  41
ggtgcgcata annnnnntta tgttaaat                                          28


<210>  42
<211>  28
<212>  DNA
<213>  Artificial Sequence

<220>
<223>  synthetic nucleotide for cer sequence


<220>
<221>  misc_feature
<222>  (12)..(17)
<223>  n is a, c, g, or t

<400>  42
ggtgcgtaca annnnnntta tggtaaat                                          28


<210>  43
<211>  28
<212>  DNA
<213>  Artificial Sequence

<220>
<223>  synthetic nucleotide for psi sequence


<220>
<221>  misc_feature
<222>  (12)..(17)
<223>  n is a, c, g, or t

<400>  43
ggtgcgcgca annnnnntta tgttaaac                                          28


<210>  44
<211>  28
<212>  DNA
<213>  Artificial Sequence

<220>
<223>  synthetic nucleotide for dif sequence

<400>  44
ggtgcgcata atgtatatta tgttaaat                                          28


<210>  45

<211>  28
<212>  DNA
<213>  Artificial Sequence

<220>
<223>  synthetic nucleotide for cer sequence

<400>  45
ggtgcgtaca agggatgtta tggtaaat                                          28


<210>  46
<211>  28
<212>  DNA
<213>  Artificial Sequence

<220>
<223>  synthetic nucleotide for psi sequence

<400>  46
ggtgcgcgca agatccatta tgttaaac                                          28


<210>  47
<211>  34
<212>  DNA
<213>  Artificial Sequence

<220>
<223>  synthetic nucleotide for loxP consensus sequence

<400>  47
ataacttcgt atagcataca ttatacgaag ttat                                   34


<210>  48
<211>  34
<212>  DNA
<213>  Artificial Sequence

<220>
<223>  synthetic nucleotide for lox511 sequence

<400>  48
ataacttcgt atagtataca ttatacgaag ttat                                   34


<210>  49
<211>  34
<212>  DNA
<213>  Artificial Sequence

<220>
<223>  synthetic nucleotide for lox2272 sequence

<400>  49
ataacttcgt ataggatact ttatacgaag ttat                                   34


<210>  50
<211>  34
<212>  DNA
<213>  Artificial Sequence

<220>
<223>  synthetic nucleotide for loxFAS sequence

<400>  50
   ataacttcgt atataccttt ctatacgaag ttat                                 34


<210>  51
<211>  34
<212>  DNA
<213>  Artificial Sequence

<220>
<223>  synthetic nucleotide for lox RE sequence

<400>  51
ataacttcgt atagcataca ttatacgaac ggta                                34


<210>  52
<211>  34
<212>  DNA
<213>  Artificial Sequence

<220>
<223>  synthetic nucleotide for lox LE sequence

<400>  52
taccgttcgt atagcataca ttatacgaag ttat                                34


<210>  53
<211>  34
<212>  DNA
<213>  Artificial Sequence

<220>
<223>  synthetic nucleotide for FRT sequence

<400>  53
gaagttccta ttctctagaa agtataggaa cttc                                34


<210>  54
<211>  32
<212>  DNA
<213>  Artificial Sequence

<220>
<223>  synthetic nucleotide for rox sequence

<400>  54
taactttaaa taatgccaat tatttaaagt ta                                  32


<210>  55
<211>  19
<212>  DNA
<213>  Artificial Sequence

<220>
<223>  synthetic nucleotide for Outside End (OE) sequence

<400>  55
ctgtctctta tacacatct                                                    19


<210>  56
<211>  60
<212>  DNA
<213>  Artificial Sequence

<220>
<223>  synthetic nucleotide for SUE4386

<400>  56
ggaaacagct atgaccatga ttacggatag cctggccgtc gttttacaac gtcgtgactg       60


<210>  57
<211>  60
<212>  DNA
<213>  Artificial Sequence

<220>
<223>  synthetic nucleotide for SUE4387

<400>  57
ggaaacagct atgaccatga ttacggatga attcactggc cgtcgtttta caacgtcgtg       60


<210>  58
<211>  39
<212>  DNA
<213>  Artificial Sequence

<220>
<223>  synthetic nucleotide for partial sequence of lacZ wt

<400>  58
atgaccatga ttacggattc actggccgtc gttttacaa                              39


<210>  59
<211>  40
<212>  DNA
<213>  Artificial Sequence

<220>
<223>  synthetic nucleotide for partial sequence of lacZ ins

<400>  59
atgaccatga ttacggattc actcggccgt cgttttacaa                             40


<210>  60
<211>  39
<212>  DNA
<213>  Artificial Sequence

<220>
<223>  synthetic nucleotide for partial sequence of lacZ mis

<400>  60
atgaccatga ttacggatta actggccgtc gttttacaa                              39

<210>  61
<211>  61
<212>  DNA
<213>  Artificial Sequence

<220>
<223>  synthetic nucleotide for partial sequence of pPKOZins

<400>  61
ggaaacagct atgaccatga ttacggattc actcggccgt cgttttacaa cgtcgtgact      60

g                                                                      61


<210>  62
<211>  60
<212>  DNA
<213>  Artificial Sequence

<220>
<223>  synthetic nucleotide for partial sequence of pPKOZ

<400>  62
ggaaacagct atgaccatga ttacggattc actggccgtc gttttacaac gtcgtgactg      60


<210>  63
<211>  60
<212>  DNA
<213>  Artificial Sequence

<220>
<223>  synthetic nucleotide for SUE4579

<400>  63
gtgtgtgcaa ttacattagc tcgttcaagt ggcattagaa ggttacgcaa tagatgtcta      60


<210>  64
<211>  60
<212>  DNA
<213>  Artificial Sequence

<220>
<223>  synthetic nucleotide for SUE4384

<400>  64
gcctgacggc aatctatcta gcgaaacaag gggcacgaag ttcacattat agaaaaacgt      60


<210>  65
<211>  60
<212>  DNA
<213>  Artificial Sequence

<220>
<223>  synthetic nucleotide for SUE4577

<400>  65
ggatagtgtg tgcaattaca ttagctcgtt caagggcatt agaaggttac gcaatagatg      60

<210>  66
<211>  60
<212>  DNA
<213>  Artificial Sequence

<220>
<223>  synthetic nucleotide for SUE4381

<400>  66
ggcagcctga cggcaatcta tctagcgaaa caagggcacg aagttcacat tatagaaaaa        60


<210>  67
<211>  3450
<212>  DNA
<213>  Artificial Sequence

<220>
<223>  synthetic nucleotide for pK3OV preparation

<400>  67
tgatgcctcc gtgtaagggg gatttctgtt catgggggta atgataccga tgaaacgaga        60

gaggatgctc acgatacggg ttactgatga tgaacatgcc cggttactgg aacgttgtga       120

gggtaaacaa ctggcggtat ggatgcggcg ggaccagaga aaaatcactc agggtcaatg       180

ccagcgcttc gttaatacag atgtaggtgt tccacagggt agccagcagc atcctgcgat       240

gcagatccgg aacataatgg tgcagggcgc tgacttccgc gtttccagac tttacgaaac       300

acggaaaccg aagaccattc atgttgttgc tcaggtcgca gacgttttgc agcagcagtc       360

gcttcacgtt cgctcgcgta tcggtgattc attctgctaa ccagtaaggc aaccccgcca       420

gcctagccgg gtcctcaacg acaggagcac gatcatgcta gtcatgcccc gcgcccaccg       480

gaaggagctg actgggttga aggctctcaa gggcatcggt cgagatcccg gtgcctaatg       540

agtgagctaa cttacattaa ttgcgttgcg ctcactgccc gctttccagt cgggaaacct       600

gtcgtgccag ctgcattaat gaatcggcca acgcgcgggg agaggcggtt tgcgtattgg       660

gcgccagggt ggtttttctt ttcaccagtg agacgggcaa cagctgattg cccttcaccg       720

cctggccctg agagagttgc agcaagcggt ccacgctggt ttgccccagc aggcgaaaat       780

cctgtttgat ggtggttaac ggcgggatat aacatgagct gtcttcggta tcgtcgtatc       840

ccactaccga gatgtccgca ccaacgcgca gcccggactc ggtaatggcg cgcattgcgc       900

ccagcgccat ctgatcgttg gcaaccagca tcgcagtggg aacgatgccc tcattcagca       960

tttgcatggt ttgttgaaaa ccggacatgg cactccagtc gccttcccgt tccgctatcg      1020

gctgaatttg attgcgagtg agatatttat gccagccagc cagacgcaga cgcgccgaga      1080

cagaacttaa tgggcccgct aacagcgcga tttgctggtg acccaatgcg accagatgct      1140

ccacgcccag tcgcgtaccg tcttcatggg agaaaataat actgttgatg ggtgtctggt      1200

cagagacatc aagaaataac gccggaacat tagtgcaggc agcttccaca gcaatggcat      1260

cctggtcatc cagcggatag ttaatgatca gcccactgac gcgttgcgcg agaagattgt 1320

gcaccgccgc tttacaggct tcgacgccgc ttcgttctac catcgacacc accacgctgg 1380

cacccagttg atcggcgcga gatttaatcg ccgcgacaat ttgcgacggc gcgtgcaggg 1440

ccagactgga ggtggcaacg ccaatcagca acgactgttt gcccgccagt tgttgtgcca 1500

cgcggttggg aatgtaattc agctccgcca tcgccgcttc cactttttcc cgcgttttcg 1560

cagaaacgtg gctggcctgg ttcaccacgc gggaaacggt ctgataagag acaccggcat 1620

actctgcgac atcgtataac gttactggtt tcacattcac caccctgaat tgactctctt 1680

ccgggcgcta tcatgccata ccgcgaaagg ttttgcgcca ttcgatggtg tccgggatct 1740

cgacgctctc ccttatgcga ctcctgcatt aggaagcagc ccagtagtag gttgaggccg 1800

ttgagcaccg ccgccgcaag gaatggtgca tgcaaggaga tggcgcccaa cagtcccccg 1860

gccacggggc ctgccaccat acccacgccg aaacaagcgc tcatgagccc gaagtggcga 1920

gcccgatctt ccccatcggt gatgtcggcg atataggtta aataccctgt agcggtatag 1980

ataaaacgtt ggtttgcaaa aaaaatcctt agctttcgct aaggatctgc attagaaaaa 2040

ctcatcgagc atcaaatgaa actgcaattt attcatatca ggattatcaa taccatattt 2100

ttgaaaaagc cgtttctgta atgaaggaga aaactcaccg aggcagttcc ataggatggc 2160

aagatcctgg tatcggtctg cgattccgac tcgtccaaca tcaatacaac ctattaattt 2220

cccctcgtca aaaataaggt tatcaagtga gaaatcacca tgagtgacga ctgaatccgg 2280

tgagaatggc aaaagcttat gcatttcttt ccagacttgt tcaacaggcc agccattacg 2340

ctcgtcatca aaatcactcg catcaaccaa accgttattc attcgtgatt gcgcctgagc 2400

gagacgaaat acgcgatcgc tgttaaaagg acaattacaa acaggaatcg aatgcaaccg 2460

gcgcaggaac actgccagcg catcaacaat attttcacct gaatcaggat attcttctaa 2520

tacctggaat gctgttttcc cggggatcgc agtggtgagt aaccatgcat catcaggagt 2580

acggataaaa tgcttgatgg tcggaagagg cataaattcc gtcagccagt ttagtctgac 2640

catctcatct gtaacatcat tggcaacgct acctttgcca tgtttcagaa acaactctgg 2700

cgcatcgggc ttcccataca atcgatagat tgtcgcacct gattgcccga cattatcgcg 2760

agcccattta tacccatata aatcagcatc catgttggaa tttaatcgcg gcctcgagca 2820

agacgtttcc cgttgaatat ggctcataac acgttccttg tattacgtta caagtataac 2880

acaaagtttt ttatgttgtc aagtttatgt aagcagacag ttttattgtt catgatgata 2940

tatttttatc ttgtgcaatg taacatcaga gattttgaga cacaacgtgg ctttgaagat 3000

ccggcagaag aatggagtat gttgtaacta aagataactt cgtataatgt atgctatacg 3060

aagttataca gatcgtgcga tctactgtgg ataactctgt caggaagctt ggatcaaccg 3120

gtagttatcc aaagaacaac tgttgttcag tttttgagtt gtgtataacc cctcattctg     3180

atcccagctt atacggtcca ggatcaccga tcattcacag ttaatgatcc tttccaggtt     3240

gttgatctta aaagccggat ccttgttatc cacagggcag tgcgatccta ataagagatc     3300

acaatagaac agatctctaa ataaatagat cttcttttta atacccagga tcctttagtt     3360

acaacatact caggtctttc tcaagccgac cgccagcaac cgcacctgtg gcgccggtga     3420

tgccggccac gatgcgtccg gcgtagccta                                      3450

<210>  68
<211>  61
<212>  DNA
<213>  Artificial Sequence

<220>
<223>  synthetic nucleotide for vioA that one base inserted

<400>  68
ggatagtgtg tgcaattaca ttagctcgtt caagtggcat tagaaggtta cgcaatagat     60

g                                                                     61

<210>  69
<211>  61
<212>  DNA
<213>  Artificial Sequence

<220>
<223>  synthetic nucleotide for vioC that one base inserted

<400>  69
ggcagcctga cggcaatcta tctagcgaaa caaggggcac gaagttcaca ttatagaaaa     60

a                                                                     61